# EUROPÄISCHE PATENTSCHRIFT

(11) **EP 1 280 811 B1**
(45) Veröffentlichungstag und Bekanntmachung des Hinweises auf die Patenterteilung: **13.10.2004**
(21) Anmeldenummer: 01931693.4
(22) Anmeldetag: 11.05.2001
(51) Int. Cl.: C07F 9/00

(54) **VERFAHREN ZUR HYDROFORMYLIERUNG, VERBRÜCKTE VERBINDUNGEN DES PHOSPHORS, ARSENS UND DES ANTIMONS UND KATALYSATOR, UMFASSEND EINEN KOMPLEX DIESER VERBRÜCKTEN VERBINDUNGEN**
HYDROFORMYLATION METHOD, BRIDGED COMPOUNDS OF PHOSPHORUS, ARSENIC AND ANTIMONY AND CATALYST COMPRISING A COMPLEX OF SAID BRIDGED COMPOUNDS
PROCEDE D'HYDROFORMYLATION, COMPOSES PONTES DU PHOSPHORE, DE L'ARSENIC ET DE L'ANTIMOINE ET CATALYSEUR COMPRENANT UN COMPLEXE DE CES COMPOSES PONTES

(30) Priorität: 12.05.2000 DE 10023471; 17.01.2001 DE 10101939
(43) Veröffentlichungstag der Anmeldung: 05.02.2003
(73) Patentinhaber: BASF AKTIENGESELLSCHAFT, 67056 Ludwigshafen (DE)
(72) Erfinder: AHLERS, Wolfgang, 67549 Worms (DE); PACIELLO, Rocco, 67098 Bad Dürkheim (DE); RÖPER, Michael, 67157 Wachenheim (DE); HOFMANN, Peter, 69118 Heidelberg (DE); TENSFELDT, Markus, 69120 Heidelberg (DE); GOETHLICH, Alexander, 69120 Heidelberg (DE)
(74) Vertreter: Pohl, Michael, Dr.
(86) Internationale Anmeldenummer: PCT/EP2001/005407
(87) Internationale Veröffentlichungsnummer: WO 2001/085739

(56) Entgegenhaltungen:
- US-A- 5 817 883
- DATABASE CHEMABS [Online] CHEMICAL ABSTRACTS SERVICE, COLUMBUS, OHIO, US; SATO, KEIICHI ET AL: "Preparation of aldehydes by catalytic hydroformylation" retrieved from STN Database accession no. 114:246815 XP002171613 & JP 03 017039 A (MITSUBISHI KASEI CORP., JAPAN) 25. Januar 1991 (1991-01-25)
- BENNETT, MARTIN A. ET AL: "Complexes of platinum(II), platinum(IV), rhodium(III) and iridium(III) containing orthometalated triphenylphosphine" DALTON (2000), (20), 3537-3545 , XP002171612
- DATABASE CHEMABS [Online] CHEMICAL ABSTRACTS SERVICE, COLUMBUS, OHIO, US; ADAM, WALDEMAR ET AL: "Electronic substituent effects in radical chemistry: the.DELTA.D scale for benzyl-type radicals" retrieved from STN Database accession no. 128:47943 XP002171614 & AN. QUIM. INT. ED. (1997), 93(4), 271-276 ,
- DATABASE CHEMABS [Online] CHEMICAL ABSTRACTS SERVICE, COLUMBUS, OHIO, US; TERFORT, ANDREAS ET AL: "Enantioselective catalysis. 99. Phosphine ligands with two binding sites of differing hardness for enantioselective Grignard cross coupling" retrieved from STN Database accession no. 125:195803 XP002171615 & J. CHEM. SOC., PERKIN TRANS. 1 (1996), (12), 1467-1479 ,

## Beschreibung

Die vorliegende Erfindung betrifft ein Verfahren zur Hydroformylierung ethylenisch ungesättigter Verbindungen, wobei man als Hydroformylierungskatalysator wenigstens einen Komplex eines Metalls der VIII. Nebengruppe mit mindestens einem Liganden einsetzt, der zwei Triarylgruppen umfasst, wobei jede der Triarylgruppen einen Phosphor-, Arsen- oder Antimonatom-haltigen Rest aufweist und wobei jeweils ein Arylrest der beiden Triarylgruppen über eine Einfachbindung an eine nichtaromatische 3- bis 8-gliedrige carbocyclische oder heterocyclische verbrückende Gruppe gebunden ist. Die Erfindung betrifft weiterhin neue verbrückte Verbindungen dieses Typs und Katalysatoren, die wenigstens einen Komplex eines Metalls der VIII. Nebengruppe mit mindestens einer solchen Verbindung als Liganden umfassen.

Die Hydroformylierung oder Oxo-Synthese ist ein wichtiges großtechnisches Verfahren und dient der Herstellung von Aldehyden aus Olefinen, Kohlenmonoxid und Wasserstoff. Diese Aldehyde können gegebenenfalls im gleichen Arbeitsgang mit Wasserstoff zu den entsprechenden Oxo-Alkoholen hydriert werden. Die Reaktion selbst ist stark exotherm und läuft im Allgemeinen unter erhöhtem Druck und bei erhöhten Temperaturen in Gegenwart von Katalysatoren ab. Als Katalysatoren werden Co-, Rh-, Ir-, Ru-, Pd- oder Pt-Verbindungen bzw. -komplexe eingesetzt, die zur Aktivitäts- und/oder Selektivitätsbeeinflussung mit N- oder P-haltigen Liganden modifiziert sein können. Bei der Hydroformylierungsreaktion kommt es aufgrund der möglichen CO-Anlagerung an jedes der beiden C-Atome einer Doppelbindung zur Bildung von Gemischen isomerer Aldehyde. Zusätzlich kann es auch zu einer Doppelbindungsisomerisierung kommen. Aufgrund der wesentlich größeren technischen Bedeutung der α-Aldehyde wird eine Optimierung der Hydroformylierungskatalysatoren zur Erzielung einer möglichst hohen Hydroformylierungsaktivität bei gleichzeitig möglichst geringer Neigung zur Bildung nicht α-ständiger Doppelbindungen angestrebt. Zudem besteht ein Bedarf an Hydroformylierungskatalysatoren, die auch ausgehend von internen linearen Olefinen in guten Ausbeuten zu α-ständigen und insbesondere n-ständigen Aldehyden führen. Hierbei muss der Katalysator sowohl die Einstellung eines Gleichgewichts zwischen internen und terminalen Doppelbindungsisomeren als auch möglichst selektiv die Hydroformylierung der terminalen Olefine ermöglichen.

Es ist bekannt, bei der Rhodium-Niederdruck-Hydroformylierung phosphorhaltige Liganden zur Stabilisierung und/oder Aktivierung des Katalysatormetalls einzusetzen. Geeignete phosphorhaltige Liganden sind z. B. Phosphine, Phosphinite, Phosphonite, Phosphite, Phosphoramidite, Phosphole und Phosphabenzole. Die derzeit am weitesten verbreiteten Liganden sind Triarylphosphine, wie z. B. Triphenylphosphin und sulfoniertes Triphenylphosphin, da diese unter den Reaktionsbedingungen eine hinreichende Stabilität besitzen. Nachteilig an diesen Liganden ist jedoch, dass im Allgemeinen nur sehr hohe Ligandenüberschüsse zufriedenstellende Ausbeuten insbesondere an linearen Aldehyden liefern. Demgegenüber erlauben Chelatphosphite bereits bei im Allgemeinen sehr geringen Ligandenüberschüssen hohe Ausbeuten an linearen Aldehyden. Nachteilig an diesen Liganden ist jedoch ihre geringe Stabilität, die sich verbunden mit ihren relativ hohen Beschaffungskosten negativ auf die Wirtschaftlichkeit des Hydroformylierungsprozesses auswirkt.

In Beller et al., Journal of Molecular Catalysis A, 104 (1995), Seiten 17-85, werden rhodiumhaltige, phosphinmodifizierte Katalysatoren zur Hydroformylierung von niedrig siedenden Olefinen beschrieben.

Die DE-A-196 523 50 beschreibt Katalysatoren auf Basis von 4,5-Diphosphinoacridin-Liganden. Diese eignen sich zur Katalyse der Kohlenmonoxid-Konvertierung über das Wassergasgleichgewicht. Nachteilig an diesen Liganden ist ihr aufwendiger mehrstufiger Syntheseweg sowie der für eine Rhodium-Chelatisierung ungünstige

Bisswinkel.

Haenel et al. beschreiben in Tetrahedron Letters, Band 34, Nr. 13, Seiten 2107 ff. (1993), in Tetrahedron Letters, Band 36, Nr. 1, Seiten 75 ff. (1995) und in Chem. Ber. 124, Seite 1705 ff. (1991) die Synthese von Bis-(diphenylphosphino)chelaten mit Anthracen-, Dibenzofuran-, Dibenzothiophen- und Xanthen-Grundkörpern. Ein Einsatz dieser Verbindungen in der Katalyse wird nicht beschrieben. Nachteilig an diesen Verbindungen ist ihr mehrstufiger Syntheseweg sowie wiederum der für eine Rhodium-Chelatisierung ungünstige Bisswinkel.

Van Leeuwen et al. beschreiben in Organometallics 1995, 14, Seite 3081 ff. die Synthese von Chelatphosphinen mit Xanthen-Grundkörpern sowie deren Einsatz als Cokatalysatoren in der Rhodium-Niederdruck-Hydroformylierung von α-Olefinen. Nachteilig an diesen Liganden ist die aufwendige Synthese des Xanthen-Grundkörpers sowie die Notwendigkeit zum Einsatz empfindlicher metallorganischer Verbindungen bei der Synthese. Hydroformylierungsverfahren unter Einsatz von Katalysatoren auf Basis dieser Liganden sind daher wirtschaftlich benachteiligt.

Die WO-A-87/07600 beschreibt den Einsatz von Chelatliganden in Katalysatoren für die Niederdruck-Hydroformylierung. Als Chelatliganden werden dabei z. B. Verbindungen eingesetzt, bei denen zwei Diarylphosphingruppen jeweils über eine C₁-Alkylengruppe an eine 1,1'-Bisarylgruppe als verbrückende Gruppe gebunden sind. Nachteilig an diesem Verfahren ist die hohe Basizität der eingesetzten Liganden, deren katalytische Aktivität verbesserungswürdig ist.

Die WO-A-95/30680 beschreibt zweizähnige Phosphinliganden, bei denen die Phosphoratome direkt an eine verbrückende Gruppe gebunden sind, welche zwei Arylgruppen umfasst, die Teil eines orthoanellierten Ringsystems sind.

Van der Veen et al. beschreiben in Angewandte Chemie 1999, 111, S. 349 ff. Rhodiumkatalysatoren für die Hydroformylierung interner Olefine zu linearen Aldehyden, wobei als Liganden Bisphosphine auf Xanthen-Basis eingesetzt werden. Nachteilig an diesen Katalysatoren ist die aufwendige Synthese des Xanthen-Grundkörpers sowie die Notwendigkeit zum Einsatz metallorganischer Verbindungen bei der Synthese.

Fukuda et al. beschreiben in Tetrahedron Letters, Vol. 31, Nr. 49, S. 7185 bis 7188 (1990) die Synthese von trans-Cyclopentylenverbrückten Bis(triphenylphosphinen) und deren Einsatz als Liganden in Katalysatoren für die asymmetrische Hydrierung. Ein Einsatz in der Hydroformylierung wird nicht beschrieben.

Die JP-A-3261791 beschreibt ebenfalls die Synthese von Cyclopentylen-verbrückten Bis(triphenylphosphinen) und deren Einsatz als Liganden in Übergangsmetallkatalysatoren zur Hydrierung von Ketonen und α-Acylaminoacrylsäuren.

Die JP-A-3017039 beschreibt ein Verfahren zur Herstellung von Aldehyden durch Hydroformylierung von Olefinen in Gegenwart eines Rhodiumkatalysators, der als Liganden ein cyclisches Siloxan mit daran über Phenylenbrücken gebundenen Diphenylphosphinresten aufweist.

Die US 5,817,883 beschreibt ein Verfahren zur Herstellung von substituierten oder unsubstituierten Hydroxyaldehyden, z. B. 6-Hydroxyhexanalen, bei dem man Alkadiene einer Hydrocarbonylierung und einer Hydroformylierung unterzieht, wobei beide Umsetzungen in Gegenwart eines Metallkomplexkatalysators mit Organophosphorliganden erfolgen können.

W. Adam und H. M. Harrer beschreiben in An. Chim. Int. Ed. (1997), 93(4), S. 271-276 elektronische Substituenteneffekte bestimmter Monoradikale und 1,3-Diradikale. In diesem Dokument sind unter Anderem Verbindungen beschrieben, bei denen zwei Benzolringe über eine Ethylen-, -CH₂-Hg-CH₂- oder eine Hexafluorcyclobutylen-1,2-brücke miteinander verknüpft sind, wobei an die Benzolringe zusätzlich je ein Diphenylphosphinitrest gebunden sein kann.

A. Terfort und H. Brunner beschreiben in J. Chem. Soc., Perkin Trans. 1 (1996), (12), S. 1467-1479 zweizähnige Phosphinliganden für enantioselektive Grignard-Kreuzkupplungs-Reaktionen.

Der vorliegenden Erfindung liegt die Aufgabe zugrunde, ein verbessertes Verfahren zur Hydroformylierung von Verbindungen, die wenigstens eine ethylenisch ungesättigte Doppelbindung enthalten, zur Verfügung zu stellen. Dabei soll bei der Hydroformylierung von α-Olefinen vorzugsweise ein möglichst hoher Anteil an α-Aldehyden, bzw. -Alkoholen erzielt werden. Bevorzugt soll das Verfahren auch zur Hydroformylierung interner linearer Olefine bei möglichst hoher Regioselektivität zugunsten terminaler Produktaldehyde geeignet sein. Insbesondere sollen dabei auch die Katalysatorstandzeiten möglichst hoch sein. Der Erfindung liegt weiterhin die Aufgabe zugrunde, neue Verbindungen und neue Katalysatoren, die wenigstens einen Komplex eines Metalls der VIII. Nebengruppe mit einer solchen Verbindung als Liganden umfassen, zur Verfügung zu stellen. Vorzugsweise sollen diese Katalysatoren bei ihrem Einsatz in der Hydroformylierung eine hohe α-Selektivität und/oder Regioselektivität und/oder unter den Reaktionsbedingungen eine hohe Stabilität aufweisen.

Überraschenderweise wurde nun gefunden, dass diese Aufgabe durch ein Verfahren zur Hydroformylierung gelöst wird, wobei man als Hydroformylierungskatalysator wenigstens einen Komplex eines Metalls der VIII. Nebengruppe mit mindestens einem Liganden einsetzt, der zwei Triarylgruppen umfasst, wobei jede der Triarylgruppen einen Phosphor-, Arsen- oder Antimonatom-haltigen Rest aufweist, wobei jeweils ein Arylrest der beiden Triarylgruppen über eine Einfachbindung an eine nichtaromatische 3- bis 8-gliedrige carbocyclische oder heterocyclische verbrückende Gruppe gebunden ist.

Gegenstand der vorliegenden Erfindung ist ein Verfahren zur Hydroformylierung von Verbindungen, die wenigstens eine ethylenisch ungesättigte Doppelbindung enthalten, durch Umsetzung mit Kohlenmonoxid und Wasserstoff in Gegenwart eines Hydroformylierungskatalysators, dadurch gekennzeichnet, dass man als Hydroformylierungskatalysator wenigstens einen Komplex eines Metalls der VIII. Nebengruppe mit mindestens einem Liganden der allgemeinen Formel I 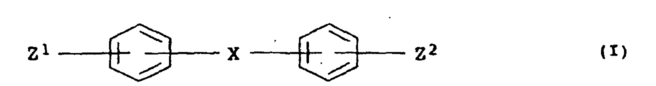 einsetzt, worin
- Z¹und Z²: unabhängig voneinander für mindestens ein Phosphor-, Arsen- oder Antimonatom aufweisende Reste stehen, und
- X: für eine zweiwertige, nichtaromatische, 3- bis 8-gliedrige, carbocyclische oder heterocyclische, verbrückende Gruppe steht, die eine, zwei oder drei Doppelbindungen, und/oder einen, zwei, drei oder vier Substituenten, die ausgewählt sind unter Alkyl, Cycloalkyl, Aryl, Hetaryl, Alkoxy, Acyl, Carboxyl, Alkoxycarbonyl, Hydroxy, Nitro, Cyano, Trifluormethyl, Oxo und den Ketalen davon, oder NE¹E², tragen können, wobei E¹ und E² gleich oder verschieden sein können und für Alkyl, Cycloalkyl oder Aryl stehen, und/oder wobei die verbrückende Gruppe X Teil eines kondensierten Ringsystems mit 1, 2 oder 3 weiteren Ringen sein kann, wobei die ankondensierten Ringe je einen, zwei oder drei Substituenten, die unter den zuvor als Substituenten der Gruppe X genannten ausgewählt sind, tragen können, und/oder wobei die verbrückende Gruppe X ihrerseits durch -O-, -S-, -N(R^{a})- oder eine davon verschiedene zweiwertige verbrückende Gruppe überbrückt sein kann, wobei R^{a} für Wasserstoff, Alkyl, Cycloalkyl oder Aryl steht, und wobei X ausgewählt ist unter Gruppen der Formeln II.1 bis II.3 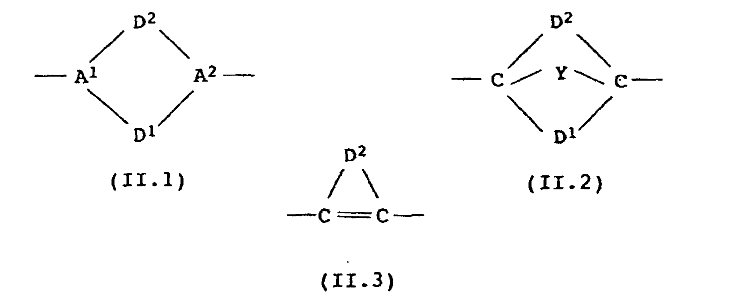 worin
- A¹ und A²: unabhängig voneinander für B, N, P oder CR⁵ stehen, wobei R⁵ für Wasserstoff, Alkyl, Cycloalkyl, Aryl oder Hetaryl steht,
- D¹: für eine Einfachbindung oder eine C₁- bis C₃-Alkylenbrücke steht, die eine Doppelbindung und/oder einen oder zwei Substituenten, die ausgewählt sind unter Alkyl, Cycloalkyl, Aryl, Alkoxy, Acyl, Carboxyl, Alkoxycarbonyl, Hydroxy, Nitro, Cyano, Trifluormethyl, Oxo und den Ketalen davon, oder NE¹E², tragen können, wobei E¹ und E² gleich oder verschieden sein können und für Alkyl, Cycloalkyl oder Aryl stehen,
- D²: für eine C₃- bis C₆-Alkylenbrücke steht, die eine zwei oder drei Doppelbindungen und/oder einen, zwei, drei oder vier der für D¹ genannten Substituenten aufweisen kann und/oder die Alkylenbrücke D² in den Formeln II.1 und II.3 durch eine Gruppe -Y- überbrückt sein kann, und
- Y: für O, S, CR^{x}R^{y} oder einen Rest der Formeln III.1 bis III.4 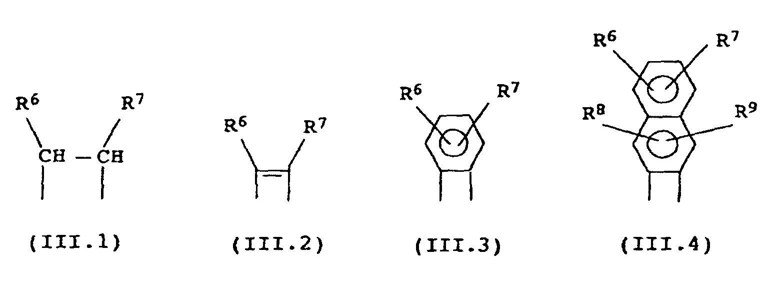 steht, worin
R^{x} und R^{y} ausgewählt sind unter Wasserstoff, Alkyl, Cycloalkyl, Aryl oder Hetaryl,
R⁶, R⁷, R⁸ und R⁹ unabhängig voneinander für Wasserstoff, Alkyl, Cycloalkyl, Aryl, Alkoxy, Halogen, SO₃H, Sulfonat, NE¹E², Alkylen-NE¹E², Trifluormethyl, Nitro, Alkoxycarbonyl, Carboxyl oder Cyano stehen, wobei E¹ und E² unabhängig voneinander für Alkyl, Cycloalkyl oder Aryl stehen.

Z¹ und Z² stehen vorzugsweise für ein Phosphoratom aufweisende Reste. Bevorzugt sind Z¹ und Z² ausgewählt unter Phosphin-, Phosphinit-, Phosphonit- und Phosphitresten. Insbesondere stehen Z¹ und Z² unabhängig voneinander für einen Rest der Formeln PR¹R², OPR¹R², P(OR¹)R², P(OR¹)(OR²), OP(OR¹)R² oder OP(OR¹)(OR²), worin
- R¹ und R²: unabhängig voneinander für Alkyl, Cycloalkyl, Heterocycloalkyl, Aryl oder Hetaryl stehen, welche gegebenenfalls einen, zwei oder drei Substituenten, ausgewählt unter Alkyl, Cycloalkyl, Heterocycloalkyl, Aryl, Hetaryl, COOR^{f}, COO⁻M⁺, SO₃R^{f}, SO⁻₃M⁺, NE³E⁴, Alkylen-NE³E⁴, NE³E⁴E⁵⁺X⁻, Alkylen-NE³E⁴E⁵⁺X⁻, OR^{f}, SR^{f}, (CHR^{g}CH₂O)_{y}R^{f}, (CH₂N(E³))_{y}R^{f}, (CH₂CH₂N(E³))_{y}R^{f}, Halogen, Trifluormethyl, Nitro, Acyl oder Cyano aufweisen können, worin
R^{f}, E³, E⁴ und E⁵ jeweils gleiche oder verschiedene Reste, ausgewählt unter Wasserstoff, Alkyl, Cycloalkyl oder Aryl bedeuten,
R^{g} für Wasserstoff, Methyl oder Ethyl steht,
M⁺ für ein Kation steht,
X⁻ für ein Anion steht, und
y für eine ganze Zahl von 1 bis 120 steht, oder
R¹ und R² zusammen mit dem Phosphoratom und gegebenenfalls dem/den Sauerstoffatom(en), an die sie gebunden sind für einen 5- bis 8-gliedrigen Heterocyclus stehen, der gegebenenfalls zusätzlich ein-, zwei- oder dreifach mit Cycloalkyl, Heterocycloalkyl, Aryl oder Hetaryl anelliert ist, wobei der Heterocyclus und, falls vorhanden, die anellierten Gruppen unabhängig voneinander je einen, zwei, drei oder vier Substituenten tragen können, die ausgewählt sind unter Alkyl, Cycloalkyl, Heterocycloalkyl, Aryl, Hetaryl, COOR^{f}, COO⁻M⁺, SO₃R^{f}, SO⁻₃M⁺, NE³E⁴, Alkylen-NE³E⁴, NE³E⁴E⁵⁺X⁻, Alkylen-NE³E⁴E⁵⁺X⁻, OR^{f}, SR^{f}, (CHR^{g}CH₂O)_{y}R^{f}, (CH₂N(E¹))_{y}R^{f}, (CH₂CH₂N(E⁴))_{y}R^{f}, Halogen, Trifluormethyl, Nitro, Acyl oder Cyano, wobei R^{f}, R^{g}, E³, E⁴, E⁵, M⁺, X⁻ und y die zuvor angegebenen Bedeutungen besitzen.

Nach einer ersten bevorzugten Ausführungsform des erfindungsgemäßen Verfahrens wird als Hydroformylierungskatalysator wenigstens ein Komplex mit einer Verbindung der Formel I als Ligand eingesetzt, wobei Z¹ und Z² für Phosphonitgruppen stehen. Nach einer weiteren bevorzugten Ausführungsform stehen Z¹ und Z² für Phosphingruppen.

Bevorzugt ist ein Verfahren, bei dem man als Hydroformylierungskatalysator wenigstens einen Komplex eines Metalls der VIII. Nebengruppe mit mindestens einem Phosphinliganden der allgemeinen Formel I' 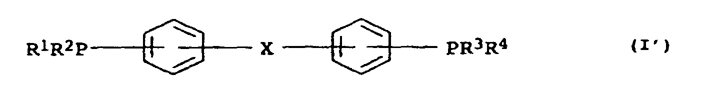 einsetzt, worin
- R¹, R², R³ und R⁴: unabhängig voneinander für Alkyl, Cycloalkyl, Aryl oder Hetaryl stehen, wobei die Cycloalkyl-, Aryl- und Hetarylgruppen je einen, zwei oder drei Substituenten, die ausgewählt sind unter Alkyl, Cycloalkyl,Aryl, Alkoxy, Cycloalkoxy, Aryloxy, Acyl, Halogen, Trifluormethyl, Nitro, Cyano, Carboxyl, Alkoxycarbonyl oder NE³E⁴,tragen können, wobei E³ und E⁴ gleich oder verschieden sein können und für Alkyl, Cycloalkyl oder Aryl stehen, und
- X: für eine zweiwertige, nichtaromatische, 3- bis 8-gliedrige, carbocyclische oder heterocyclische, verbrückende Gruppe steht, die eine, zwei oder drei Doppelbindungen, und/oder einen, zwei, drei oder vier Substituenten, die ausgewählt sind unter Alkyl, Cycloalkyl, Aryl, Alkoxy, Acyl, Carboxyl, Alkoxycarbonyl, Hydroxy, Nitro, Cyano, Trifluormethyl, Oxo und den Ketalen davon, oder NE¹E², tragen können, wobei E¹ und E² gleich oder verschieden sein können und für Alkyl, Cycloalkyl oder Aryl stehen, und/oder wobei die verbrückende Gruppe X Teil eines kondensierten Ringsystems mit 1, 2 oder 3 weiteren Ringen sein kann, wobei die ankondensierten Ringe je einen, zwei oder drei Substituenten, die unter den zuvor als Substituenten der Gruppe X genannten ausgewählt sind, tragen können, und/oder wobei die verbrückende Gruppe X ihrerseits durch -O-, -S-, -N(R^{a})- oder eine davon verschiedene zweiwertige verbrückende Gruppe überbrückt sein kann, wobei R^{a} für Wasserstoff, Alkyl, Cycloalkyl oder Aryl steht, und wobei X ausgewählt ist unter Gruppen der Formeln II.1 bis II.3 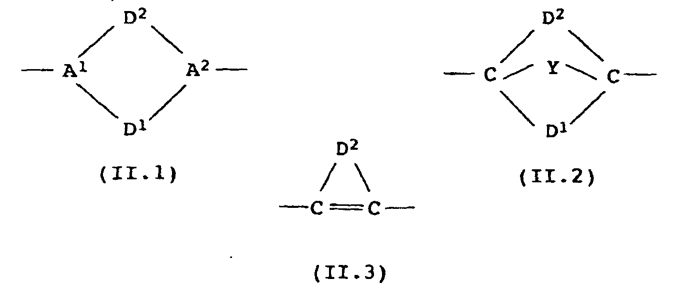 worin
A¹ und A² unabhängig voneinander für B, N, P oder CR⁵ stehen, wobei R⁵ für Wasserstoff, Alkyl, Cycloalkyl, Aryl oder Hetaryl steht,
D¹ für eine Einfachbindung oder eine C₁- bis C₃-Alkylenbrücke steht, die eine Doppelbindung und/oder einen oder zwei Substituenten, die ausgewählt sind unter Alkyl, Cycloalkyl, Aryl, Alkoxy, Acyl, Carboxyl, Alkoxycarbonyl, Hydroxy, Nitro, Cyano, Trifluormethyl, Oxo und den Ketalen davon, oder NE¹E², tragen können, wobei E¹ und E² gleich oder verschieden sein können und für Alkyl, Cycloalkyl oder Aryl stehen,
D² für eine C₃- bis C₆-Alkylenbrücke steht, die eine zwei oder drei Doppelbindungen und/oder einen, zwei, drei oder vier der für D¹ genannten Substituenten aufweisen kann und/oder die Alkylenbrücke D² in den Formeln II.1 und II.3 durch eine Gruppe -Y- überbrückt sein kann, und
Y für O, S, CR^{x}R^{y} oder einen Rest der Formeln III.1 bis III.4 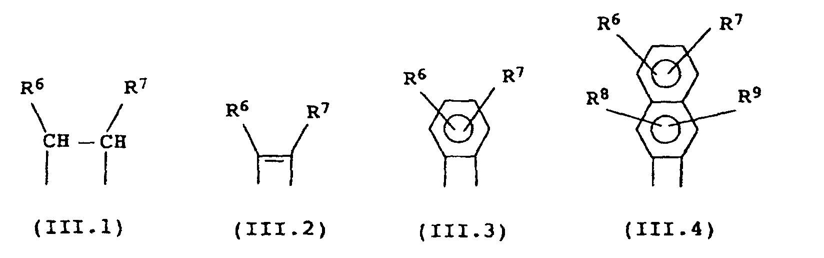 steht, worin
R^{x} und R^{y} ausgewählt sind unter Wasserstoff, Alkyl, Cycloalkyl, Aryl oder Hetaryl,
R⁶, R⁷, R⁸ und R⁹ unabhängig voneinander für Wasserstoff, Alkyl, Cycloalkyl, Aryl, Alkoxy, Halogen, SO₃H, Sulfonat, NE¹E², Alkylen-NE¹E², Trifluormethyl, Nitro, Alkoxycarbonyl, Carboxyl oder Cyano stehen, wobei E¹ und E² unabhängig voneinander für Alkyl, Cycloalkyl oder Aryl stehen.

Im Rahmen der vorliegenden Erfindung umfasst der Ausdruck 'Alkyl' geradkettige und verzweigte Alkylgruppen. Vorzugsweise handelt es sich dabei um geradkettige oder verzweigte C₁-C₈-Alkyl-, bevorzugterweise C₁-C₆-Alkyl- und besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl.

Bei der Cycloalkylgruppe handelt es sich vorzugsweise um eine C₄bis C₈-Cycloalkylgruppe und insbesondere um eine C₅-C₇-Cycloalkylgruppe, wie Cyclopentyl, Cyclohexyl oder Cycloheptyl.

Wenn die Cycloalkylgruppe substituiert ist, weist sie vorzugsweise 1, 2, 3, 4 oder 5, insbesondere 1, 2 oder 3 Substituenten, ausgewählt unter Alkyl, Alkoxy oder Halogen auf.

Aryl steht vorzugsweise für Phenyl, Tolyl, xylyl, Mesityl, Naphthyl, Anthracenyl, Phenanthrenyl, Naphthacenyl und insbesondere für Phenyl oder Naphthyl.

Substituierte Arylreste weisen vorzugsweise 1, 2, 3, 4 oder 5, insbesondere 1, 2 oder 3 Substituenten, ausgewählt unter Alkyl, Alkoxy, Carboxyl, Carboxylat, Trifluormethyl, -SO₃H, Sulfonat, NE¹E², Alkylen-NE¹E², Nitro, Cyano oder Halogen auf.

Hetaryl steht vorzugsweise für Pyrrolyl, Pyrazolyl, Imidazolyl, Indolyl, Carbazolyl, Pyridyl, Chinolinyl, Acridinyl, Pyridazinyl, Pyrimidinyl oder Pyrazinyl.

Substituierte Hetarylreste weisen vorzugsweise 1, 2 oder 3 Substituenten, ausgewählt unter Alkyl, Alkoxy, -SO₃H, Sulfonat, NE¹E², Alkylen-NE¹E², Trifluormethyl oder Halogen auf.

Die obigen Ausführungen zu Alkyl-, Cycloalkyl- und Arylresten gelten entsprechend für Alkoxy-, Cycloalkyloxy- und Aryloxyreste.

Die Reste NE¹E² und NE³E⁴ stehen vorzugsweise für N,N-Dimethyl, N,N-Diethyl, N,N-Dipropyl, N,N-Diisopropyl, N,N-Di-n-butyl, N,N-Di-t.-butyl, N,N-Dicyclohexyl oder N,N-Diphenyl.

Halogen steht für Fluor, Chlor, Brom und Iod, bevorzugt für Fluor, Chlor und Brom.

Carboxylat und Sulfonat stehen im Rahmen dieser Erfindung vorzugsweise für ein Derivat einer Carbonsäurefunktion bzw. einer Sulfonsäurefunktion, insbesondere für ein Metallcarboxylat oder - sulfonat, eine Carbonsäure- oder Sulfonsäureesterfunktion oder eine Carbonsäure- oder Sulfonsäureamidfunktion, besonders bevorzugt für eine Esterfunktion. Dazu zählen z. B. die Ester mit C₁-C₄-Alkanolen, wie Methanol, Ethanol, n-Propanol, Isopropanol, n-Butanol, sec.-Butanol und tert.-Butanol.

M⁺ steht vorzugsweise für ein Kation, wie Li⁺, Na⁺, K⁺ oder NH₄⁺,

X⁻ steht vorzugsweise für ein Anion, wie F⁻, Cl⁻ oder Br⁻.

Y steht vorzugsweise für eine ganze Zahl von 2 bis 100.

Nach einer bevorzugten Ausführungsform ist in der Formel I einer der Reste Z¹ oder Z² oder sind beide Reste Z¹ und Z² ausgewählt unter solchen Resten der Formeln PR¹R², OPR¹R², P(OR¹)R², P(OR¹)(OR²), OP(OR¹)R² und OP(OR¹)(OR²), worin R¹ und R² zusammen mit dem Phosphoratom und gegebenenfalls dem/den Sauerstoffatom(en), an die sie gebunden sind, für einen 5- bis 8-gliedrigen Heterocyclus stehen, der gegebenenfalls zusätzlich ein-, zweioder dreifach mit Cycloalkyl, Heterocycloalkyl, Aryl und/oder Hetaryl anelliert ist, wobei der Heterocyclus und/oder die anellierten Gruppen unabhängig voneinander je einen, zwei, drei oder vier Substituenten, die ausgewählt unter Alkyl, Alkoxy, Halogen, Nitro, Cyano, Carboxyl, SO₃H, Sulfonat, NE³E⁴, Alkylen-NE³E⁴ und Carboxylat, tragen können.

Bevorzugt sind die Reste Z¹ und Z² ausgewählt unter Resten der allgemeinen Formel IV 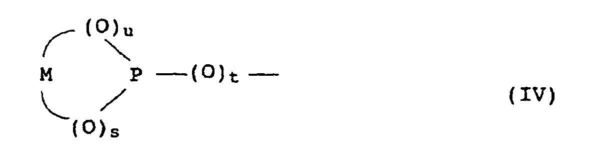 worin
- s, t und u: unabhängig voneinander für 0 oder 1 stehen,
- M: zusammen mit dem Phosphoratom und dem/den Sauerstoffatom(en), an die es gebunden ist, für einen 5- bis 8-gliedrigen Heterocyclus steht, der gegebenenfalls ein-, zwei- oder dreifach mit Cycloalkyl, Heterocycloalkyl, Aryl und/oder Hetaryl anelliert ist, wobei die anellierten Gruppen unabhängig voneinander je einen, zwei, drei oder vier Substituenten, ausgewählt unter Alkyl, Alkoxy, Halogen, SO₃H, Sulfonat, NE³E⁴, AlkylenNE³E⁴, Nitro, Cyano, Carboxyl und Carboxylat, tragen können und/oder M einen, zwei oder drei Substituenten, die ausgewählt sind unter Alkyl, Alkoxy, gegebenenfalls substituiertem Cycloalkyl und gegebenenfalls substituiertem Aryl, aufweisen kann und/oder M durch 1, 2 oder 3 gegebenenfalls substituierte Heteroatome unterbrochen sein kann.

Der Rest M steht vorzugsweise für eine C₂- bis C₆-Alkylenbrücke, die 1-, oder 2-fach mit Aryl anelliert ist und/oder die einen Substituenten, der ausgewählt ist unter Alkyl, gegebenenfalls substituiertem Cycloalkyl und gegebenenfalls substituiertem Aryl, aufweisen kann und/oder die durch ein gegebenenfalls substituiertes Heteroatom unterbrochen sein kann.

Bei den anellierten Arylen der Reste M handelt es sich bevorzugt um Benzol oder Naphthalin. Anellierte Benzolringe sind vorzugsweise unsubstituiert oder weisen 1, 2 oder 3, insbesondere 1 oder 2 Substituenten auf, die ausgewählt sind unter Alkyl, Alkoxy, Halogen, SO₃H, Sulfonat, NE³E⁴, Alkylen-NE³E⁴, Trifluormethyl, Nitro, Carboxyl, Alkoxycarbonyl, Acyl und Cyano. Anellierte Naphthaline sind vorzugsweise unsubstituiert oder weisen im nicht anellierten Ring und/oder im anellierten Ring jeweils 1, 2 oder 3, insbesondere 1 oder 2 der zuvor bei den anellierten Benzolringen genannten Substituenten auf. Bei den Substituenten der anellierten Aryle steht Alkyl vorzugsweise für C₁- bis C₄-Alkyl und insbesondere für Methyl, Isopropyl und tert.-Butyl. Alkoxy steht dabei vorzugsweise für C₁- bis C₄-Alkoxy und insbesondere für Methoxy.

Alkoxycarbonyl steht vorzugsweise für C₁- bis C₄-Alkoxycarbonyl.

Halogen steht dabei insbesondere für Fluor und Chlor.

Wenn die C₂- bis C₆-Alkylenbrücke des Restes M durch 1, 2 oder 3, gegebenenfalls substituierte Heteroatome unterbrochen ist, so sind diese vorzugsweise ausgewählt unter O, S oder NR^{h}, wobei R^{h} für Alkyl, Cycloalkyl oder Aryl steht. Vorzugsweise ist die C₂bis C₆-Alkylenbrücke des Restes M durch ein gegebenenfalls substituiertes Heteroatom unterbrochen.

Wenn die C₂- bis C₆-Alkylenbrücke des Restes M substituiert ist, so weist sie vorzugsweise 1, 2 oder 3, insbesondere 1 Substituenten auf, der/die ausgewählt ist/sind unter Alkyl, Cycloalkyl und Aryl, wobei der Arylsubstituent 1, 2 oder 3 der für Aryl genannten Substituenten tragen kann. Vorzugsweise weist die Alkylenbrücke D einen Substituenten auf, der ausgewählt ist unter Methyl, Ethyl, Isopropyl, Phenyl, p-(C₁- bis C₄-Alkyl)phenyl, bevorzugt p-Methylphenyl, p-(C₁- bis C₄-Alkoxy)phenyl, bevorzugt p-Methoxyphenyl, p-Halogenphenyl, bevorzugt p-Chlorphenyl und p-Trifluormethylphenyl.

Vorzugsweise steht der Rest M für eine C₃- bis C₆-Alkylenbrücke, die wie zuvor beschrieben anelliert und/oder substituiert und/oder durch gegebenenfalls substituierte Heteroatome unterbrochen ist. Insbesondere steht der Rest M für eine C₃- bis C₆-Alkylenbrücke, die ein- oder zweifach mit Phenyl und/oder Naphthyl anelliert ist, wobei die Phenyl- oder Naphthylgruppen 1, 2 oder 3, insbesondere 1 oder 2 der zuvor genannten Substituenten tragen können.

Vorzugsweise steht der Rest M (d. h. R¹ und R² gemeinsam) zusammen mit dem Phosphoratom und dem/den Sauerstoffatom(en), an die er gebunden ist, für einen 5- bis 8-gliedrigen Heterocyclus, wobei M (R¹ und R² gemeinsam) für einen Rest steht, der ausgewählt ist unter den Resten der Formeln IV.1 bis IV.5, 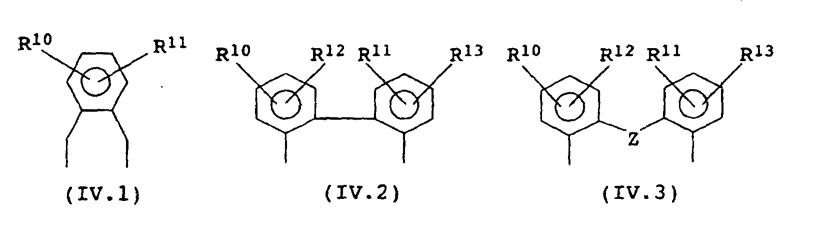 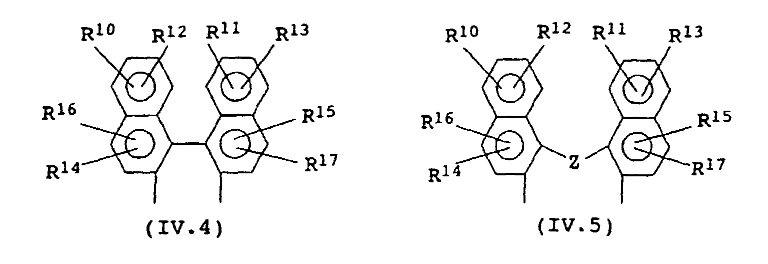 worin
- Z: für O, S oder NRⁱ steht, wobei Rⁱ für Alkyl, Cycloalkyl oder Aryl steht,
- oder Z: für eine C₁- bis C₃-Alkylenbrücke steht, die eine Doppelbindung und/oder einen Alkyl-, Cycloalkyl- oder Arylsubstituenten aufweisen kann, wobei der Arylsubstituent einen, zwei oder drei der für Aryl genannten Substituenten tragen kann,
- oder Z: für eine C₂- bis C₃-Alkylenbrücke steht, die durch O, S oder NRⁱ unterbrochen ist,
- R¹⁰, R¹¹, R¹², R¹³, R¹⁴, R¹⁵, R¹⁶ und R¹⁷: unabhängig voneinander ausgewählt sind unter Wasserstoff, Alkyl, Cycloalkyl, Aryl, Alkoxy, Cycloalkoxy, Aryloxy, Acyl, Halogen, SO₃H, Sulfonat, NE³E⁴, Alkylen-NE³E⁴, Trifluormethyl, Nitro, Alkoxycarbonyl, Carboxyl und Cyano,
- wobei E³ und E⁴: gleich oder verschieden sein können und für Alkyl, Cycloalkyl oder Aryl stehen.

Nach einer weiteren bevorzugten Ausführungsform ist/sind in der Formel I einer/beide der Reste Z¹ oder Z² ausgewählt unter solchen Resten der Formeln PR¹R², OPR¹R², P(OR¹)R², P(OR¹)(OR²), OP(OR¹)R² und OP(OR¹)(OR²), worin die Reste R¹ und R² nicht miteinander verbrückt sind. Bevorzugt stehen R¹ und R² unabhängig voneinander für Alkyl, Cycloalkyl, Aryl oder Hetaryl, wobei die Aryl- und Hetarylgruppen je einen, zwei oder drei Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE³E⁴, tragen können, wobei E³ und E⁴ gleich oder verschieden sein können und für Alkyl, Cycloalkyl oder Aryl stehen.

Bei den Phosphinliganden der allgemeinen Formel I' können die Reste R¹ und R² und/oder R³ und R⁴ gegebenenfalls miteinander verbrückt sein. Vorzugsweise sind die Reste R¹ und R² bzw. R³ und R⁴ nicht miteinander verbrückt. Verbrückte Reste R¹ und R² bzw. R³ und R⁴ stehen vorzugsweise zusammen mit dem Teil der Phosphingruppe, an die sie gebunden sind für einen 5- bis 8-gliedrigen Heterocyclus, der gegebenenfalls zusätzlich ein-, zwei- oder dreifach mit Cycloalkyl, Aryl und/oder Hetaryl anelliert sein kann, wobei die anellierten Gruppen je einen, zwei oder drei Substituenten, ausgewählt unter Alkyl, Alkoxy, Halogen, Nitro, Cyano oder Carboxyl tragen können. Die Reste R¹ und R² bzw. R³ und R⁴ stehen dann gemeinsam z. B. für einen 2,2'-Binaphthylen- oder 2,3-Xylylen-Rest, der 1, 2 oder 3 Substituenten, ausgewählt unter Alkyl, Alkoxy oder Halogen, tragen kann. Alkyl steht dabei vorzugsweise für C₁-C₄-Alkyl. Alkoxy steht dabei vorzugsweise für C₁-C₄-Alkoxy und insbesondere für Methoxy. Halogen steht insbesondere für Fluor, Chlor oder Brom.

Bevorzugt sind Phosphinliganden der allgemeinen Formel I', worin
- R¹ und R³: unabhängig voneinander für Alkyl, Cycloalkyl, Aryl oder Hetaryl stehen, welche je einen, zwei oder drei Substituenten, die ausgewählt sind unter Alkyl, Cycloalkyl und Aryl, tragen können,
- R² und R⁴: unabhängig voneinander für Alkyl, Cycloalkyl, Aryl oder Hetaryl stehen, wobei die Aryl- und Hetarylgruppen je einen, zwei oder drei Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE³E⁴, tragen können, wobei E³ und E⁴ gleich oder verschieden sein können und für Alkyl, Cycloalkyl oder Aryl stehen.

Bevorzugt stehen in der Formel I' die Reste R¹, R², R³ und R⁴ unabhängig voneinander für gegebenenfalls substituiertes Alkyl oder Aryl, insbesondere für gegebenenfalls substituiertes Phenyl, 1-Naphthyl oder 2-Naphthyl. vorzugsweise stehen R¹, R², R³ und R⁴ für Phenylreste, die gegebenenfalls 1 oder 2 Substituenten, die ausgewählt sind unter Alkyl, Alkoxy, Halogen, Trifluormethyl, Nitro, Cyano, Alkoxycarbonyl oder Carboxyl, tragen können.

Nach einer bevorzugten Ausführungs form steht die Gruppe X für eine nichtaromatische, 3- bis 8-gliedrige, vorzugsweise 5- bis 7-gliedrige, verbrückende Gruppe, wie zuvor definiert, die Teil eines kondensierten Ringsystems mit 1, 2 oder 3 weiteren Ringen, besonders bevorzugt 1 oder 2 weiteren Ringen und insbesondere einem weiteren Ring sein kann.

Bei ankondensierten Ringen der Gruppe X handelt es sich bevorzugt um Aryl, insbesondere um Benzol oder Naphthalin. Ankondensierte Benzolringe sind vorzugsweise unsubstituiert oder weisen 1, 2 oder 3, insbesondere 1 oder 2 Substituenten auf, die ausgewählt sind unter Alkyl, Alkoxy, Halogen, Trifluormethyl, Nitro, Carboxyl, Alkoxycarbonyl und Cyano. Ankondensierte Naphthaline sind vorzugsweise unsubstituiert oder weisen im X nicht direkt ankondensierten Ring und/oder im direkt ankondensierten Ring jeweils 1, 2 oder 3, insbesondere 1 oder 2 der zuvor bei den ankondensierten Benzolringen genannten Substituenten auf. Ankondensierte Naphthaline, die im direkt ankondensierten Ring substituiert sind, weisen vorzugsweise einen Substituenten auf. Dieser steht dann bevorzugt für Alkyl, Alkoxy oder Alkoxycarbonyl. Bei den Substituenten der ankondensierten Aryle steht Alkyl vorzugsweise für C₁- bis C₄-Alkyl und insbesondere für Methyl, Isopropyl und tert.-Butyl. Alkoxy steht dabei vorzugsweise für C₁- bis C₄-Alkoxy und insbesondere für Methoxy. Alkoxycarbonyl steht vorzugsweise für C₁- bis C₄-Alkoxycarbonyl. Halogen steht dabei insbesondere für Fluor und Chlor.

Nach einer besonders bevorzugten Ausführungsform ist die verbrükkende Gruppe X nicht Teil eines kondensierten Ringsystems.

Die Reste D¹ und/oder D² können gewünschtenfalls, wie zuvor beschrieben, wenigstens einen ankondensierten Ring aufweisen, sofern dies ihre Kohlenstoffatomanzahl zulässt.

Besonders bevorzugt ist X ausgewählt unter Gruppen der Formeln II.a bis II.k 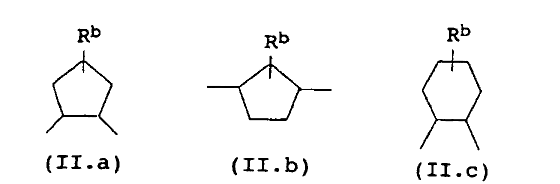 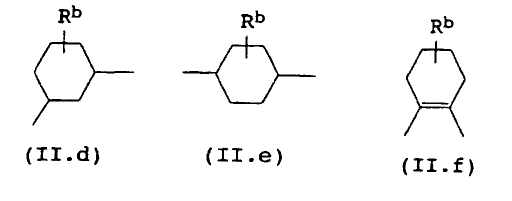 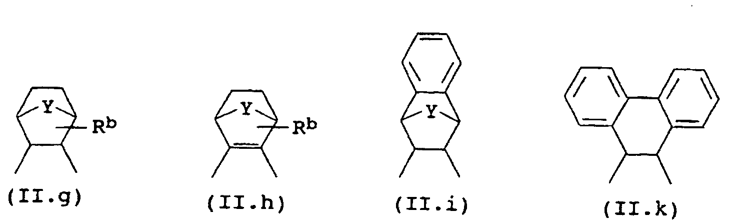 worin
- R^{b}: ausgewählt ist unter Alkyl, Cycloalkyl, Aryl, Alkoxy, Acyl, Carboxyl, Alkoxycarbonyl, Hydroxy, Nitro, Cyano, Trifluormethyl, Oxo und den Ketalen davon, oder NE¹E², wobei E¹ und E² gleich oder verschieden sein können und für Alkyl, Cycloalkyl oder Aryl stehen, und
- Y: für O, S, CH₂ oder einen Rest der Formeln III.1 bis III.4, wie zuvor definiert, steht.

Nach einer speziellen Ausführungsform steht R^{b} für einen, gegebenenfalls über eine Spacergruppe gebundenen, polymeren Träger. Dabei ist der Träger vorzugsweise ausgewählt unter Styrolhomo- und - copolymeren, insbesondere Styrol-Divinylbenzol-Copolymeren (Merrifield-Harzen), Polyamiden, aminomethylierten Polystyrolharzen etc. Geeignete Spacer umfassen Alkylenketten, die gegebenenfalls durch ein oder mehrere nicht benachbarte Heteroatome, wie O, S, NR^{x}, worin R^{x} für Wasserstoff, Alkyl, Cycloalkyl oder Aryl steht, unterbrochen sein können. Weiterhin können die Alkylenketten auch funktionelle Gruppen, wie Ester- und/oder Aminogruppen aufweisen.

Insbesondere ist X ausgewählt unter Gruppen der Formeln II.a bis II.h, worin R^{b} für Wasserstoff, Oxo oder ein Ketal, insbesondere das Neopentylketal, davon, steht und Y für O, S, CH₂, C₂H₄ oder C₂H₂ steht.

Wenn die Triarylreste, z. B. die Triarylphosphinreste, an gesättigte Kohlenstoffatome der Gruppe X gebunden sind, so können sie in Form der reinen cis- oder trans-Isomeren oder in Form eines Isomerengemisches vorliegen. Bevorzugt sind die cis-Isomeren. Besonders bevorzugt sind die cis-Isomeren, wenn X für eine 5- oder 6-gliedrige verbrückende Gruppe steht. Gute Ergebnisse bei der Hydroformylierung werden jedoch in der Regel auch mit trans-Isomeren erzielt.

Vorzugsweise sind die in dem erfindungsgemäßen Verfahren eingesetzten Liganden ausgewählt unter Liganden der Formeln IA bis IF 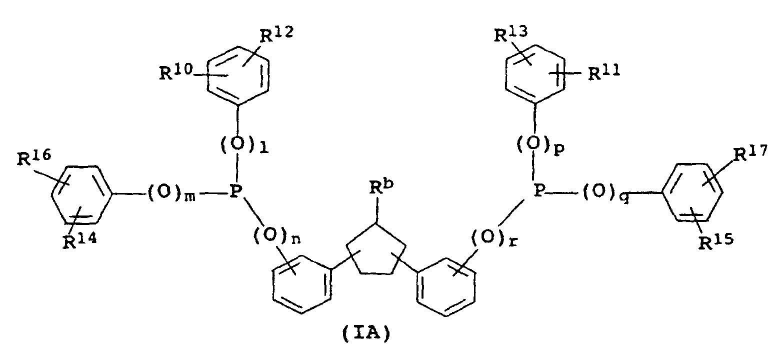 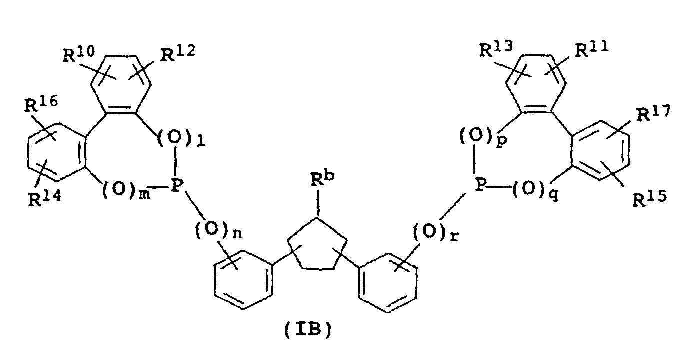 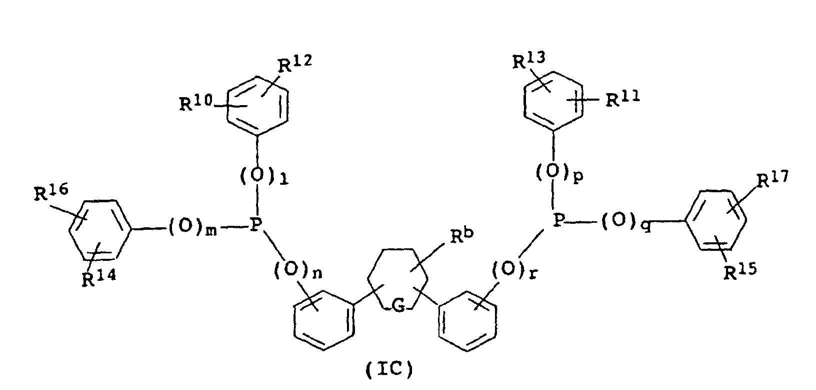 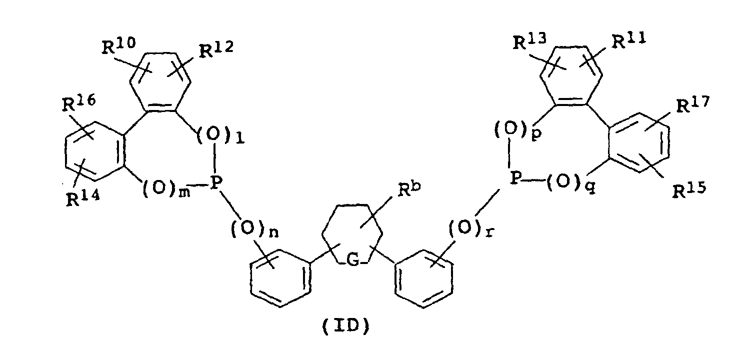 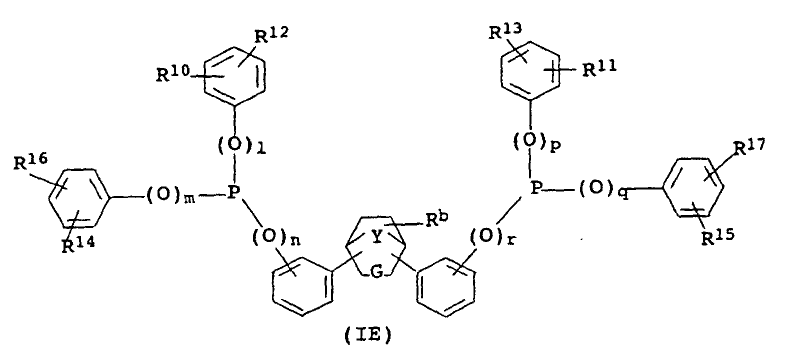 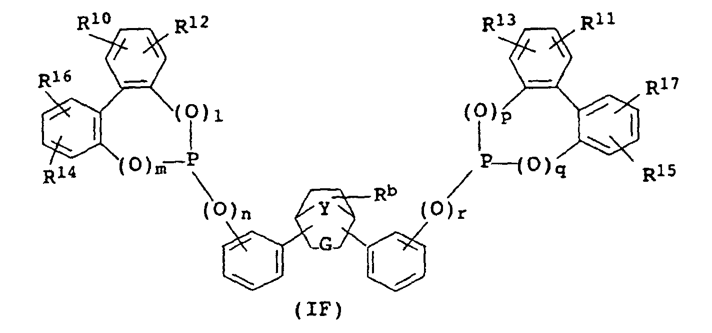 worin
- R¹⁰, R¹¹, R¹², R¹³, R¹⁴, R¹⁵, R¹⁶ und R¹⁷: unabhängig voneinander ausgewählt sind unter Wasserstoff, Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE³E⁴, wobei E³ und E⁴ gleich oder verschieden sein können und für Alkyl, Cycloalkyl oder Aryl stehen,
- l, m, n, p, q und r: unabhängig voneinander für 0 oder 1 stehen,
- R^{b}: in den Formeln IA und IB ausgewählt ist unter Wasserstoff, Alkyl, Oxo und den Ketalen davon und in den Formeln IC, ID, IE und IF ausgewählt ist unter Wasserstoff und Alkyl,
- G: für eine Einfachbindung, eine Doppelbindung oder ein Sauerstoffatom steht,
- Y: für O, S, CH₂ oder einen Rest der Formel III.1 bis III.4 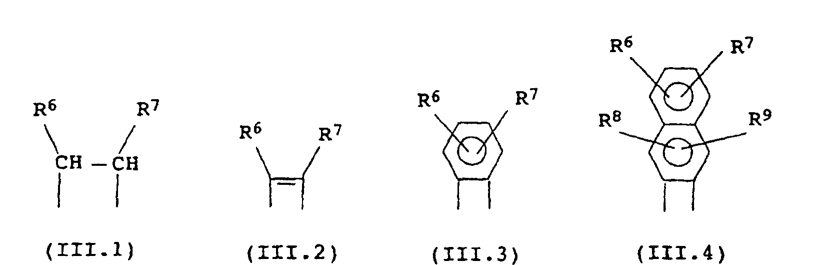 steht, worin
- R⁶, R⁷, R⁸ und R⁹: unabhängig voneinander für Wasserstoff, Alkyl, Cycloalkyl, Aryl, Alkoxy, Halogen, SO₃H, Sulfonat, NE¹E², Alkylen-NE¹E², Trifluormethyl, Nitro, Alkoxycarbonyl, Carboxyl oder Cyano stehen, wobei E¹ und E² unabhängig voneinander für Alkyl, Cycloalkyl oder Aryl stehen.

Vorzugsweise steht der Phosphinligand der allgemeinen Formel I' für eine Verbindung der Formel I.1 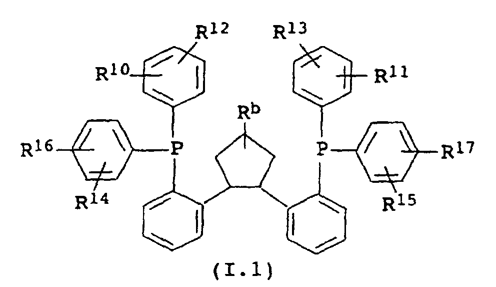 worin
- R¹⁰, R¹¹, R¹², R¹³, R¹⁴, R¹⁵, R¹⁶ und R¹⁷: unabhängig voneinander ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE³E⁴, wobei E³ und E⁴ gleich oder verschieden sein können und für Alkyl, Cycloalkyl oder Aryl stehen,
- R^{b}: wie zuvor definiert ist.

Vorzugsweise steht der Phosphinligand der allgemeinen Formel I' für eine Verbindung der Formel I.1, worin R¹⁰, R¹¹, R¹⁴ und R¹⁵ unabhängig voneinander für Wasserstoff oder C₁- bis C₄-Alkyl, bevorzugt Methyl, Ethyl, Isopropyl oder tert.-Butyl stehen und R¹², R¹³, R¹⁶ und R¹⁷ unabhängig voneinander für Wasserstoff, C₁- bis C₄-Alkyl, bevorzugt Methyl, Ethyl, Isopropyl oder tert.-Butyl, C₁bis C₄-Alkoxy, bevorzugt Methoxy, Fluor, Chlor, Nitro oder Trifluormethyl stehen.

Bevorzugt steht in der Formel I.1 der Rest R^{b} für Wasserstoff, Oxo oder ein Ketal, insbesondere das Neopentylketal davon.

Vorzugsweise steht der Phosphinligand der allgemeinen Formel I' für eine Verbindung der Formel I.2 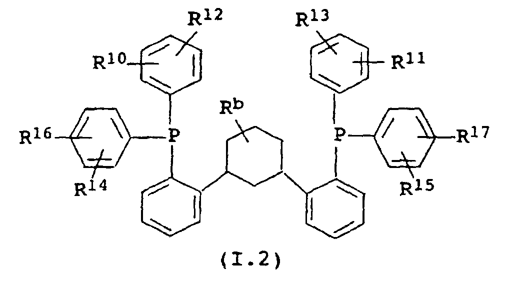 worin,
- R¹⁰, R¹¹, R¹², R¹³, R¹⁴, R¹⁵, R¹⁶, R¹⁷ und R^{b}: die zuvor bei der Formel I.1 angegebenen Bedeutungen besitzt.

Vorzugsweise steht der Phosphinligand der allgemeinen Formel I' für eine Verbindung der Formel I.3 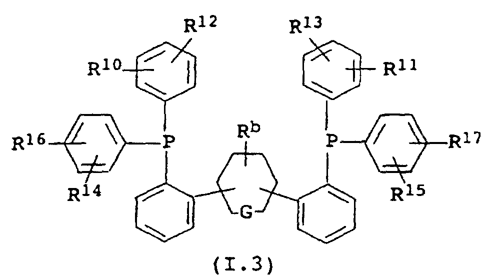 worin
- G: für Sauerstoff, eine Einfach- oder eine Doppelbindung steht, und
- R¹⁰, R¹¹, R¹², R¹³, R¹⁴, R¹⁵, R¹⁶, R¹⁷ und R^{b}: die zuvor bei der Formel I.1 angegebenen Bedeutungen besitzen.

Vorzugsweise steht der Phosphinligand der allgemeinen Formel I' für eine Verbindung der Formel I.4 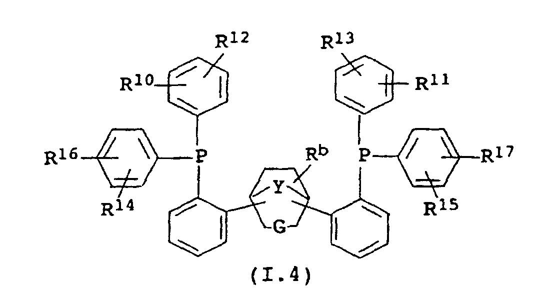 worin
- G: für Sauerstoff, eine Einfach- oder eine Doppelbindung steht,
- Y: für einen Rest der Formeln III.1 bis III.4, wie zuvor definiert, steht,
- R¹⁰, R^{11,} R¹², R¹³, R¹⁴, R¹⁵, R¹⁶, R¹⁷ und R^{b}: die zuvor bei der Formel I.1 angegebenen Bedeutungen besitzen.

Vorzugsweise steht in der Formel I.4 die Gruppe Y für O, S, CH₂, C₂H₄ oder C₂H₂.

Nach einer bevorzugten Ausführungsform des erfindungsgemäßen Verfahrens wird ein Hydroformylierungskatalysator eingesetzt, wobei der Phosphinligand der allgemeinen Formel I' ausgewählt ist unter Liganden der Formel I.a bis I.e 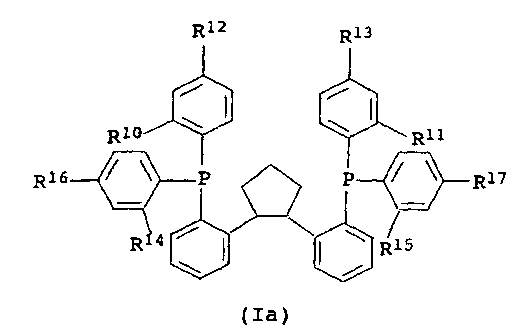 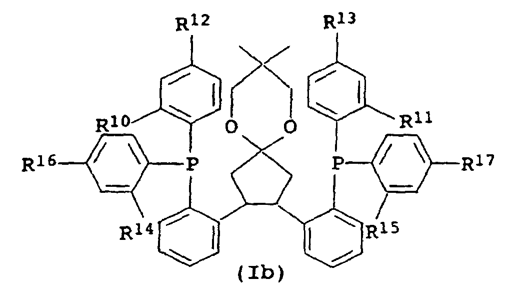 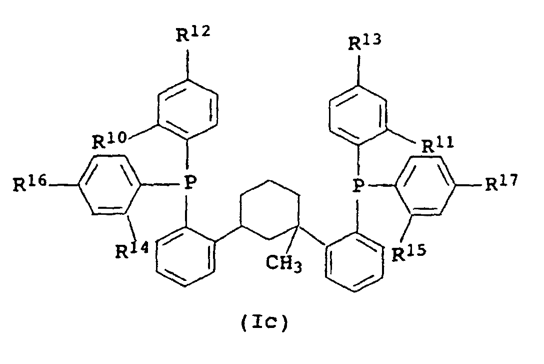 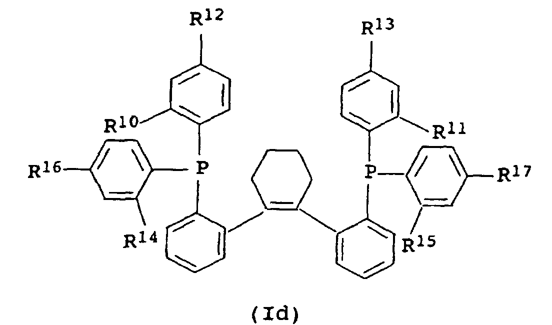 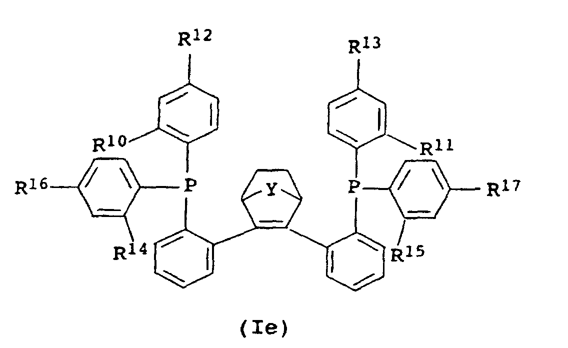 wobei in den Formeln Ia bis Ie die Substituenten R¹⁰, R¹¹, R¹², R¹³, R¹⁴, R¹⁵, R¹⁶ und R¹⁷ die folgenden Bedeutungen besitzen:

| R¹⁰ | R¹¹ | R¹² | R¹³ | R¹⁴ | R¹⁵ | R¹⁶ | R¹⁷ |
|---|---|---|---|---|---|---|---|
| H | H | H | H | H | H | H | H |
| CH₃ | CH₃ | H | H | H | H | H | H |
| CH₃ | CH₃ | H | H | CH₃ | CH₃ | H | H |
| CH₃ | CH₃ | CH₃ | CH₃ | H | H | H | H |
| CH₃ | CH₃ | CH₃ | CH₃ | CH₃ | CH₃ | H | H |
| CH₃ | CH₃ | CH₃ | CH₃ | CH₃ | CH₃ | CH₃ | CH₃ |

und in der Formel Ie Y für O, S, CH₂ oder einen Rest der Formeln III.1 bis III.4 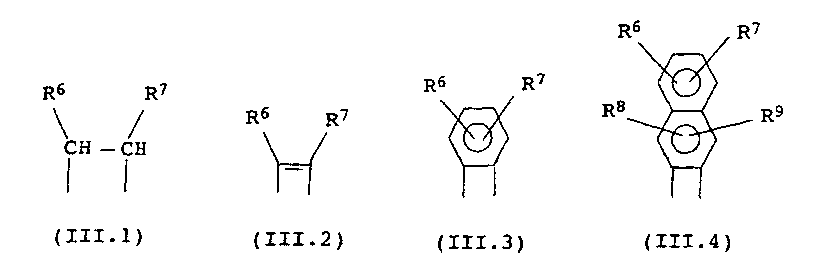 steht, worin
R⁶, R⁷, R⁸ und R⁹ unabhängig voneinander für Wasserstoff, Alkyl, Cycloalkyl, Aryl, Alkoxy, Halogen, SO₃H, Sulfonat, NE¹E², Alkylen-NE¹E², Trifluormethyl, Nitro, Alkoxycarbonyl, Carboxyl oder Cyano stehen, wobei E¹ und E² unabhängig voneinander für Alkyl, Cycloalkyl oder Aryl stehen.

Ein weiterer Gegenstand der Erfindung sind Verbindungen der allgemeinen Formel I 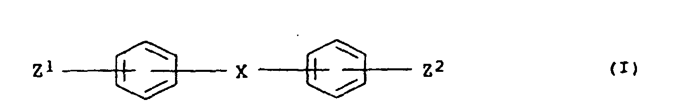 worin
- Z¹ und Z²: unabhängig voneinander für mindestens ein Phosphor-, Arsen- oder Antimonatom aufweisende Reste stehen, wobei an das Phosphor-, Arsen- oder Antimonatom jeweils mindestens zwei gegebenenfalls substituierte Heteroatome, die ausgewählt sind unter O, S und NR^{c}, direkt gebunden sind, wobei R^{c} für Wasserstoff, Alkyl, Cycloalkyl oder Aryl steht,und
Verbindungen der allgemeinen Formel I' 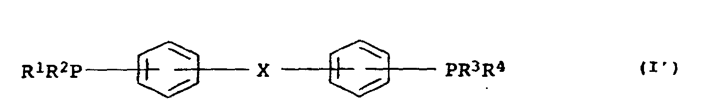 worin
- R¹, R², R³ und R⁴: unabhängig voneinander für Alkyl, Cycloalkyl, Aryl oder Hetaryl stehen, wobei die Aryl- und Hetarylgruppen je einen, zwei oder drei Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE¹E², tragen können, wobei E¹ und E² gleich oder verschieden sein können und für Alkyl, Cycloalkyl oder Aryl stehen,
wobei in den Verbindungen der Formeln I und I'
- X: ausgewählt ist unter Gruppen der Formeln II.a bis II.k 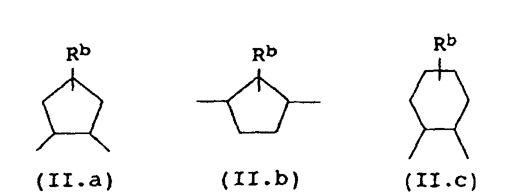 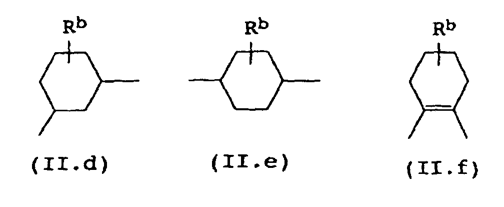 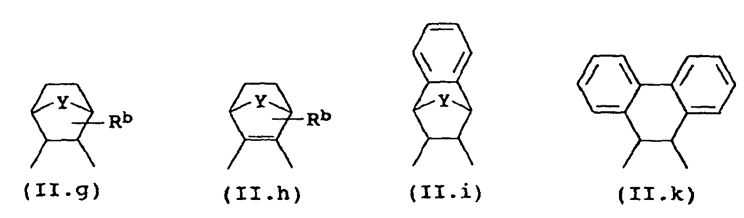
worin
- R^{b}: ausgewählt ist unter Alkyl, Cycloalkyl, Aryl, Alkoxy, Acyl, Carboxyl, Alkoxycarbonyl, Hydroxy, Nitro, Cyano, Trifluormethyl, Oxo und den Ketalen davon, oder NE¹E², wobei E¹ und E² gleich oder verschieden sein können und für Alkyl, Cycloalkyl oder Aryl stehen, und
- Y: wie in Anspruch 1 definiert ist.

Bezüglich bevorzugter Ausführungsformen der Verbindungen der Formeln I und I' wird auf die vorherigen Ausführungen zu den in dem erfindungsgemäßen Hydroformylierungsverfahren eingesetzten Liganden der Formeln I und I' verwiesen.

Ein weiterer Gegenstand der Erfindung ist ein Katalysator, umfassend wenigstens einen Komplex eines Metalls der VIII. Nebengruppe mit mindestens einem erfindungsgemäßen Liganden, wie zuvor definiert.

Die erfindungsgemäßen Katalysatoren können einen oder mehrere der Liganden der allgemeinen Formeln I und I' aufweisen. Zusätzlich zu den zuvor beschriebenen Liganden der allgemeinen Formeln I und I' können sie noch wenigstens einen weiteren Liganden der ausgewählt ist unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und

Heteroaromaten, Ethern, PF₃, Phospholen, Phosphabenzolen sowie ein-, zwei- und mehrzähnigen Phosphin-, Phosphinit-, Phosphonit-, Phosphoramidit und Phosphitliganden aufweisen. Diese weiteren Liganden können 1-, 2- oder mehrzähnig sein und an das Metall der VIII. Nebengruppe koordinieren. Geeignete weitere phosphorhaltige Liganden sind z. B. die zuvor als Stand der Technik beschriebenen Phosphinliganden.

Bevorzugt handelt es sich bei dem Metall der VIII. Nebengruppe um Cobalt, Ruthenium, Rhodium, Nickel, Palladium, Platin, Osmium oder Iridium und insbesondere um Cobalt, Rhodium, Ruthenium und Iridium.

Zur Herstellung der erfindungsgemäß eingesetzten und erfindungsgemäßen Verbindungen der Formel I' kann man, gemäß dem folgenden Schema 1, z. B. eine Verbindung der Formel IV, worin Z³ und Z⁴ für Halogen, vorzugsweise Chlor, stehen zunächst lithiieren. Das dabei gebildete Zwischenprodukt wird dann mit einer Verbindung, die am Phosphoratom ein Halogenatom, vorzugsweise ein Chloratom trägt, zur Verbindung der Formel I' umgesetzt. Für den Fall, dass in der Formel (I') R¹ = R³ und R² = R⁴ ist, können gemäß Variante 2a) zwei Äquivalente R¹R²P-Hal in einer einstufigen Reaktion mit einem Äquivalent einer Verbindung der Formel V umgesetzt werden. Ansonsten wird zunächst ein Äquivalent einer Verbindung der Formel R¹R²P-Hal mit einem Äquivalent einer Verbindung der Formel V umgesetzt und nach Bildung des Monokondensationsproduktes wird eine zweite Verbindung der Formel R³R⁴P-Hal zugegeben und weiter zu dem Phosphin der Formel (I') umgesetzt.

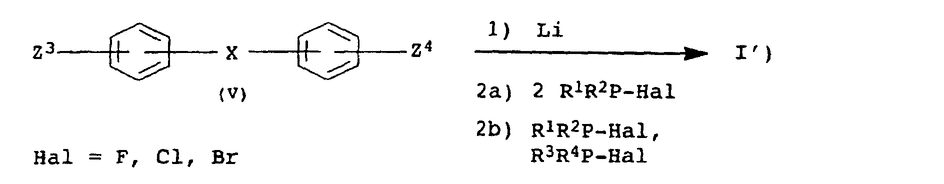

Die Herstellung von Verbindungen der Formel I', worin X für eine 1,2-trans-Cyclopentylengruppe steht, kann auch analog der von N. Fukuda et al. in Tetra vchedron Letters, Vol. 31, Nr. 49, S. 7185 bis 7188 (1990) beschriebenen Umsetzung durchgeführt werden, auf die hier in vollem Umfang Bezug genommen wird.

Die Herstellung von Verbindungen der Formel V kann ausgehend von kommerziell erhältlichen Edukten erfolgen.

So gelangt man z.B. ausgehend von Halogenzimtsäureestern durch Umsetzung mit Natrium zu 2-Carbalkoxy-cis/trans-3,4-bis(halogenphenyl)cyclopentanonen, die, gegebenenfalls nach Auftrennung in die Isomeren, durch Verseifung und anschließende Decarboxylierung in die entsprechenden Cyclopentanone (VI) überführt werden können. Die Reduktion dieser Cyclopentanone zu den entsprechenden Cyclopentanen kann in üblicher Weise erfolgen. Dazu gehört z.B. die Überführung in die Hydrazone und deren anschließende Zersetzung in Gegenwart einer starken Base (Wolff-Kishner-Reduktion), wobei die Bildung des Hydrazons auch in Gegenwart eines hochsiedenden Glykols erfolgen kann (Huang-Minlon-Reduktion), die Umsetzung mit Zink-Amalgam und konzentrierter Salzsäure (Clemmensen-Reduktion) sowie die Reduktion mit aktiviertem Zink. Geht man bei der Reduktion von reinen cis- oder trans-Isomeren aus, so erhält man in der Regel auch überwiegend die entsprechenden Cyclopentan-Isomeren. Lediglich bei der Reduktion von cis-Cyclopentanonen nach der Huang-Minlon-Variante wird auch das trans-Isomer in nennenswerten Mengen erhalten (Schema 2). Die Darstellung des reinen cis-Isomeren gelingt beispielsweise durch Überführung des Cyclopentanons in das entsprechende Tosylhydrazon und anschließende Reduktion mit Catecholboran.

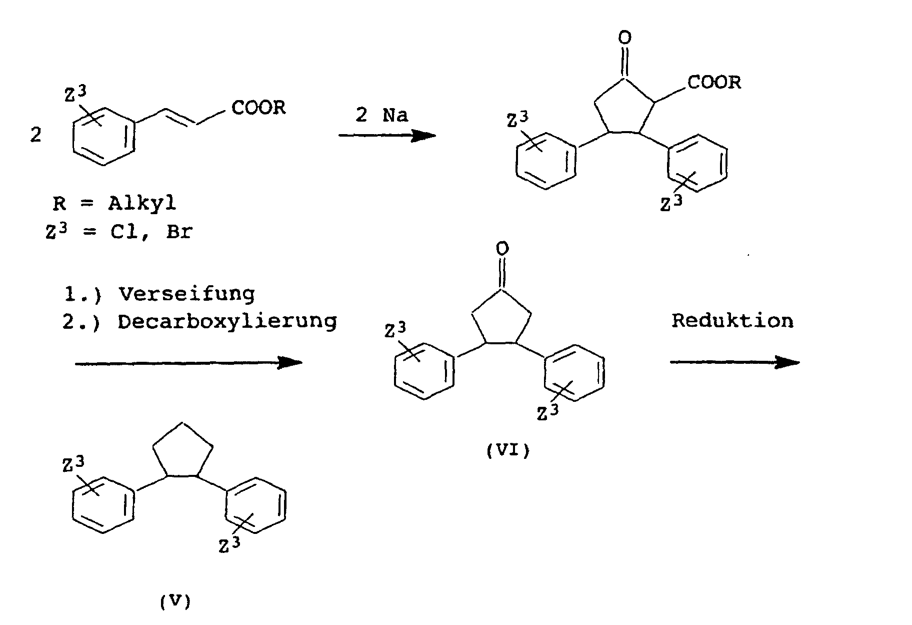

Ausgehend von den Cyclopentanonen (VI) kann auch eine weitere Derivatisierung erfolgen, wie die Reduktion zu den entsprechenden Alkoholen oder die Acetalisierung, wie z. B. mit Neopentylglykol. Ausgehend von den Cyclopentanonen kann auch eine ein- oder mehrmalige Ringerweiterung zum Aufbau von verbrückenden Gruppen mit 6- bis 8-gliedrigen Ringen erfolgen. Derartige Ringerweiterungsreaktionen sind dem Fachmann bekannt und werden z. B. in Carey, Sundberg, Advanced Organic Chemistry, 2nd ed., Teil B, S. 455-458 beschrieben. Darauf, und auf die dort zitierten Literaturstellen wird hier Bezug genommen.

Die Herstellung von Verbindungen der Formel (V) kann auch durch geeignete Kupplungsreaktionen, ausgehend von (Halogenphenyl)cyclo-C₅-C₈-alkanonen erfolgen, bei denen die Ketofunktion des Cycloalkanonrings vorzugsweise in der Nachbarposition zur Halogenphenylgruppe steht. Geeignete (Halogenphenyl)cyclo-C₅-C₈-alkanone sind z. B. 2-(o-Chlorphenyl)cyclohexanon, 2-(o-Chlorphenyl)cycloheptanon, 2-(o-Chlorphenyl)cyclooctanon, 3-(o-Chlorphenyl)bicyclo[2.2.1]heptan-2-on, etc. Eine geeignete Kupplung umfasst die Überführung des Alkanons in das entsprechende Enoltriflat und anschließende Umsetzung des Enoltriflats mit (Halogenphenyl)boronsäure, z. B. (o-Chlorphenyl)boronsäure (Suzuki-Kupplung). Zur Überführung des Alkanons in das Enoltriflat kann man dieses zunächst mit einer starken Base, wie z. B. Natriumhydrid, zum Enolat-Anion und dieses dann anschließend z. B. mit N,N-Bis(trifluormethansulfonyl)anilin zum Enoltriflat umsetzen. 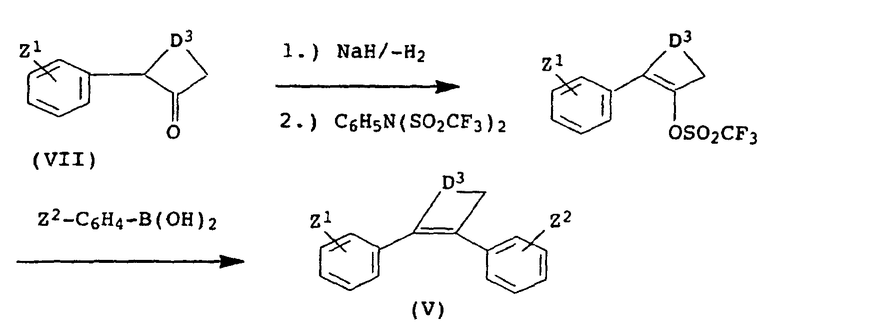
- Z¹, Z² =: F, Cl, Br
- D³: ist eine C₂- bis C₆-Alkylenbrücke, die eine zwei oder drei Doppelbindungen und/oder einen, zwei, drei oder vier der zuvor genannten Substituenten aufweisen kann und/oder die durch eine Gruppe -Y- überbrückt sein kann, wobei Y für O, S, CR^{x}R^{y} (mit R^{x} und R^{y} = H, Alkyl, Cycloalkyl, Aryl oder Hetaryl) oder einen Rest der Formeln III.1 bis III.4, wie zuvor definiert, steht.

Im Allgemeinen werden unter Hydroformylierungsbedingungen aus den jeweils eingesetzten Katalysatoren oder Katalysatorvorstufen katalytisch aktive Spezies der allgemeinen Formel HₓM_{y}(CO)_{z}L_{q} gebildet, worin M für ein Metall der VIII. Nebengruppe, L für eine erfindungsgemäße phosphorhaltige Verbindung und q, x, y, z für ganze Zahlen, abhängig von der Wertigkeit und Art des Metalls sowie der Bindigkeit des Liganden L, stehen. Vorzugsweise stehen z und q unabhängig voneinander mindestens für einen Wert von 1, wie z. B. 1, 2 oder 3. Die Summe aus z und q steht bevorzugt für einen Wert von 2 bis 5. Dabei können die Komplexe gewünschtenfalls zusätzlich noch mindestens einen der zuvor beschriebenen weiteren Liganden aufweisen.

Nach einer bevorzugten Ausführungsform werden die Hydroformylierungskatalysatoren in situ, in dem für die Hydroformylierungsreaktion eingesetzten Reaktor, hergestellt. Gewünschtenfalls können die erfindungsgemäßen Katalysatoren jedoch auch separat hergestellt und nach üblichen Verfahren isoliert werden. Zur in situ-Herstellung der erfindungsgemäßen Katalysatoren kann man z. B. wenigstens eine Verbindung der allgemeinen Formel I, eine Verbindung oder einen Komplex eines Metalls der VIII. Nebengruppe, gegebenenfalls wenigstens einen weiteren zusätzlichen Liganden und gegebenenfalls ein Aktivierungsmittel in einem inerten Lösungsmittel unter den Hydroformylierungsbedingungen umsetzen.

Geeignete Rhodiumverbindungen oder -komplexe sind z. B. Rhodium(II)- und Rhodium(III)-salze, wie Rhodium(III)-chlorid, Rhodium(III)-nitrat, Rhodium(III)-sulfat, Kalium-Rhodiumsulfat, Rhodium(II)- bzw. Rhodium(III)-carboxylat, Rhodium(II)- und Rhodium(III)-acetat, Rhodium(III)-oxid, Salze der Rhodium(III)-säure, Trisammoniumhexachlororhodat(III) etc. Weiterhin eignen sich Rhodiumkomplexe, wie Rhodiumbiscarbonylacetylacetonat, Acetylacetonatobisethylenrhodium(I) etc. Vorzugsweise werden Rhodiumbiscarbonylacetylacetonat oder Rhodiumacetat eingesetzt.

Ebenfalls geeignet sind Rutheniumsalze oder -verbindungen. Geeignete Rutheniumsalze sind beispielsweise Ruthenium(III)chlorid, Ruthenium(IV)-, Ruthenium(VI)- oder Ruthenium(VIII)oxid, Alkalisalze der Rutheniumsauerstoffsäuren wie K₂RuO₄ oder KRuO₄ oder Komplexverbindungen, wie z. B. RuHCl(CO)(PPh₃)₃. Auch können die Metallcarbonyle des Rutheniums wie Trisrutheniumdodecacarbonyl oder Hexarutheniumoctadecacarbonyl, oder Mischformen, in denen CO teilweise durch Liganden der Formel PR₃ ersetzt sind, wie Ru(CO)₃(PPh₃)₂, im erfindungsgemäßen Verfahren verwendet werden.

Geeignete Cobaltverbindungen sind beispielsweise Cobalt(II)chlorid, Cobalt(II)sulfat, Cobalt(II)carbonat, Cobalt(II)nitrat, deren Amin- oder Hydratkomplexe, Cobaltcarboxylate, wie Cobaltacetat, Cobaltethylhexanoat, Cobaltnaphthanoat, sowie der Cobalt-Caprolactamat-Komplex. Auch hier können die Carbonylkomplexe des Cobalts wie Dicobaltoctacarbonyl, Tetracobaltdodecacarbonyl und Hexacobalthexadecacarbonyl eingesetzt werden.

Die genannten und weitere geeignete Verbindungen des Cobalts, Rhodiums, Rutheniums und Iridiums sind im Prinzip bekannt und in der Literatur hinreichend beschrieben oder sie können vom Fachmann analog zu den bereits bekannten Verbindungen hergestellt werden.

Geeignete Aktivierungsmittel sind z. B. Brönsted-Säuren, LewisSäuren, wie z. B. BF₃, AlCl₃, ZnCl₂, und Lewis-Basen.

Als Lösungsmittel werden vorzugsweise die Aldehyde eingesetzt, die bei der Hydroformylierung der jeweiligen Olefine entstehen, sowie deren höher siedende Folgereaktionsprodukte, z. B. die Produkte der Aldolkondensation. Ebenfalls geeignete Lösungsmittel sind Aromaten, wie Toluol und Xylole, Kohlenwasserstoffe oder Gemische von Kohlenwasserstoffen, auch zum Verdünnen der oben genannten Aldehyde und der Folgeprodukte der Aldehyde. Weitere Lösungsmittel sind Ester aliphatischer Carbonsäuren mit Alkanolen, beispielsweise Essigester oder Texanol™, Ether wie tert.-Butylmethylether und Tetrahydrofuran. Bei ausreichend hydrophilisierten Liganden können auch Alkohole, wie Methanol, Ethanol, n-Propanol, Isopropanol, n-Butanol, Isobutanol, Ketone, wie Aceton und Methylethylketon etc., eingesetzt werden. Ferner können als Lösungsmittel auch sogenannten "Ionic Liquids" verwendet werden. Hierbei handelt es sich um flüssige Salze, beispielsweise um N,N'-Dialkylimidzoliumsalze wie die N-Butyl-N'-methylimidazoliumsalze, Tetraalkylammoniumsalze wie die Tetra-n-butylammoniumsalze, N-Alkylpyridiniumsalze wie die n-Butylpyridiniumsalze, Tetraalkylphosphoniumsalze wie die Trishexyl(tetradecyl)phosphoniumsalze, z. B. die Tetrafluoroborate, Acetate, Tetrachloroaluminate, Hexafluorophosphate, Chloride und Tosylate.

Weiterhin ist es möglich die Umsetzungen auch in Wasser oder wässrigen Lösungsmittelsystemen, die neben Wasser ein mit Wasser mischbares Lösungsmittel, beispielsweise einen Alkohol wie Methanol, Ethanol, n-Propanol, Isopropanol, n-Butanol, Isobutanol, ein Keton wie Aceton und Methylethylketon oder ein anderes Lösungsmittel enthalten. Zu diesem Zweck setzt man Liganden der Formel I ein, die mit polaren Gruppen, beispielsweise ionischen Gruppen wie SO₃Me, CO₂Me mit Me = Na, K oder NH₄ oder wie N(CH₃)₃⁺ modifiziert sind. Die Umsetzungen erfolgen dann im Sinne einer Zweiphasenkatalyse, wobei der Katalysator sich in der wässrigen Phase befindet und Einsatzstoffe und Produkte die organische Phase bilden. Auch die Umsetzung in den "Ionic Liquids" kann als Zweiphasenkatalyse ausgestaltet sein.

Das Molmengenverhältnis von phosphorhaltigem Ligand zu Metall der VIII. Nebengruppe liegt im Allgemeinen in einem Bereich von etwa 1:1 bis 1000:1.

Als Substrate für das erfindungsgemäße Hydroformylierungsverfahren kommen prinzipiell alle Verbindungen in Betracht, welche eine oder mehrere ethylenisch ungesättigte Doppelbindungen enthalten. Dazu zählen z. B. Olefine, wie α-Olefine, interne geradkettige und interne verzweigte Olefine. Geeignete α-Olefine sind z. B. Ethylen, Propen, 1-Buten, 1-Penten, 1-Hexen, 1-Hepten, 1-Octen, 1-Nonen, 1-Decen, 1-Undecen, 1-Dodecen etc.

Geeignete geradkettige interne Olefine sind vorzugsweise C₄- bis C₂₀-Olefine, wie 2-Buten, 2-Penten, 2-Hexen, 3-Hexen, 2-Hepten, 3-Hepten, 2-Octen, 3-Octen, 4-Octen etc.

Geeignete verzweigte, interne Olefine sind vorzugsweise C₄- bis C₂₀-Olefine, wie 2-Methyl-2-Buten, 2-Methyl-2-Penten, 3-Methyl-2-Penten, verzweigte, interne Hepten-Gemische, verzweigte, interne Octen-Gemische, verzweigte, interne Nonen-Gemische, verzweigte, interne Decen-Gemische, verzweigte, interne Undecen-Gemische, verzweigte, interne Dodecen-Gemische etc.

Geeignete zu hydroformylierende Olefine sind weiterhin C₅- bis C₈-Cycloalkene, wie Cyclopenten, Cyclohexen, Cyclohepten, Cycloocten und deren Derivate, wie z. B. deren C₁- bis C₂₀-Alkylderivate mit 1 bis 5 Alkylsubstituenten. Geeignete zu hydroformylierende Olefine sind weiterhin Vinylaromaten, wie Styrol, α-Methylstyrol, 4-Isobutylstyrol etc. Geeignete zu hydroformylierende Olefine sind weiterhin α,β-ethylenisch ungesättigte Mono- und/oder Dicarbonsäuren, deren Ester, Halbester und Amide, wie Acrylsäure, Methacrylsäure, Maleinsäure, Fumarsäure, Crotonsäure, Itaconsäure, 3-Pentensäuremethylester, 4-Pentensäuremethylester, Ölsäuremethylester, Acrylsäuremethylester, Methacrylsäuremethylester, ungesättigte Nitrile, wie 3-Pentennitril, 4-Pentennitril, Acrylnitril, Vinylether, wie Vinylmethylether, Vinylethylether, Vinylpropylether etc., C₁- bis C₂₀-Alkenole, -Alkendiole und -Alkadienole, wie 2,7-Octadienol-1. Geeignete Substrate sind weiterhin Di- oder Polyene mit isolierten oder konjugierten Doppelbindungen. Dazu zählen z. B. 1,3-Butadien, 1,4-Pentadien, 1,5-Hexadien, 1,6-Heptadien, 1,7-Octadien, Vinylcyclohexen, Dicyclopentadien, 1,5,9-Cyclooctatrien sowie Butadienhomo- und -copolymere.

Bevorzugt ist ein Verfahren, das dadurch gekennzeichnet ist, dass der Hydroformylierungskatalysator in situ hergestellt wird, wobei man mindestens eine Verbindung der Formel I, eine Verbindung oder einen Komplex eines Metalls der VIII. Nebengruppe und gegebenenfalls ein Aktivierungsmittel in einem inerten Lösungsmittel unter den Hydroformylierungsbedingungen zur Reaktion bringt.

Die Hydroformylierungsreaktion kann kontinuierlich, semikontinuierlich oder diskontinuierlich erfolgen.

Geeignete Reaktoren für die kontinuierliche Umsetzung sind dem Fachmann bekannt und werden z. B. in Ullmanns Enzyklopädie der technischen Chemie, Bd. 1, 3. Aufl., 1951, S. 743 ff. beschrieben.

Geeignete druckfeste Reaktoren sind dem Fachmann ebenfalls bekannt und werden z. B. in Ullmanns Enzyklopädie der technischen Chemie, Bd. 1, 3. Auflage, 1951, S. 769 ff. beschrieben. Im Allgemeinen wird für das erfindungsgemäße Verfahren ein Autoklav verwendet, der gewünschtenfalls mit einer Rührvorrichtung und einer Innenauskleidung versehen sein kann.

Die Zusammensetzung des im erfindungsgemäßen Verfahren eingesetzten Synthesegases aus Kohlenmonoxid und Wasserstoff kann in weiten Bereichen variieren. Das molare Verhältnis von Kohlenmonoxid und Wasserstoff beträgt in der Regel etwa 5:95 bis 70:30, bevorzugt etwa 40:60 bis 60:40. Insbesondere bevorzugt wird ein molares Verhältnis von Kohlenmonoxid und Wasserstoff im Bereich von etwa 1:1 eingesetzt.

Die Temperatur bei der Hydroformylierungsreaktion liegt im Allgemeinen in einem Bereich von etwa 20 bis 180 °C, bevorzugt etwa 50 bis 150 °C. Die Reaktion wird in der Regel bei dem Partialdruck des Reaktionsgases bei der gewählten Reaktionstemperatur durchgeführt. Im Allgemeinen liegt der Druck in einem Bereich von etwa 1 bis 700 bar, bevorzugt 1 bis 600 bar, insbesondere 1 bis 300 bar. Der Reaktionsdruck kann in Abhängigkeit von der Aktivität des eingesetzten erfindungsgemäßen Hydroformylierungskatalysators variiert werden. Im Allgemeinen erlauben die erfindungsgemäßen Katalysatoren auf Basis von phosphorhaltigen Verbindungen eine Umsetzung in einem Bereich niedriger Drücke, wie etwa im Bereich von 1 bis 100 bar.

Die erfindungsgemäßen Hydroformylierungskatalysatoren lassen sich nach üblichen, dem Fachmann bekannten Verfahren vom Austrag der Hydroformylierungsreaktion abtrennen und können im Allgemeinen erneut für die Hydroformylierung eingesetzt werden.

Die zuvor beschriebenen, erfindungsgemäßen Katalysatoren, die chirale Verbindungen der allgemeinen Formel I umfassen, eignen sich zur enantioselektiven Hydroformylierung. Die zuvor beschriebenen Katalysatoren können auch in geeigneter Weise, z. B. durch Anbindung über als Ankergruppen geeignete funktionelle Gruppen, Adsorption, Pfropfung, etc. an einen geeigneten Träger, z. B. aus Glas, Kieselgel, Kunstharzen etc., immobilisiert werden. Sie eignen sich dann auch für einen Einsatz als Festphasenkatalysatoren.

Die Hydroformylierungsaktivität von Katalysatoren auf Basis von Liganden der Formel I ist überraschenderweise in der Regel höher als die Isomerisierungsaktivität bezüglich der Bildung mittenständiger Doppelbindungen. Vorteilhafterweise zeigen die erfindungsgemäßen und die erfindungsgemäß eingesetzten Katalysatoren bei der Hydroformylierung von α-Olefinen eine hohe Selektivität zugunsten der α-Aldehyde bzw. -Alkohole. Zudem werden im Allgemeinen auch bei der Hydroformylierung von internen linearen Olefinen (isomerisierende Hydroformylierung) gute Ausbeuten an α-Aldehyden bzw. -Alkoholen und insbesondere auch an n-Aldehyden bzw. - Alkoholen erhalten. Weiterhin weisen diese Katalysatoren im Allgemeinen eine hohe Stabilität unter den Hydroformylierungsbedingungen auf, so dass mit Ihnen in der Regel längere Katalysatorstandzeiten erzielt werden, als mit aus dem Stand der Technik bekannten Katalysatoren auf Basis herkömmlicher Chelatliganden. Vorteilhafter Weise zeigen die erfindungsgemäßen und erfindungsgemäß eingesetzten Katalysatoren weiterhin eine hohe Aktivität, so dass in der Regel die entsprechenden Aldehyde, bzw. Alkohole in guten Ausbeuten erhalten werden. Bei der Hydroformylierung von α-Olefinen sowie von innenständigen, linearen Olefinen zeigen Sie zudem eine sehr geringe Selektivität zum Hydrierprodukt des eingesetzten Olefins.

Die Erfindung wird anhand der folgenden, nicht einschränkenden Beispiele näher erläutert.

### Beispiele

Zur Hydroformylierung wurden die folgenden Liganden eingesetzt:

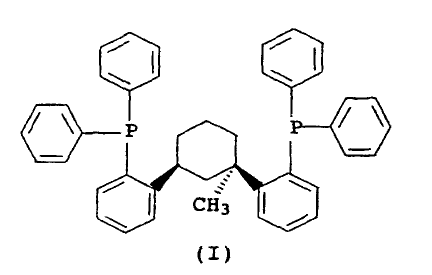

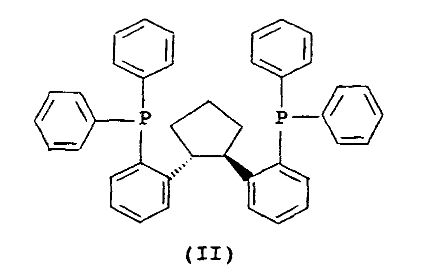

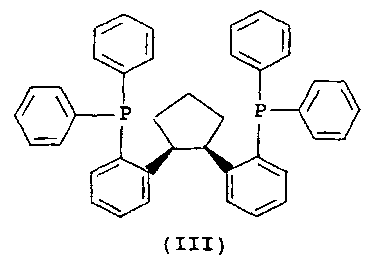

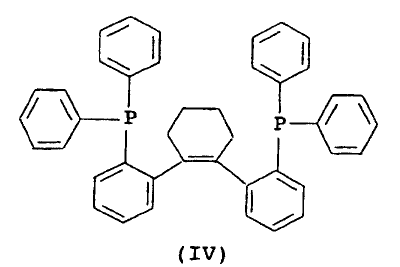

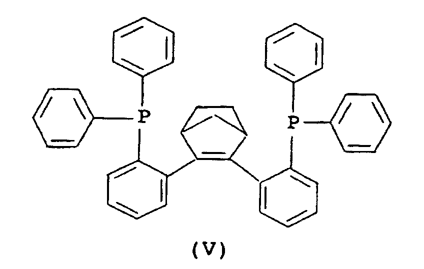

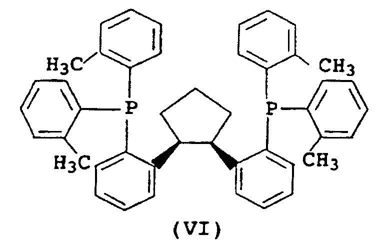

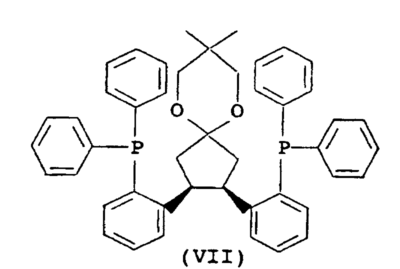

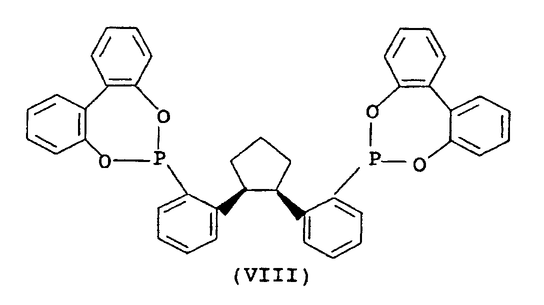

### I. Herstellung der Liganden

### I.1 Herstellung von Ligand II

### Darstellung von 2-Carbmethoxy-cis/trans-3,4-bis(o-chlorphenyl)cyclopentanon

3,2 g fein disperser Natrium-Sand werden in 200 ml absolutem THF suspendiert und über einen Tropftrichter innerhalb von 1,5 Stunden mit einer Lösung von 20,18 g (102,6 mmol) o-Chlor-trans-zimtsäuremethylester in 40 ml absolutem THF versetzt, wobei sich die Reaktionssuspension tief rotbraun verfärbt. Nach beendeter Zugabe (ca. 1 Stunde) erhitzt man 5 Stunden unter Rückflussbedingungen und rührt über Nacht. Die Lösungen werden auf dem Eisbad auf 0 °C abgekühlt und unter heftigem Rühren jeweils mit 80 ml 30%iger H₂SO₄-Lösung versetzt. Zur besseren Phasentrennung wird mit 100 ml Ether versetzt. Die abgetrennte wässrige Phase wird dreimal mit jeweils 150 ml Ether ausgeschüttelt. Die vereinigten organischen Phasen werden mit gesättigter NaHCO₃- und NaCl-Lösung gewaschen und über MgSO₄ getrocknet. Nach dem Entfernen des Lösungsmittels am Rotationsverdampfer nimmt man das gelblich-braune Öl in 250 ml Ether auf und lässt es zur Kristallisation des trans-Isomeren bei Raumtemperatur für 24 Stunden stehen. Nach Abfiltrieren des trans-Produktes erfolgt die Entfernung des Lösungsmittels im Vakuum. Bei der sich anschließenden Chromatographie mit einer Flash-Kieselgel-Frittensäule (H: 8 cm, ⌀: 6 cm) wird Pentan/Essigester im Verhältnis von 400:10 bis 400:50 (in ml) verwendet.

Nach Umkristallisieren aus dem jeweiligen Lösungsmittelgemisch und Trocknen im Hochvakuum erhält man 3,03 g (8,34 mmol, 16,3 % d. Th.) des trans-Isomers und 6,44 g (17,73 mmol, 34,6 % d. Th.) des cis-Isomers bezüglich der Stellung der Phenyl-Substituenten am Cyclopentan-Gerüst.

| | |
|---|---|
| Schmelzpunkt (cis-Isomer) | 135-137 °C |
| Schmelzpunkt (trans-Isomer) | 168-169 °C |

### Darstellung von trans-3,4-Bis(o-chlorphenyl)cyclopentanon

In einem 50 ml-Kolben werden 900 mg (2,56 mmol) trans-2-Carbmethoxy-3,4-bis-(o-chlorphenyl)cyclopentanon mit 50 ml 48%iger HBr-Lösung versetzt. Diese Suspension wird 2 Stunden auf 130 °C erwärmt. Der Feststoff geht hierbei in ölige, braune Tropfen über. Die Reaktionslösung wird nach dem Abkühlen auf ca. 500 g Eis, das mit 20 g Na₂CO₃ versetzt ist, geleert. Es wird solange festes Na₂CO₃ zugefügt bis der pH-Wert zwischen 7 und 8 liegt. Die wässrige Phase wird dreimal mit je 75 ml Ether extrahiert. Die vereinigten Etherextrakte werden über MgSO₄ getrocknet. Nach dem Entfernen des Lösungsmittels am Rotationsverdampfer erhält man 840 mg eines gelblich, farblosen Feststoffs, der mittels Kieselgel-Flash-Chromatographie (H: 30 cm, ⌀: 2 cm) gereinigt wird. Als Laufmittel dient Pentan/Essigester im Verhältnis 90:10.

Man erhält 628 mg (2,06 mmol, 80,5 % d. Th.) analysenreines, farbloses Produkt.

### Darstellung von trans-1,2-Bis(o-chlorphenyl)cyclopentan

915 mg (3,0 mmol) trans-3,4-Bis(o-chlorphenyl)cyclopentanon werden mit 9 ml Triethylenglycol, 450,0 mg (9,0 mmol) Hydrazinhydrat (100 %) und 673 mg (12,0 mmol) fein gepulvertem Kaliumhydroxid versetzt. Das Reaktionsgemisch wird 3 Stunden bei 160 °C unter Rückflussbedingungen erhitzt. Man versieht den Reaktionskolben mit einer Destillationsbrücke, um ein Gemisch aus H₂O und überschüssigem Hydrazinhydrat abzudestillieren. Dann wird die Reaktionstemperatur auf 200 °C gesteigert. Bei dieser Temperatur hält man das Gemisch bis die Stickstoffentwicklung (ca. 45-60 min) beendet ist. Das Lösungsmittel wird unter leichtem Erwärmen im Hochvakuum teilweise entfernt, der verbleibende braune Rückstand mit 10 ml H₂O und 20 ml Ether versetzt und die wässrige Phase nochmals mit 20 ml Ether extrahiert. Mit 1N HCl-Lösung und Wasser schüttelt man die vereinigten Etherextrakte aus. Nach dem Trocknen über MgSO₄ und Entfernen des Lösungsmittels am Rotationsverdampfer wird der Rückstand aus Ether umkristallisiert. Die Ausbeute an farblosen Kristallen beträgt 744 mg (2,55 mmol, 83,5 % d. Th.).

### Darstellung von trans-1,2-Bis[o-(diphenylphosphino)phenyl]cyclopentan

98 mg (14,16 mmol) Lithium-Pulver werden in der Glovebox in eine Kammer eines ausgeheizten Reaktionsgefäßes mit zwei durch eine Fritte getrennten Kammern eingewogen, mit 15 ml absolutem THF suspendiert und anschließend auf -78 °C abgekühlt. Zu dieser Suspension werden mittels einer gasdichten Spritze, die in 5 ml absolutem THF gelösten und vorher entgasten 1030 mg (3,54 mmol) trans-1,2-Bis(o-chlorphenyl)cyclopentan zugetropft. Diese tiefrote Lösung wird bei -78 °C für 21 Stunden gerührt, bevor zur Abtrennung überschüssigem Lithiums und gebildeten Lithiumchlorids die Reaktionsmischung durch leichtes Kippen des Reaktionsgefäßes und Anlegen eines schwachen Vakuums in die, durch eine Fritte getrennte zweite Kammer überführt wird. Die Reaktionskammer der Lithiierung wird mit 5 ml absolutem THF gespült. Die Zugabe von 1642 mg (7,43 mmol) Diphenylchlorphosphan erfolgt zügig mittels einer gasdichten Spritze, da bei dieser Temperatur das Diphenylchlorphosphan ausfriert. Die Reaktionslösung wird langsam auf -50 °C, dann auf -30 °C erwärmt, bevor sie über Nacht Raumtemperatur annimmt und eine hellgelbe, klare Lösung entsteht, die in ein Schlenkrohr überführt wird. Nach Entfernen des Lösungsmittels und Trocknen im Hochvakuum verbleibt eine hellgelbe Substanz, die mit 100 ml Sodalösung (3 g in 250 ml entgastem H₂O, pH 10) versetzt wird. Nach Abkanülieren der Lösung wird erneut mit 100 ml Sodalösung gewaschen und die überstehende Lösung entfernt. Nach weiterem zweimaligen Waschen mit 100 ml entgastem Wasser und Verwerfen der Waschlösungen wird der Rückstand im Hochvakuum getrocknet. Die Substanz wird mit 15 ml absolutem Hexan versetzt und kräftig gerührt, wobei man eine hellgelbe Lösung und einen feinen, farblosen Niederschlag erhält. Der Feststoff wird nochmals mit 15 ml Hexan gewaschen und dann aus heißem, absolutem Aceton umkristallisiert.

Die Ausbeute an farblosem Pulver beträgt 770 mg (1,31 mmol, 38 % d. Th.).

### I.2 Herstellung von Ligand III

Die Herstellung erfolgt in der ersten Stufe analog der Herstellung von Ligand II, wobei jedoch nach der ersten Reaktionsstufe mit dem cis-Isomer weitergearbeitet wird.

### Darstellung des Tosylhydrazons von cis-3,4-Bis(o-chlorphenyl)cyclopentanon

1612 mg (5,28 mmol) cis-3,4-Bis(o-chlorphenyl)cyclopentanon und 1346 mg (7,23 mmol) Toluol-4-sulfonsäurehydrazid werden in 80 ml absolutem Methanol gelöst und die farblose Lösung zum Rückfluss erhitzt. Nach einer Stunde beginnt das Produkt in Form eines flockigen, farblosen Niederschlags auszufallen. Um die Reaktion zu vervollständigen wird noch weitere 5 Stunden unter Rückfluss erhitzt, der Niederschlag nach dem Abkühlen auf Raumtemperatur über eine Fritte abgesaugt, mit kaltem Methanol nachgewaschen und im Hochvakuum getrocknet. Die Ausbeute an analysenreinem, farblosem Produkt beträgt 2122 mg (4,49 mmol, 85 % d. Th.).

### Darstellung von cis-1,2-Bis(o-chlorphenyl)cyclopentan

2094 mg (4,43 mmol) des Tosylhydrazons von cis-3,4-Bis(o-chlorphenyl)cyclopentanon werden bei Raumtemperatur in 140 ml absolutem Chloroform gelöst und unter Rühren vorsichtig mit 796 mg (6,65 mmol) 1,3,2-Benzodioxaborol (Catecholboran) versetzt. Diese Reaktionslösung wird für weitere 3,5 Stunden bei Raumtemperatur gerührt, anschließend mit 2180 mg (26,58 mmol) Natriumacetat-Trihydrat versetzt und 5 Stunden zum Rückfluss erhitzt, wobei eine farblose Suspension entsteht, die nach dem Abkühlen mit Wasser und gesättigter NaCl-Lösung ausgeschüttelt wird. Nach dem Trock-5 nen über MgSO₄ wird das Lösungsmittel im Vakuum entfernt. Der Rückstand wird mittels Kieselgel-Flash-Chromatographie (H: 50 cm, ⌀: 4 cm) gereinigt. Als Laufmittel dient Petrolether/Essigester im Verhältnis 95:5.

Das Produkt, 888 mg (3,05 mmol, 68,8 % d. Th.), wird als farbloses Öl erhalten, das nach kurzer Zeit zu einer farblosen Masse erstarrt.

### Darstellung von cis-1,2-Bis[o-(diphenylphosphino)phenyl]cyclopentan

95 mg (13,72 mmol) Lithium-Pulver werden in der Glovebox in eine Kammer eines ausgeheizten Reaktionsgefäßes mit zwei Kammern eingewogen, in 20 ml absolutem Tetrahydrofuran suspendiert und anschließend auf -78 °C abgekühlt. Zu dieser Suspension werden 1000 mg (3,43 mmol) cis-1,2-Bis(o-chlorphenyl)cyciopentan, gelöst in 5 ml absolutem THF, zugetropft. Diese Lösung wird 24 Stunden bei -78 °C gerührt, bevor zur Abtrennung überschüssigem Lithiums und des gebildeten Lithiumchlorids die Reaktionsmischung durch leichtes Kippen des Reaktionsgefäßes und Anlagen eines schwachen Vakuums in die, durch eine Fritte getrennte, zweite Kammer überführt wird. Die Reaktionskammer der Lithiierung wird mit 10 ml absolutem THF nachgespült. 1526 mg (6,91 mmol) Diphenylchlorphosphan werden mittels einer gasdichten Spritze zügig zugegeben. Die Reaktionslösung wird langsam auf -50 °C, dann auf -30 °C erwärmt, bevor sie über Nacht Raumtemperatur annimmt und eine hellgelbe, klare Lösung entsteht, die in ein Schlenkrohr überführt wird. Nach Entfernen des Lösungsmittels und Trocknen im Hochvakuum bleibt ein hellgelber Feststoff zurück, der dreimal mit 50 ml Sodalösung (3 g in 250 ml entgastem Wasser, pH 10) gewaschen wird. Nach Abkanülieren der Lösung erhält man einen flockigen, farblosen Niederschlag, der nochmals mit entgastem Wasser gewaschen und anschließend im Hochvakuum getrocknet wird.

### I.3 Herstellung von Ligand IV

### Darstellung von trans-2-(o-Chlorphenyl)cyclohexan-1-ol aus Cyclohexenoxid

In einem ausgeheizten Schlenkrohr werden 407 mg (250 µl, 2,12 mmol, 2,0 eq.) 1,2-Bromchlorbenzol in 5 ml einer 1:1 Mischung aus absolutem THF/Diethylether vorgelegt und in einem Kältebad aus flüssigem Stickstoff und Diethylether auf -110 °C gekühlt. Über eine Spritze gibt man langsam 2,65 ml (2,12 mmol, 2,0 eq.) 1,6M n-Butyllithiumlösung zu. Dabei fällt ein weißer Niederschlag aus. Die Suspension wird 45 min bei einer Temperatur von -110 °C bis -105 °C gerührt. Dann werden 104 mg (110 µl, 1,06 mmol, 1,0 eq.) Cyclohexenoxid und anschließend tropfenweise 226 mg (400 µl, 1,59 mmol, 1,5 eq.) BF₃-Etherat zugegeben. Man lässt noch eine Stunde bei -110 °C rühren und erwärmt die Mischung langsam auf Raumtemperatur. Unter Eiskühlung wird mit ca. 5 ml NH₄Cl-Lösung hydrolysiert. Die wässrige Phase extrahiert man dreimal mit 20 ml Diethylether, wäscht die vereinigten organischen Phasen mit gesättigter NaCl-Lösung und trocknet über Na₂SO₄. Nach Entfernen des Lösungsmittels am Rotationsverdampfer wird das verbleibende hellgelbe Öl durch Flash-Kieselgelchromatographie (Petrolether/Essigester 80:20, 30 cm, ⌀ = 1,5 cm) gereinigt. Man erhält nach Abdestillation des Laufmittels 200 mg (0,95 mmol, 89,5 % d. Th.) eines farblosen, kristallinen Feststoffs.

### Darstellung von 2-(o-Chlorphenyl)cyclohexan-1-on

In einem 100 ml Einhalskolben werden 1,99 g (9,4 mmol) trans-2-(o-Chlorphenyl)cyclohexan-1-ol in 20 ml Methylenchlorid gelöst und mit 3,99 g Pyridiniumchlorochromat sowie 2,5 g gemahlenem Molsieb versetzt und die erhaltene Suspension 4,5 Stunden bei Raumtemperatur gerührt. Die Suspension wird am Rotationsverdampfer vom Lösungsmittel befreit und in Diethylether aufgeschlämmt. Zur Abtrennung der Chromsalze wird über eine kurze Kieselgelsäule (20 cm, ⌀ = 2 cm) filtriert und mit ca. 300 ml Diethylether eluiert. Das Eluat wird am Rotationsverdampfer vom Lösungsmittel befreit und säulenchromatographisch gereinigt (Kieselgel, Petrolether/Essigester 80:20, 30 cm, ⌀ = 4 cm). Man erhält 1,76 g (8,4 mmol, 89,1 % d. Th.) Produkt.

### Darstellung von 2-(o-Chlorphenyl)-1-cyclohexenyltriflat

In einem ausgeheizten Schlenkrohr werden unter Schutzgasatmosphäre 67 mg (2,76 mmol) Natriumhydrid in 10 ml absolutem Dimethylformamid suspendiert und mit in 3 ml absolutem Dimethylformamid gelösten 500 mg (2,40 mmol) 2-(o-Chlorphenyl)cyclohexanon tropfenweise versetzt, wobei die Reaktionssuspension mit zunehmender Menge von 2-(o-Chlorphenyl)cyclohexanon in eine gelbe Lösung übergeht. Nach beendeter Zugabe wird für weitere 2 Stunden bei Raumtemperatur gerührt, bevor 986 mg (2,76 mmol) N,N-Bis(trifluormethansulfonyl)anilin in einer Portion als Feststoff zugegeben werden. Die Reaktionslösung lässt man über Nacht bei Raumtemperatur rühren, extrahiert danach mit Ether und wäscht mit gesättigter NH₄Cl-Lösung, Wasser und gesättigter NaCl-Lösung. Die Trocknung erfolgt über MgSO₄. Nach dem Entfernen des Lösungsmittels im Vakuum erhält man eine hellgelbe Verbindung, die mittels Kieselgel-Flash-Chromatographie mit Petrolether als Laufmittel (H: 45 cm, ⌀: 3 cm) gereinigt wird.

### Darstellung von 1,2-Bis(o-chlorphenyl)cyclohexen durch Umsetzung des Enoltriflats von 2-(o-Chlorphenyl)cyclohexanon mit (o-Chlorphenyl)boronsäure (Suzuki-Kupplung)

In einem ausgeheizten Schlenkrohr mit Kühlfinger werden in der Glovebox 28,8 mg (25 µmol, 5 mol-%) Tetrakis(triphenylphosphin)palladium eingewogen und in 3 ml absolutem Dimethylformamid gelöst. In einem anderen Schlenkrohr werden 86 mg (0,55 mmol, 1,1 eq.) (o-Chlorphenyl)boronsäure und 170 mg (0,5 mmol) Triflat vorgelegt und sorgfältig entgast. Man löst in 5 ml absolutem Dimethylformamid, gibt diese Lösung zur Katalysatorlösung und spült mit 3 ml absolutem Dimethylformamid nach. Zum Reaktionsgemisch werden 5 ml entgaster 2M Sodalösung gegeben und die Emulsion unter heftigem Rühren 24 Stunden zum Rückfluss erhitzt. Die Phasen werden getrennt und die wässrige Phase zweimal mit 50 ml Diethylether extrahiert. Die vereinigten organischen Phasen werden mit Wasser und gesättigter NaCl-Lösung gewaschen, über Na₂SO₄ getrocknet und am Rotationsverdampfer eingeengt. Man erhält ein gelbliches Öl, das durch Flash-Kieselgelchromatographie (40 cm, ⌀ = 1,5 cm, Pentan) gereinigt wird. Nach Entfernen des Laufmittels erhält man 130 mg (0,43 mmol, 85,9 % der Theorie) farbloser, analysenreiner Kristalle.

Die weitere Umsetzung des 1,2-Bis(o-Chlorphenyl)cyclohexens zu Ligand IV erfolgte durch Umsetzung mit Diphenylchlorphosphan, wie zuvor bei der Herstellung von Ligand II beschrieben.

### I.4 Darstellung von Ligand V

### Darstellung von 2-(o-Chlorphenyl)bicyclo[2.2.1]hept-2-en

In einem ausgeheizten 500 ml Dreihalskolben mit Rückflusskühler, Tropftrichter und Überdruckventil werden 0,73 g (30,07 mmol, 1.1 eq.) Mg-Späne unter Argon-Atmosphäre 48 Stunden gerührt. Man fügt 10 ml absoluten Diethylether sowie 5 ml einer Lösung aus 10 ml (27,34 mmol) 1,2-Bromchlorbenzol in 90 ml absolutem Diethylether hinzu. Der Rest wird in den Tropftrichter gegeben. Der Kolbeninhalt wird vorsichtig erwärmt, bis eine leichte Trübung der Lösung das Einsetzen der Reaktion anzeigt. Man dosiert die restliche 1,2-Bromchlorbenzol-Lösung so zu, dass die Reaktionsmischung leicht siedet. Nach beendeter Zugabe wird 6 Stunden zum Rückfluss erhitzt und anschließend über Nacht bei Raumtemperatur gerührt. Man gibt unter Schutzgas eine Lösung aus 2,109 g (19,14 mmol, 1 eq.) Norcampher in 80 ml absolutem Diethylether über 30 min hinzu und erhitzt 5 Stunden zum Rückfluss. Die Lösung wird im Eisbad bei 0 °C mit gesättigter NH₄-Cl-Lösung hydrolysiert und die wässrige Phase dreimal mit 100 ml Diethylether ausgeschüttelt. Die vereinigten organischen Phasen werden über Na₂SO₄ getrocknet und am Rotationsverdampfer eingeengt. Das verbleibende Öl wird in 100 ml absolutem Benzol aufgenommen und mit katalytischen Mengen p-Toluolsulfonsäure in einer Wasserabscheider-Apparatur 16 Stunden zum Rückfluss erhitzt. Bei 0 °C wird mit gesättigter Na₂CO₃-Lösung ausgeschüttelt. Die wässrige Phase wird dreimal mit 100 ml Diethylether extrahiert, die vereinigten organischen Phasen über Na₂SO₄ getrocknet. Das verbleibende hellgelbe Öl wird säulenchromatographisch (Kieselgel, Petrolether, 45 cm, ⌀ = 4 cm) gereinigt. Man erhält 2,64 g (12,90 mmol, 67,5 % d. Th.) eines farblosen Öls.

### Darstellung von 3-(o-Chlorphenyl)bicyclo[2.2.1]heptan-2-ol

In einem ausgeheizten Schlenkrohr mit Septum werden 3,243 g (15,87 mmol) 2-(o-Chlorphenyl)bicyclo[2.2.1]hept-2-en in 5 ml absolutem Pentan gelöst und unter Rühren tropfenweise mit einer Lösung von 0,824 g BH₃-1,4-Oxathian-Komplex in 10 ml absolutem Pentan versetzt. Man spült zweimal mit 2 ml Pentan nach, ersetzt das Septum durch einen Kühlfinger mit Überdruckventil und erhitzt 5,5 Stunden zum Rückfluss. Nach Rühren über Nacht bei Raumtemperatur werden 7,6 ml Ethanol, 5,3 ml 3N NaOH und langsam über 15 min 2 ml 30 %ige H₂O₂ zugesetzt und eine Stunde unter Rückfluss erhitzt. Die Lösung wird in 150 ml Eiswasser gegeben, die organische Phase abgetrennt und die wässrige Phase dreimal mit 100 ml Diethylether extrahiert. Nach Trocknung der vereinigten organischen Phasen über Na₂SO₄ entfernt man das Lösungsmittel am Rotationsverdampfer und reinigt den leicht gelblichen Feststoff säulenchromatographisch (Kieselgel, Petrolether/Essigester 90:10, 40 cm, ⌀ = 4 cm). Man erhält 1,439 g (6,50 mmol, 41,0 % d. Th.) eines farblosen kristallinen Feststoffs.

### Darstellung von 3-(o-Chlorphenyl)biryclo[2.2.1]heptan-2-on

In einem 100 ml Einhalskolben werden 2,166 g (9,4 mmol) 3-(o-Chlorphenyl)bicyclo[2.2.1]heptan-2-ol in 20 ml Methylenchlorid gelöst und mit 4,172 g Pyridiniumchlorochromat sowie 2,600 g gemahlenem Molsieb versetzt. Die resultierende Suspension wird 4,5 Stunden bei Raumtemperatur gerührt, am Rotationsverdampfer vom Lösungsmittel befreit und in Diethylether aufgeschlämmt. Zur Abtrennung der Chromsalze wird über eine kurze Kieselgelsäule (20 cm, ⌀ = 2 cm) filtriert und mit ca. 300 ml Diethylether eluiert. Das Eluat wird am Rotationsverdampfer vom Lösungsmittel befreit und säulenchromatographisch gereinigt (Kieselgel, Petrolether/Essigester 90:10, 30 cm, ⌀ = 4 cm). Zurück bleiben 1,835 g (8,36 mmol, 85,6 % d. Th.) Produkt.

### Darstellung des Enoltriflats von 3-(o-Chlorphenyl)bicyclo[2.2.1]heptan-2-on

In einem ausgeheizten Schlenkrohr werden 1,780 g 2-(o-Chlorphenyl)cyclohexanon (8,19 mmol) vorgelegt, durch dreimaliges Evakuieren und Spülen mit Argon entgast, in 10 ml absolutem Dimethylformamid gelöst und diese Lösung zu einer Suspension aus 214 mg Natriumhydrid (8,93 mmol, 1,1 eq.) in 20 ml absolutem Dimethylformamid getropft. Nach 16 Stunden Rühren bei Raumtemperatur fügt man 3,503 g (9,34 mmol, 1,15 eq.) festes N,N-Bis(trifluormethansulfonyl)anilin auf einmal hinzu. Es wird über Nacht gerührt und am nächsten Tag nach Zugabe von 150 ml Diethylether bei 0 °C mit gesättigter Na₂SO₄ -Lösung hydrolysiert, zweimal mit 100 ml destilliertem Wasser und einmal mit 50 ml gesättigter NaCl-Lösung gewaschen. Die organische Phase trocknet man über Na₂SO₄ und engt am Rotationsverdampfer zur Trockene ein. Nach Flash-Chromatographie über Kieselgel (45 cm, ⌀ = 4 cm, Petrolether) erhält man 1,391 g (3,94 mmol, 48,2 % d. Th.) Produkt.

### Darstellung von 2,3-Bis(o-Chlorphenyl)bicyclohept-2-en

In einem ausgeheizten Schlenkrohr werden in der Glovebox 217 mg (188 µmol, 5 mol%) Pd(PPh₃)₄-Katalysator eingewogen und in 25 ml absolutem Dimethoxyethan gelöst. In einem ausgeheizten 500 ml Dreihalskolben mit Rückflusskühler und Überdruckventil werden 637 mg (4,14 mmol, 1,1 eg.) (o-Chlorphenyl)boronsäure und 1326 mg (3,76 mmol) Triflat vorgelegt, entgast, in 80 ml absolutem Dimethoxyethan gelöst und diese Lösung zur Katalysatorlösung gegeben. Zum Reaktionsgemisch werden 100 ml entgaster 2M Sodalösung gegeben und die Emulsion unter heftigem Rühren 24 Stunden zum Rückfluss erhitzt. Die Phasen werden getrennt und die wässrige Phase zweimal mit 200 ml Diethylether extrahiert. Die vereinigten organischen Phasen werden mit Wasser, gesättigter NaCl-Lösung gewaschen, über Na₂SO₄ getrocknet und am Rotationsverdampfer eingeengt. Man erhält ein gelbliches Öl, das durch Flash-Kieselgelchromatographie (40 cm, ⌀ = 4 cm, Petrolether) gereinigt wird. Nach Entfernen des Laufmittels erhält man 1025 mg (3,25 mmol, 86,4 % d. Th.) Produkt.

### Darstellung von 2,3-Bis[2-(diphenylphosphino)phenyl]bicyclo[2.2.1]-hept-2-en

In einem ausgeheizten Schlenkrohr werden in der Glove-Box 125 mg (15,8 mmol, 10 eq.) Lithiumpulver eingewogen, in 10 ml absolutem THF suspendiert und auf -78 °C gekühlt. Über eine Spritze tropft man eine Lösung von 800 mg (1,58 mmol) 2,3-Bis(o-chlorphenyl)cyclohexen in 5 ml absolutem THF zu und spült zweimal mit 2 ml absolutem THF nach. Die Suspension wird unter Argon bei -78 °C filtriert, der Filterkuchen zweimal mit 5 ml absolutem THF nachgespült, bei -78 °C 1,273 g (5,28 mmol, 2,2 eq.) Diphenylchlorphosphan zugegeben und der Ansatz über Nacht auf Raumtemperatur erwärmt. Das Lösungsmittel wird in eine Kühlfalle gezogen. Die verbleibende Masse wird dreimal mit 20 ml entgaster 2M Sodalösung, dreimal 20 ml entgastem Wasser und dreimal 10 ml Pentan gewaschen. Dabei geht die Masse in einen gelblichen Feststoff über. Dieser wird zweimal aus Aceton umkristallisiert. Man erhält das Produkt als farblosen, mikrokristallinen Feststoff.

### I.5 Darstellung von Ligand VIII

### Darstellung von cis-1,2-Bis[o-{bis(diethylamino)phosphanyl}phenyl]cyclopentan

In einem ausgeheizten Schlenkrohr werden in der Glove-Box 190,1 mg (26,0 mmol, 10 eq.) Lithiumpulver eingewogen, in 10 ml absolutem THF suspendiert und auf -78 °C gekühlt. Über eine Spritze tropft man eine Lösung von 784,0 mg (2,69 mmol) cis-1,2-Bis(o-chlorphenyl)cyclopentan in 5 ml absolutem THF zu und spült zweimal mit 2 ml absolutem THF nach. Die Suspension wird unter Argon bei -78 °C filtriert, der Filterkuchen zweimal mit 5 ml absolutem THF nachgespült, bei -78 °C 1,143 g (5,41 mmol, 2,1 eq.) Bis(diethylamino)chlorphosphan zugegeben und der Ansatz anschließend über Nacht auf Raumtemperatur erwärmt. Das Lösungsmittel wird in eine Kühlfalle gezogen und die verbleibende Masse dreimal mit 10 ml Pentan extrahiert. Die Extraktionslösung wird auf ca. 2 ml reduziert und auch -80 °C gekühlt. Dabei kristallisiert das Produkt als weißer Feststoff (1,086 g, 1,9 mmol, 70,8 % d. Th.) aus.

### Darstellung von cis-1,2-Bis[o-(2-dibenzo[d,f]-1,3,2-dioxaphosphepinyl)phenyl]cyclopentan

In einem ausgeheizten Schlenkrohr mit Kühlfinger und Überdruckventil werden 1449 mg (2,5 mmol) cis-1,2-Bis[o-{bis(diethylamino)phosphanyl}phenyl]cyclopentan in 10 ml absolutem Toluol gelöst und mit 946,3 mg (5,0 mmol, 2 eg.) sorgfältig entgastem und getrocknetem 2,2'-Dihydroxybiphenyl versetzt. Man erhitzt 16 Stunden auf 110 °C. Nach Abkühlen auf Raumtemperatur wird das Lösungsmittel im Hochvakuum entfernt und der verbleibende gelbe Feststoff aus Aceton umkristallisiert. Es verbleiben farblose Kristalle (665 mg, 1,0 mmol, 40,3 % d. Th.).

### II. Hydroformylierungen

### Beispiel 1:

### Hydroformylierung von 1-Octen

In einem 300 ml Stahlautoklaven mit Begasungsrührer wurden unter Argonschutzgas 1,6 mg Rhodiumbiscarbonylacetylacetonat und 19,1 mg Ligand I (Ligand/Metall-Verhältnis = 5:1) in 6 g Toluol gelöst und bei 90 °C mit einem Synthesegasgemisch CO/H₂ (1:1) bei 10 bar umgesetzt. Nach einer Reaktionszeit von 4 h wurde der Autoklav entspannt und entleert. Das Gemisch wurde mittels Gaschromatographie (GC) mit internem Standard analysiert. Der Umsatz betrug 96 %. Der Anteil an α-Isomeren (n-Aldehyd und iso-Aldehyd) betrug 98 %.

### Beispiel 2:

### Hydroformylierung von 1-Octen mit Ligand II

Analog zu Beispiel 1 wurden 2,1 mg Rhodiumbiscarbonylacetylacetonat, 17,8 mg Ligand II, 6 g 1-Octen und 6 g Toluol zur Hydroformylierung eingesetzt (Ligand/Metall-Verhältnis = 4:1). Der Umsatz betrug 99 %, der α-Anteil betrug 97 %.

### Beispiel 3:

### Hydroformylierung von 1-Octen mit Ligand III

Analog zu Beispiel 1 wurden 1,0 mg Rhodiumbiscarbonylacetylacetonat, 10 mg Ligand III, 6 g 1-Octen und 6 g Toluol zur Hydroformylierung eingesetzt (Ligand/Metall-Verhältnis = 4:1). Der α-Anteil betrug 100 %.

### Beispiel 4:

### Hydroformylierung von 1-Octen mit Ligand III

In einem 300 ml Stahlautoklaven mit Begasungsrührer wurden 0,56 mg Rhodiumbiscarbonylacetylacetonat und 13 mg Ligand III in 2,2 g Toluol gelöst und bei 100 °C mit einem Synthesegasgemisch CO/H₂ (1:1) bei 10 bar begast (Ligand/Metall-Verhältnis = 10:1). Nach 30 min wurde der Autoklav entspannt, dann wurden 2,2 g 1-Octen zugegeben und weitere 4 h bei 80 °C und 10 bar hydroformyliert. Der α-Anteil betrug 100 %.

### Beispiel 5:

### Hydroformylierung von 1-Octen mit Ligand IV

Analog zu Beispiel 4 wurden 0,6 mg Rhodiumbiscarbonylacetylacetonat, 13,7 mg Ligand IV, 2 g 1-Octen und 2 g Toluol zur Hydroformylierung eingesetzt (Ligand/Metall-Verhältnis = 10:1). Der α-Anteil betrug 100 %.

### Beispiel 6:

### Hydroformylierung von 1-Octen mit Ligand V

Analog zu Beispiel 4 wurden 0,65 mg Rhodiumbiscarbonylacetylacetonat, 15,4 mg Ligand V, 2,2 g 1-Octen und 2,2 g Toluol zur Hydroformylierung eingesetzt (Ligand/Metall-Verhältnis = 10:1). Der α-Anteil betrug 100 %.

### Beispiel 7:

### Hydroformylierung von 1-Octen mit Ligand VI

Analog zu Beispiel 4 wurden 0,56 mg Rhodiumbiscarbonylacetylacetonat, 14 mg Ligand VI, 2,2 g 1-Octen und 2,2 g Toluol zur Hydroformylierung eingesetzt (Ligand/Metall-Verhältnis = 10:1). Der α-Anteil betrug 100 %.

### Beispiel 8:

### Hydroformylierung von 1-Octen mit Ligand VII

Analog zu Beispiel 4 wurden 0,56 mg Rhodiumbiscarbonylacetylacetonat, 14,7 mg Ligand VII, 2,2 g 1-Octen und 2,2 g Toluol zur Hydroformylierung eingesetzt (Ligand/Metall-Verhältnis = 10:1). Der α-Anteil betrug 100 %.

### Beispiel 9:

### Hydroformylierung von 1-Octen mit Ligand IV

In einem 300 ml Stahlautoklaven mit Begasungsrührer wurden 2,47 mg Rhodiumbiscarbonylacetylacetonat und 51,3 mg Ligand IV in 8,2 g Toluol gelöst (Ligand/Metall-Verhältnis = 10:1) und bei 100 °C mit 10 bar Synthesegas CO/H₂ (1:1) begast. Nach 3 h wurde der Autoklav entspannt und 8,2 g 1-Octen zugegeben. Anschließend wurde noch 4 h bei 80 °C und 30 bar Synthesegasdruck hydroformyliert. Der α-Anteil betrug 100 %.

### Beispiel 10:

### Hydroformylierung von 1-Octen mit Ligand VIII

1,28 mg Rhodiumbiscarbonylacetylacetonat und 32 mg Ligand VIII (60 ppm Rh, Ligand/Metall-Verhältnis = 10:1) wurden separat eingewogen, in je 2,15 g Toluol gelöst, vermischt und bei 100 °C mit 10 bar Synthesegas (CO:H₂ = 1:1) begast. Nach 30 min. wurde entspannt, dann wurden 4,3 g 1-Octen zugegeben und 4 h bei 80 °C und 10 bar hydroformyliert. Der Umsatz betrug 74 %, die Aldehydselektivität 99 %, die Linearität 79 % und der α-Anteil (n-Nonanal + iso-Nonanal) betrug 100 %.

### Beispiel 11:

### Hydroformylierung von 2-Buten mit Ligand VIII

3 mg Rhodiumbiscarbonylacetylacetonat und 75,3 mg Ligand VIII (60 ppm Rh, Ligand/Metall-Verhältnis = 10:1) wurden separat eingewogen, in je 5 g Toluol gelöst, vermischt und bei 100 °C mit 10 bar Synthesegas (CO:H₂ = 1:1) begast. Nach 30 min. wurde entspannt, dann wurden 10 g 2-Buten zugegeben und 4 h bei 140 °C und 20 bar hydroformyliert. Die Aldehydselektivität betrug 93 % und die Linearität 46 %.

## Patentansprüche

1. Verfahren zur Hydroformylierung von Verbindungen, die wenigstens eine ethylenisch ungesättigte Doppelbindung enthalten, durch Umsetzung mit Kohlenmonoxid und Wasserstoff in Gegenwart eines Hydroformylierungskatalysators, **dadurch gekennzeichnet, dass** man als Hydroformylierungskatalysator wenigstens einen Komplex eines Metalls der VIII. Nebengruppe mit mindestens einem Liganden der allgemeinen Formel I 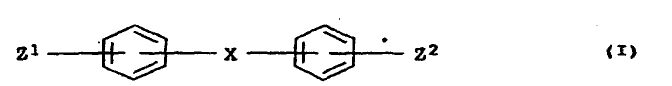 einsetzt, worin
Z¹ und Z² unabhängig voneinander für mindestens ein Phosphor-, Arsen- oder Antimonatom aufweisende Reste stehen, und
X für eine zweiwertige, nichtaromatische, 3- bis 8-gliedrige, carbocyclische oder heterocyclische, verbrückende Gruppe steht, die eine, zwei oder drei Doppelbindungen, und/oder einen, zwei, drei oder vier Substituenten, die ausgewählt sind unter Alkyl, Cycloalkyl, Aryl, Hetaryl, Alkoxy, Acyl, Carboxyl, Alkoxycarbonyl, Hydroxy, Nitro, Cyano, Trifluormethyl, Oxo und den Ketalen davon, oder NE¹E², tragen können, wobei E¹ und E² gleich oder verschieden sein können und für Alkyl, Cycloalkyl oder Aryl stehen, und/oder wobei die verbrückende Gruppe X Teil eines kondensierten Ringsystems mit 1, 2 oder 3 weiteren Ringen sein kann, wobei die ankondensierten Ringe je einen, zwei oder drei Substituenten, die unter den zuvor als Substituenten der Gruppe X genannten ausgewählt sind, tragen können, und/oder wobei die verbrückende Gruppe X ihrerseits durch -O-, -S-, -N(R^{a})- oder eine davon verschiedene zweiwertige verbrückende Gruppe überbrückt sein kann, wobei R^{a} für Wasserstoff, Alkyl, Cycloalkyl oder Aryl steht, und wobei X ausgewählt ist unter Gruppen der Formeln II.1 bis II.3 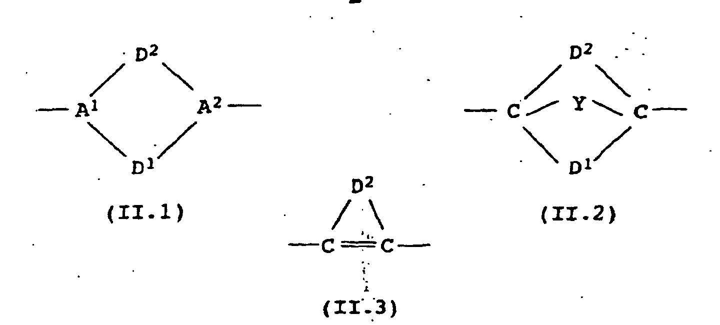 worin
A¹ und A² unabhängig voneinander für B, N, P oder CR⁵ stehen, wobei R⁵ für Wasserstoff, Alkyl, Cycloalkyl, Aryl oder Hetaryl steht,
D¹ für eine Einfachbindung oder eine C₁- bis C₃-Alkylenbrücke steht, die eine Doppelbindung und/oder einen oder zwei Substituenten, die ausgewählt sind unter Alkyl, Cycloalkyl, Aryl, Alkoxy, Acyl, Carboxyl, Alkoxycarbonyl, Hydroxy, Nitro, Cyano, Trifluormethyl, Oxo und den Ketalen davon, oder NE¹E², tragen können, wobei E¹ und E² gleich oder verschieden sein können und für Alkyl, Cycloalkyl oder Aryl stehen,
D² für eine C₃- bis C₆-Alkylenbrücke steht, die eine zwei oder drei Doppelbindungen und/oder einen, zwei, drei oder vier der für D¹ genannten Substituenten aufweisen kann und/oder die Alkylenbrücke D² in den Formeln II.1 und II.3 durch eine Gruppe -Y- überbrückt sein kann, und
Y für O, S, CR^{x}R^{y} oder einen Rest der Formeln III.1 bis III.4 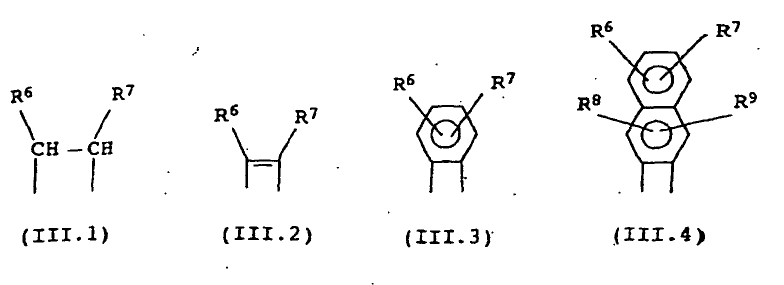 steht, worin
R^{x} und R^{y} ausgewählt sind unter Wasserstoff, Alkyl, Cycloalkyl, Aryl oder Hetaryl,
R⁶, R⁷, R⁸ und R⁹ unabhängig voneinander für Wasserstoff, Alkyl, Cycloalkyl, Aryl, Alkoxy, Halogen, SO₃H, Sulfonat, NE¹E², Alkylen-NE¹E², Trifluormethyl, Nitro, Alkoxycarbonyl, Carboxyl oder Cyano stehen, wobei E¹ und E² unabhängig voneinander für Alkyl, Cycloalkyl oder Aryl stehen.

2. Verfahren nach Anspruch 1, wobei in der Formel I Z¹ und Z² unabhängig voneinander für je einen Rest der Formeln PR¹R², OPR¹R², P(OR¹)R², P(OR¹)(OR²), OP(OR¹)R² oder OP(OR¹)(OR²) stehen, worin
R¹ und R² unabhängig voneinander für Alkyl, Cycloalkyl, Heterocycloalkyl, Aryl oder Hetaryl stehen, welche gegebenenfalls einen, zwei oder drei Substituenten, ausgewählt unter Alkyl, Cycloalkyl, Heterocycloalkyl, Aryl, Hetaryl, COOR^{f}, COO-M⁺, SO₃R^{f}, SO⁻₃M⁺, NE³E⁴, Alkylell-NE³E⁴, NE³E⁴E⁵⁺X⁻, Alkylen-NE³E⁴E⁵⁺X⁻, OR^{f}, SR^{f}, (CHR^{g}CH₂O)_{y}R^{f}, (CH₂N(E³))_{y}R^{f}, (CH₂CH₂N(E³))_{y}R^{f}, Halogen, Trifluormethyl, Nitro, Acyl oder Cyano aufweisen können, worin
R^{f}, E³, E⁴ und E⁵ jeweils gleiche oder verschiedene Reste, ausgewählt unter Wasserstoff, Alkyl, Cycloalkyl oder Aryl bedeuten,
R^{g} für Wasserstoff, Methyl oder Ethyl steht,
M⁺ für ein Kation steht,
X⁻ für ein Anion steht, und
y für eine ganze Zahl von 1 bis 120 steht, oder
R¹ und R² zusammen mit dem Phosphoratom und gegebenenfalls dem/den Sauerstoffatom(en), an die sie gebunden sind für einen 5- bis 8-gliedrigen Heterocyclus stehen, der gegebenenfalls zusätzlich ein-, zwei- oder dreifach mit Cycloalkyl, Heterocycloalkyl, Aryl oder Hetaryl anelliert ist, wobei der Heterocyclus und, falls vorhanden, die anellierten Gruppen unabhängig voneinander je einen, zwei, drei oder vier Substituenten tragen können, die ausgewählt sind unter Alkyl, Cycloalkyl, Heterocycloalkyl, Aryl, Hetaryl, COOR^{f}, COO-M⁺, SO₃R^{f}, SO⁻₃M⁺, NE³E⁴, Alkylen-NE³E⁴, NE³E⁴E⁵⁺X⁻, Alkylen-NE³E⁴E⁵⁺X⁻, OR^{f}, SR^{f}, (CHR^{g}CH₂O)_{y}R^{f}, (CH₂N(E¹))_{y}R^{f}, (CH₂CH₂N(E⁴))_{y}R^{f}, Halogen, Trifluormethyl, Nitro, Acyl oder Cyano, wobei R^{f}, R^{g}, E³, E⁴, E⁵, M⁺, X⁻ und y die zuvor angegebenen Bedeutungen besitzen.

3. Verfahren nach Anspruch 1, **dadurch gekennzeichnet, dass** man als Hydroformylierungskatalysator wenigstens einen Komplex eines Metalls der VIII. Nebengruppe mit mindestens einem Phosphinliganden der allgemeinen Formel I' 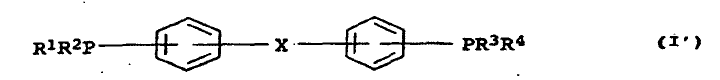 einsetzt, worin
R¹, R², R³ und R⁴ unabhängig voneinander für Alkyl, Cycloalkyl, Aryl oder Betaryl stehen, wobei die Cycloalkyl-, Aryl- und Hetarylgruppen je einen, zwei oder drei Substituenten, die ausgewählt sind unter Alkyl, Cycloalkyl,Aryl, Alkoxy, Cycloalkoxy, Aryloxy, Acyl, Halogen, Trifluormethyl, Nitro, Cyano, Carboxyl, Alkoxycarbonyl oder NE³E⁴,tragen können, wobei E³ und E⁴ gleich oder verschieden sein können und für Alkyl, Cycloalkyl oder Aryl stehen, und
X für eine zweiwertige, nichtaromatische, 3- bis 8-gliedrige, carbocyclische oder heterocyclische, verbrückende Gruppe steht, die eine, zwei oder drei Doppelbindungen, und/oder einen, zwei, drei oder vier Substituenten, die ausgewählt sind unter Alkyl, Cycloalkyl, Aryl, Alkoxy, Acyl, Carboxyl, Alkoxycarbonyl, Hydroxy, Nitro, Cyano, Trifluormethyl, Oxo und den Ketalen davon, oder NE¹E², tragen können, wobei E¹ und E² gleich oder verschieden sein können und für Alkyl, Cycloalkyl oder Aryl stehen, und/oder wobei die verbrückende Gruppe X Teil eines kondensierten Ringsystems mit 1, 2 oder 3 weiteren Ringen sein kann, wobei die ankondensierten Ringe je einen, zwei oder drei Substituenten, die unter den zuvor als Substituenten der Gruppe X genannten ausgewählt sind, tragen können, und/oder wobei die verbrückende Gruppe X ihrerseits durch -O-, -S-, -N(R^{a})- oder eine davon verschiedene zweiwertige verbrückende Gruppe überbrückt sein kann, wobei R^{a} für Wasserstoff, Alkyl, Cycloalkyl oder Aryl steht, und wobei X ausgewählt ist unter Gruppen der Formeln II.1 bis II.3 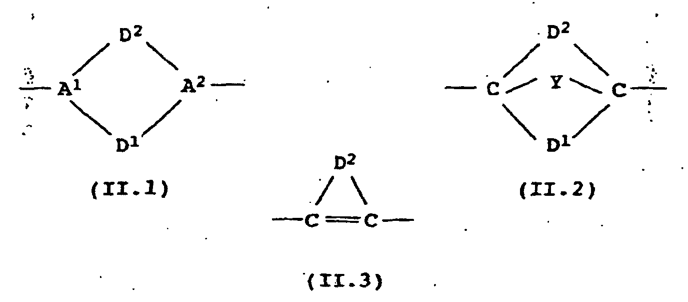 worin
A¹ und A² unabhängig voneinander für B, N, P oder CR⁵ stehen, wobei R⁵ für Wasserstoff, Alkyl, Cycloalkyl, Aryl oder Hetaryl steht,
D¹ für eine Einfachbindung oder eine C₁- bis C₃-Alkylenbrücke steht, die eine Doppelbindung und/oder einen oder zwei Substituenten, die ausgewählt sind unter Alkyl, Cycloalkyl, Aryl, Alkoxy, Acyl, Carboxyl, Alkoxycarbonyl, Bydroxy, Nitro, Cyano, Trifluormethyl, Oxo und den Ketalen davon, oder NE¹E², tragen können, wobei E¹ und E² gleich oder verschieden sein können und für Alkyl, Cycloalkyl oder Aryl stehen,
D² für eine C₃- bis C₆-Alkylenbrücke steht, die eine zwei oder drei Doppelbindungen und/oder einen, zwei, drei oder vier der für D¹ genannten Substituenten aufweisen kann und/oder die Alkylenbrücke D² in den Formeln II.1 und II.3 durch eine Gruppe -Y- überbrückt sein kann, und
Y für O, S, CR^{x}R^{y} oder einen Rest der Formeln III.1 bis III.4 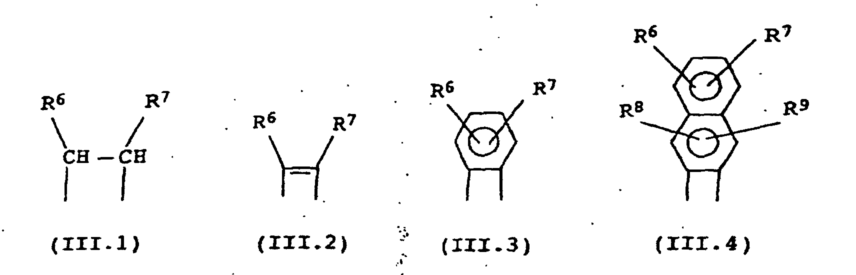 steht, worin
R^{x} und R^{y} ausgewählt sind unter Wasserstoff, Alkyl, Cycloalkyl, Aryl oder Hetaryl,
R⁶, R⁷, R⁸ und R⁹ unabhängig voneinander für Wasserstoff, Alkyl, Cycloalkyl, Aryl, Alkoxy, Halogen, SO₃H, Sulfonat, NE¹E², Alkylen-NE¹E², Trifluormethyl, Nitro, Alkoxycarbonyl, Carboxyl oder Cyano stehen, wobei E¹ und E² unabhängig voneinander für Alkyl, Cycloalkyl oder Aryl stehen.

4. Verfahren nach einem der vorhergehenden Ansprüche, wobei x ausgewählt ist unter Gruppen der Formeln II.a bis II.k 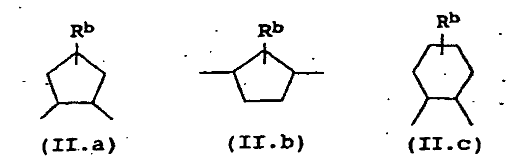 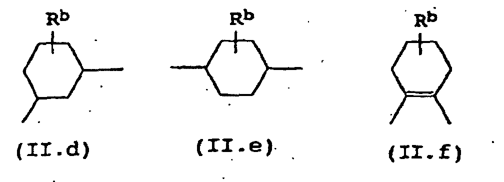 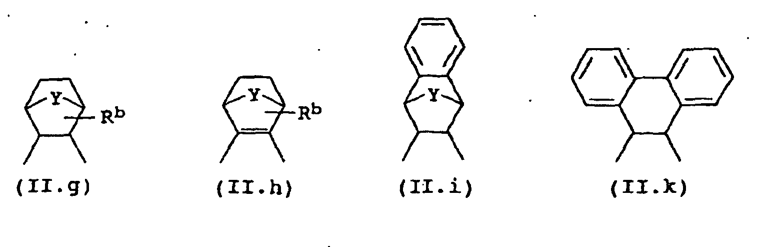 worin
R^{b} ausgewählt ist unter Alkyl, Cycloalkyl, Aryl, Alkoxy, Acyl, Carboxyl, Alkoxycarbonyl, Hydroxy, Nitro, Cyano, Trifluormethyl, Oxo und den Ketalen davon, oder NE¹E², wobei E¹ und E² gleich oder verschieden sein können und für Alkyl, Cycloalkyl oder Aryl stehen, und
Y wie in Anspruch 1 definiert ist.

5. Verfahren nach einem der vorhergehenden Ansprüche, wobei der Ligand der Formel I ausgewählt ist unter Liganden der Formeln IA bis IF 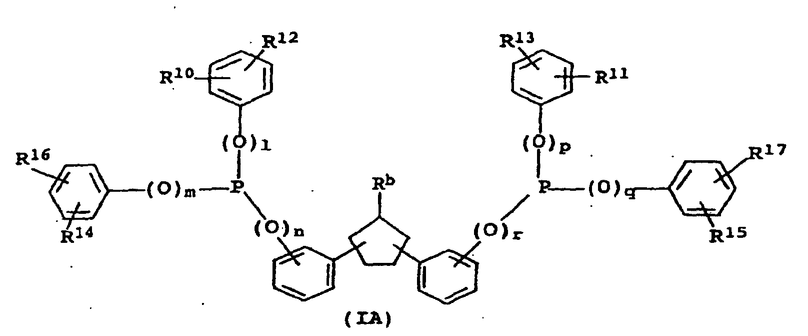 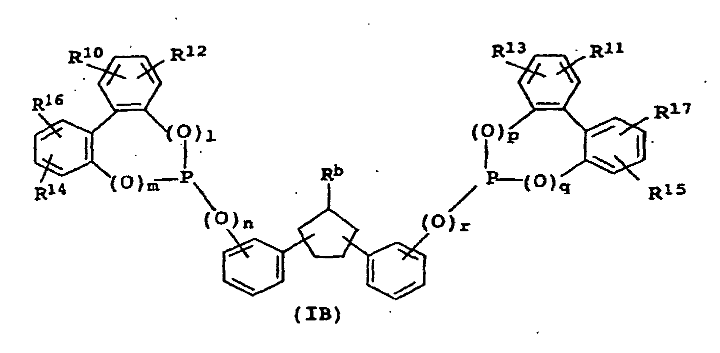 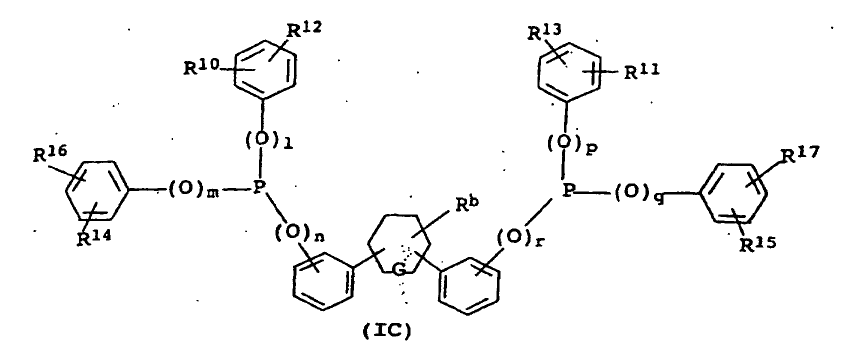 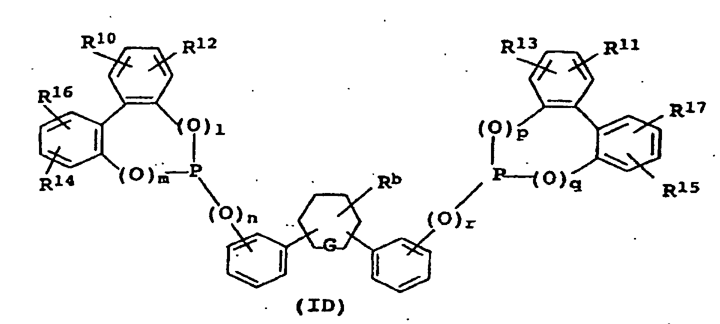 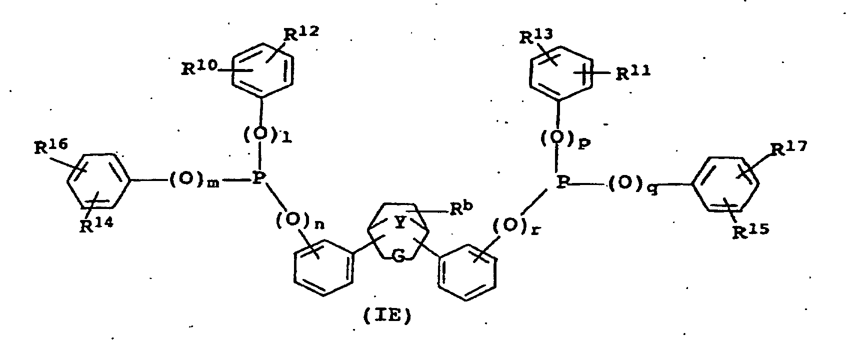 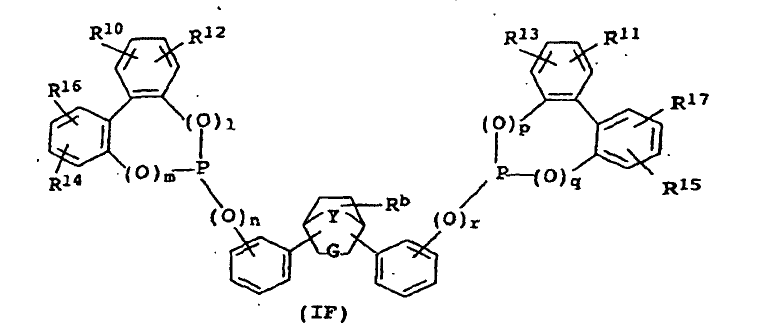 worin
R¹⁰, R¹¹, R¹², R¹³, R¹⁴, R¹⁵, R¹⁶ und R¹⁷ unabhängig voneinander ausgewählt sind unter Wasserstoff, Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE³E⁴, wobei E³ und E⁴ gleich oder verschieden sein können und für Alkyl, Cycloalkyl oder Aryl stehen,
l, m, n, p, q und r unabhängig voneinander für 0 oder 1 stehen,
R^{b} in den Formeln IA und IB ausgewählt ist unter Wasserstoff, Alkyl, Oxo und den Ketalen davon und in den Formeln IC, ID, IE und IF ausgewählt ist unter Wasserstoff und Alkyl,
G für eine Einfachbindung, eine Doppelbindung oder ein Sauerstoffatom steht,
Y für O, S, CH₂ oder einen Rest der Formel III.1 bis III.4 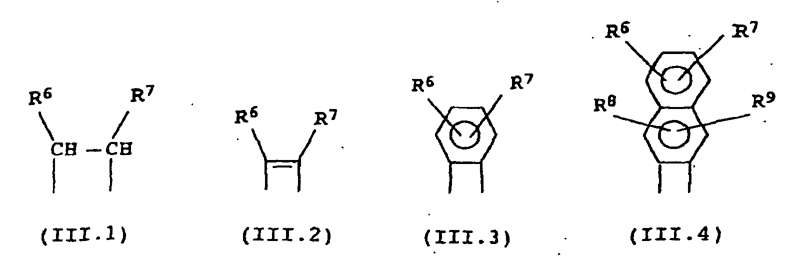 steht, worin
R⁶, R⁷, R⁸ und R⁹ unabhängig voneinander für Wasserstoff, Alkyl, Cycloalkyl, Aryl, Alkoxy, Halogen, SO₃H, Sulfonat, NE¹E², Alkylen-NE¹E², Trifluormethyl, Nitro, Alkoxycarbonyl, Carboxyl oder cyano stehen, wobei E¹ und E² unabhängig voneinander für Alkyl, Cycloalkyl oder Aryl stehen.

6. Verfahren nach einem der vorhergehenden Ansprüche, wobei der Ligand der Formel I ausgewählt ist unter Phosphinliganden der Formeln Ia-Ie 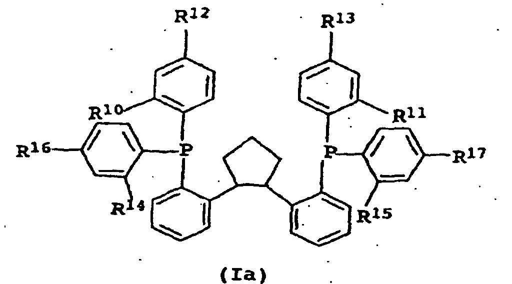 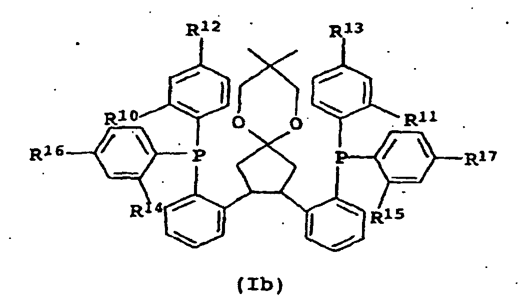 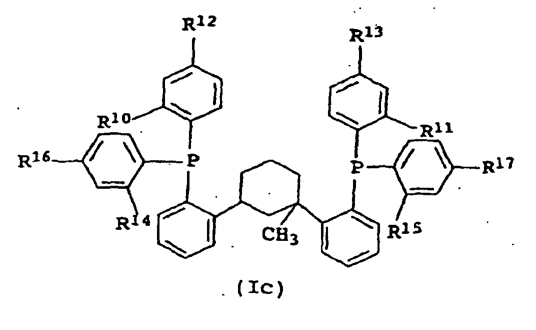 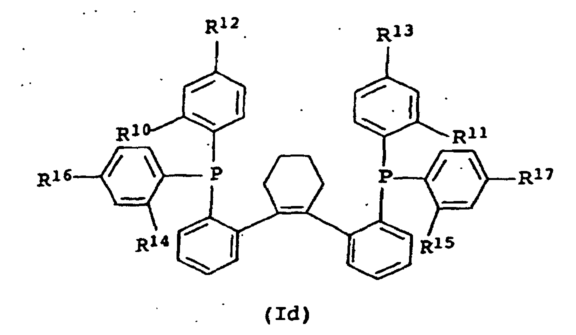 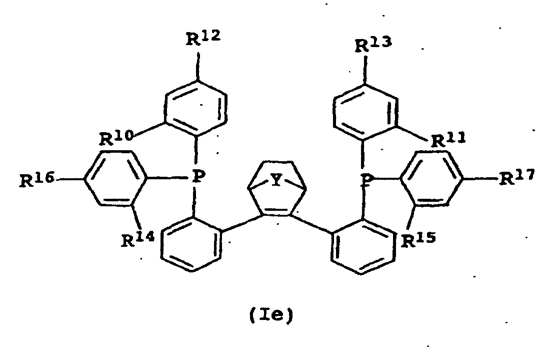 wobei in den Formeln Ia bis Ie die Substituenten R¹⁰, R¹¹,
R¹², R¹³, R¹⁴, R¹⁵, R¹⁶ und R¹⁷ die folgenden Bedeutungen besitzen:
| R¹⁰ | R¹¹ | R¹² | R¹³ | R¹⁴ | R¹⁵ | R¹⁶ | R¹⁷ |
|---|---|---|---|---|---|---|---|
| H | H | H | H | H | H | H | H |
| CH₃ | CH₃ | H | H | H | H | H | H |
| CH₃ | CH₃ | H | H | CH₃ | CH₃ | H | H |
| CH₃ | CH₃ | CH₃ | CH₃ | H | H | H | H |
| CH₃ | CH₃ | CH₃ | CH₃ | CH₃ | CH₃ | H | H |
| CH₃ | CH₃ | CH₃ | CH₃ | CH₃ | CH₃ | CH₃ | CH₃ |
und in der Formel Ie Y für O, S, CH₂ oder einen Rest der Formeln III.1 bis III.4 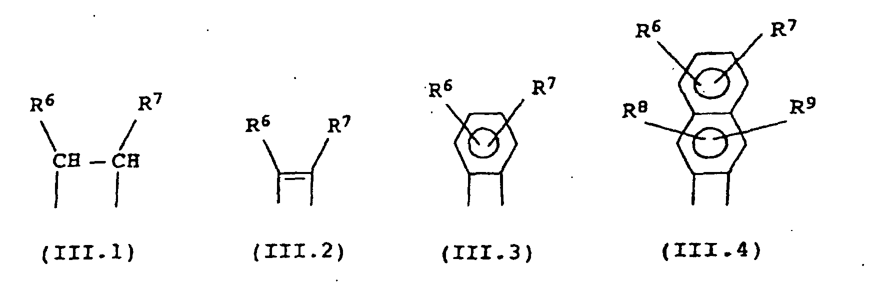 steht, worin
R⁶, R⁷, R⁸ und R⁹ unabhängig voneinander für Wasserstoff, Alkyl, Cycloalkyl, Aryl, Alkoxy, Halogen, SO₃H, Sulfonat, NE¹E², Alkylen-NE¹E², Trifluormethyl, Nitro, Alkoxycarbonyl, Carboxyl oder Cyano stehen, wobei E¹ und E² unabhängig voneinander für Alkyl, Cycloalkyl oder Aryl stehen.

7. Verbindungen der allgemeinen Formel I 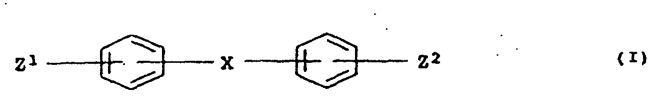 worin
Z¹ und Z² unabhängig voneinander für mindestens ein Phosphor-, Arsen- oder Antimonatom aufweisende Reste stehen, wobei an das Phosphor-, Arsen- oder Antimonatom jeweils mindestens zwei gegebenenfalls substituierte Heteroatome, die ausgewählt sind unter O, S und NR^{c}, direkt gebunden sind, wobei R^{c} für Wasserstoff, Alkyl, Cycloalkyl oder Aryl steht,
und
Verbindungen der allgemeinen Formel I' 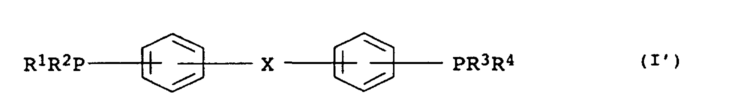 worin
R¹, R², R³ und R⁴ unabhängig voneinander für Alkyl, Cycloalkyl, Aryl oder Hetaryl stehen, wobei die Aryl- und Hetarylgruppen je einen, zwei oder drei Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE¹E², tragen können, wobei E¹ und E² gleich oder verschieden sein können und für Alkyl, Cycloalkyl oder Aryl stehen.
wobei in den Verbindungen der Formeln I und I'
X ausgewählt ist unter Gruppen der Formeln II.a bis II.k 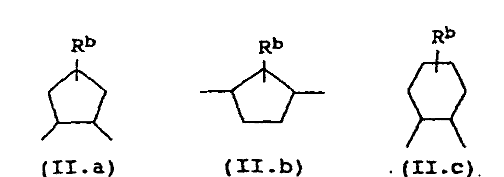 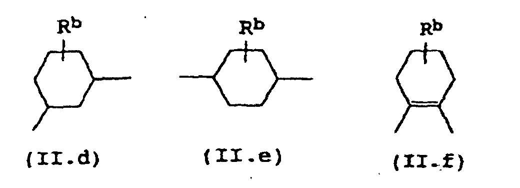 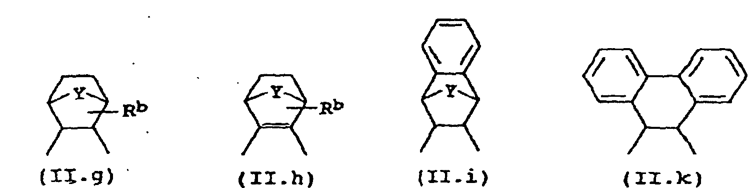 worin
R^{b} ausgewählt ist unter Alkyl, Cycloalkyl, Aryl, Alkoxy, Acyl, Carboxyl, Alkoxycarbonyl, Hydroxy, Nitro, Cyano, Trifluormethyl, Oxo und den Ketalen davon, oder NE¹E², wobei E¹ und E² gleich oder verschieden sein können und für Alkyl, Cycloalkyl oder Aryl stehen, und
Y wie in Anspruch 1 definiert ist,

8. Katalysator, umfassend wenigstens einen Komplex eines Metalls der VIII. Nebengruppe mit mindestens einem Liganden, der ausgewählt ist unter Verbindungen der allgemeinen Formeln I und I', wie in Anspruch 7 definiert.

9. Katalysator nach Anspruch 8, **dadurch gekennzeichnet, dass** das Metall der VIII. Nebengruppe ausgewählt ist unter Cobalt, Ruthenium, Iridium, Rhodium, Nickel, Palladium und Platin.

10. Katalysator nach einem der Ansprüche 8 oder 9, der zusätzlich wenigstens einen weiteren Liganden, ausgewählt unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃, Phospholen, Phosphabenzolen sowie ein-, zwei- und mehrzähnigen Phosphin-, Phosphinit-, Phosphonit-, Phosphoramidit und Phosphitliganden aufweist.

## Claims

1. A process for the hydroformylation of compounds containing at least one ethylenically unsaturated double bond by reaction with carbon monoxide and hydrogen in the presence of a hydroformylation catalyst comprising at least one complex of a metal of transition group VIII with at least one ligand of the formula I 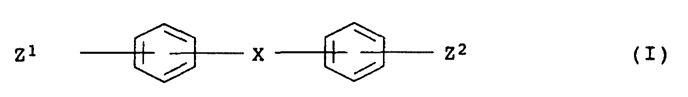 where
Z¹and Z² are, independently of one another, radicals containing at least one phosphorus, arsenic or antimony atom.
X is a divalent, nonaromatic, 3- to 8-membered, carbocyclic or heterocyclic, bridging group which may have one, two or three double bonds and/or bear one, two, three or four substituents selected from among alkyl, cycloalkyl, aryl, heteroaryl, alkoxy, acyl, carboxyl, alkoxycarbonyl, hydroxyl, nitro, cyano, trifluoromethyl, oxo and the ketals thereof and NE¹E², where E¹ and E² may be identical or different and are each alkyl, cycloalkyl or aryl, and/or the bridging group X is part of a fused ring system having 1, 2 or 3 further rings, where the fused-on rings may each bear one, two or three substituents selected from among those specified above as substituents of the group X, and/or the bridging group X may in turn be bridged by -O-, -S-, -N(R^{a})- or a different divalent bridging group, where R^{a} is hydrogen, alkyl, cycloalkyl or aryl, and wherein X is selected from among groups of the formulae II.1 to 11.3 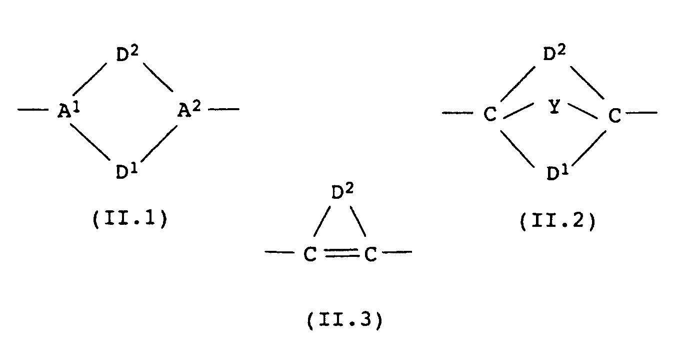 where
A¹and A² are each, independently of one another, B, N, P or CR⁵, where R⁵ is hydrogen, alkyl, cycloalkyl, aryl or heteroaryl,
D¹ is a single bond or a C₁-C₃-alkylene bridge which may have one double bond and/or bear one or two substituents selected from among alkyl, cycloalkyl, aryl, alkoxy, acyl, carboxyl, alkoxycarbonyl, hydroxy, nitro, cyano, trifluoromethyl, oxo and the ketals thereof and NE¹E², where E¹ and E² may be identical or different and are each alkyl, cycloalkyl or aryl,
D² is a C₃-C₆-alkylene bridge which may have one, two or three double bonds and/or bear one, two, three or four of the substituents specified for D¹ and/or the alkylene bridge D² in the formulae II.1 and II.3 may be bridged by a -Y- group, and
Y is O, S, CR^{x}R^{y} or a radical of the formulae III.1 to III.4 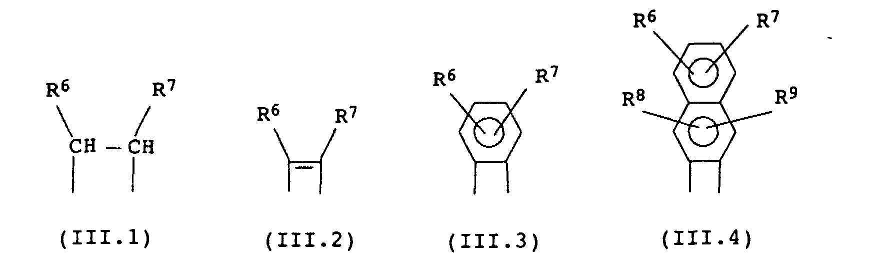
where
R^{x} and R^{y} are selected from among hydrogen, alkyl, cycloalkyl, aryl and heteroaryl,
R⁶, R⁷, R⁸ and R⁹ are each, independently of one another, hydrogen, alkyl, cycloalkyl, aryl, alkoxy, halogen, SO₃H, sulfonate, NE¹E², alkylene-NE¹E², trifluoromethyl, nitro, alkoxycarbonyl, carboxyl or cyano, where E¹ and E² are each, independently of one another, alkyl, cycloalkyl or aryl.

2. A process as claimed in claim 1, wherein Z¹ and Z² in the formula I are each, independently of one another, a radical of the formula PR¹R², OPR¹R², P(OR¹)R², P(OR¹)(OR²), OP(OR¹)R² or OP(OR¹)(OR²), where
R¹ and R² are each, independently of one another, alkyl, cycloalkyl, heterocycloalkyl, aryl or heteroaryl which may bear one, two or three substituents selected from among alkyl, cycloalkyl, heterocycloalkyl, aryl, heteroaryl, COOR^{f}, COO⁻M⁺, SO₃R^{f}, SO⁻₃M⁺, NE³E⁴, alkylene-NE³E⁴, NE³E⁴E⁵⁺X⁻, alkylene-NE³E⁴E⁵⁺X⁻, OR^{f}, SR^{f}, (CHR^{g}CH₂O)_{y}R^{f}, (CH₂N(E³))_{y}R^{f}, (CH₂CH₂N(E³))_{y}R^{f}, halogen, trifluoromethyl, nitro, acyl and cyano, where
R^{f}, E³, E⁴ and E⁵ are identical or different radicals selected from among hydrogen, alkyl, cycloalkyl and aryl,
R^{g} is hydrogen, methyl or ethyl,
M⁺ is a cation,
X⁻ is an anion, and
y is an integer from 1 to 120, or
R¹ and R² together with the phosphorus atom and any oxygen atom(s) to which they are bound form a 5- to 8-membered heterocycle which may additionally have one, two or three cycloalkyl, heterocycloalkyl, aryl or heteroaryl groups fused onto it, where the heterocycle and, if present, the fused-on groups may each, independently of one another, bear one, two, three or four substituents selected from among alkyl, cycloalkyl, heterocycloalkyl, aryl, heteroaryl, COOR^{f}, COO⁻M⁺, SO₃R^{f}, SO⁻₃M⁺, NE³E⁴, alkylene-NE³E⁴, NE³E⁴E⁵⁺X⁻, alkylene-NE³E⁴E⁵⁺X⁻, OR^{f}, SR^{f}, (CHR^{g}CH₂O)_{y}R^{f}, (CH₂N(E¹))_{y}R^{f}, (CH₂CH₂N(E⁴))_{y}R^{f}, halogen, trifluoromethyl, nitro, acyl and cyano, where R^{f}, R^{g}, E³, E⁴, E⁵, M⁺, X- and y are as defined above.

3. A process as claimed in claim 1 comprising at least one complex of a metal of transition group VIII with at least one phosphine ligand of the formula I' 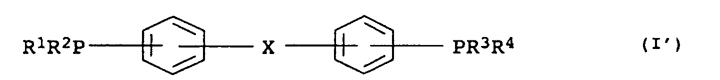 where
R¹, R², R³ and R⁴ are each, independently of another, alkyl, cycloalkyl, aryl or heteroaryl, where the cycloalkyl, aryl and heteroaryl groups may each bear one, two or three substituents selected from among alkyl, cycloalkyl, aryl, alkoxy, cycloalkoxy, aryloxy, acyl, halogen, trifluoromethyl, nitro, cyano, carboxyl, alkoxycarbonyl and NE³E⁴, where E³ and E⁴ may be identical or different and are each alkyl, cycloalkyl or aryl, and
X is a divalent, nonaromatic, 3- to 8-membered, carbocyclic or heterocyclic, bridging group which may have one, two or three double bonds and/or bear one, two, three or four substituents selected from among alkyl, cycloalkyl, aryl, alkoxy, acyl, carboxyl, alkoxycarbonyl, hydroxyl, nitro, cyano, trifluoromethyl, oxo and the ketals thereof and NE¹E², where E¹ and E² may be identical or different and are each alkyl, cycloalkyl or aryl, and/or the bridging group X is part of a fused ring system having 1, 2 or 3 further rings, where the fused-on rings may each bear one, two or three substituents selected from among those specified above as substituents of the group X, and/or the bridging group X may in turn be bridged by -O-, -S-, - N(R^{a})- or a different divalent bridging group, where R^{a} is hydrogen, alkyl, cycloalkyl or aryl, and wherein X is selected from among groups of the formulae II.1 to II.3 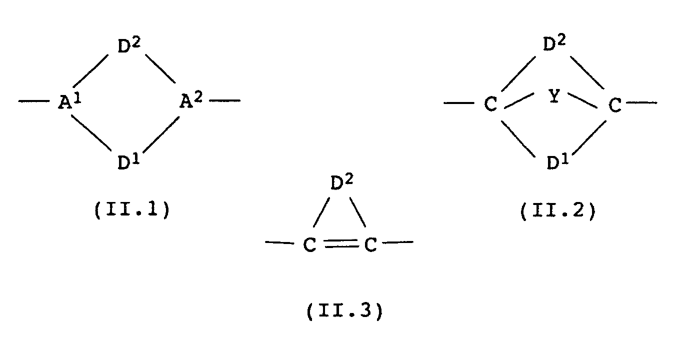 where
A¹ and A² are each, independently of one another, B, N, P or CR⁵, where R⁵ is hydrogen, alkyl, cycloalkyl, aryl or heteroaryl,
D¹ is a single bond or a C₁-C₃-alkylene bridge which may have one double bond and/or bear one or two substituents selected from among alkyl, cycloalkyl, aryl, alkoxy, acyl, carboxyl, alkoxycarbonyl, hydroxy, nitro, cyano, trifluoromethyl, oxo and the ketals thereof and NE¹E², where E¹ and E² may be identical or different and are each alkyl, cycloalkyl or aryl,
D² is a C₃-C₆-alkylene bridge which may have one, two or three double bonds and/or bear one, two, three or four of the substituents specified for D¹ and/or the alkylene bridge D² in the formulae II.1 and II.3 may be bridged by a -Y- group, and
Y is O, S, CR^{x}R^{y} or a radical of the formulae III.1 to III.4 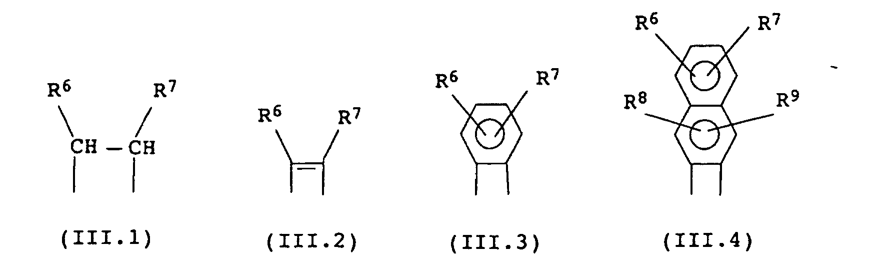
where
R^{x} and R^{y} are selected from among hydrogen, alkyl, cycloalkyl, aryl and heteroaryl,
R⁶, R⁷, R⁸ and R⁹ are each, independently of one another, hydrogen, alkyl, cycloalkyl, aryl, alkoxy, halogen, SO₃H, sulfonate, NE¹E², alkylene-NE¹E², trifluoromethyl, nitro, alkoxycarbonyl, carboxyl or cyano, where E¹ and E² are each, independently of one another, alkyl, cycloalkyl or aryl.

4. A process as claimed in any of the preceding claims, wherein X is selected from among groups of the formulae II.a to II.k 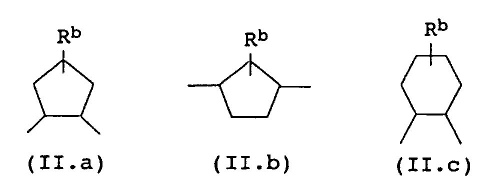 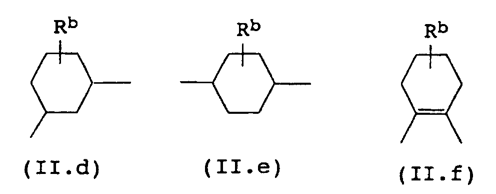 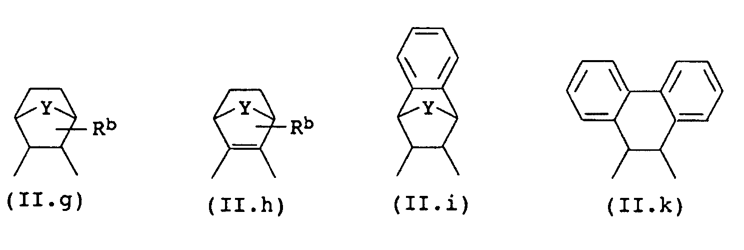 where
R^{b} is selected from among alkyl, cycloalkyl, aryl, alkoxy, acyl, carboxyl, alkoxycarbonyl, hydroxyl, nitro, cyano, trifluoromethyl, oxo and the ketals thereof, and NE¹E², where E¹ and E² may be identical or different and are each alkyl, cycloalkyl or aryl, and
Y is as defined in claim 1.

5. A process as claimed in any of the preceding claims, wherein the ligand of the formula I is selected from among ligands of the formulae IA to IF 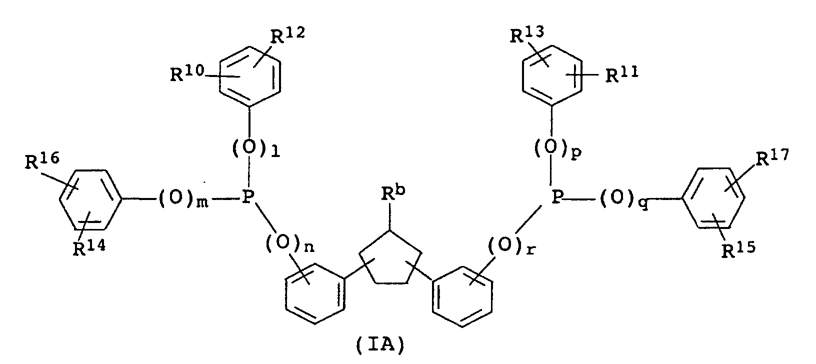 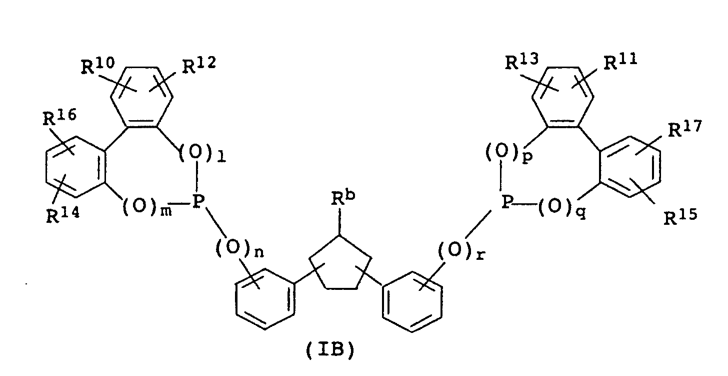 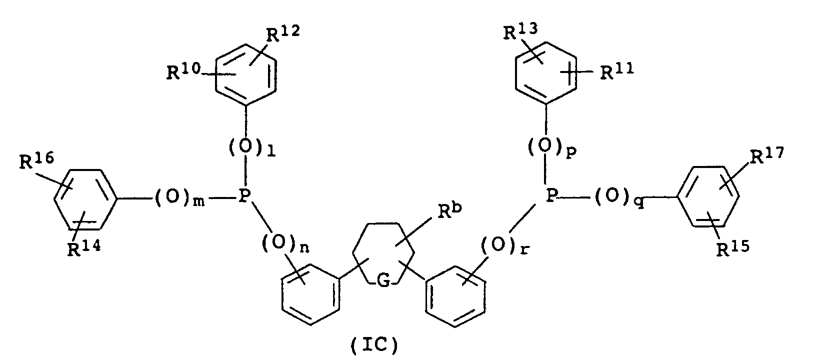 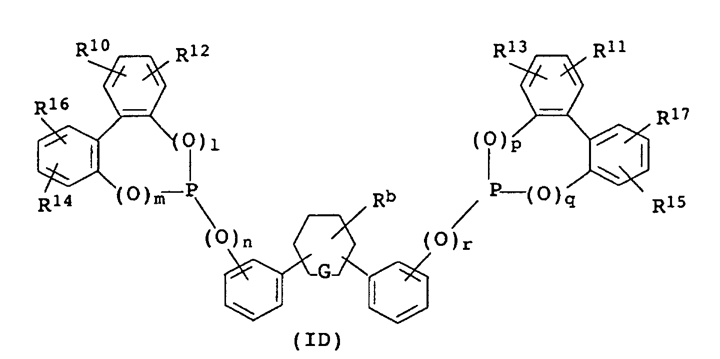 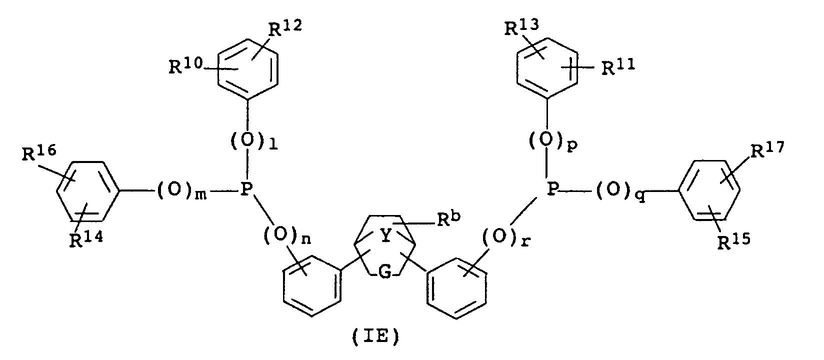 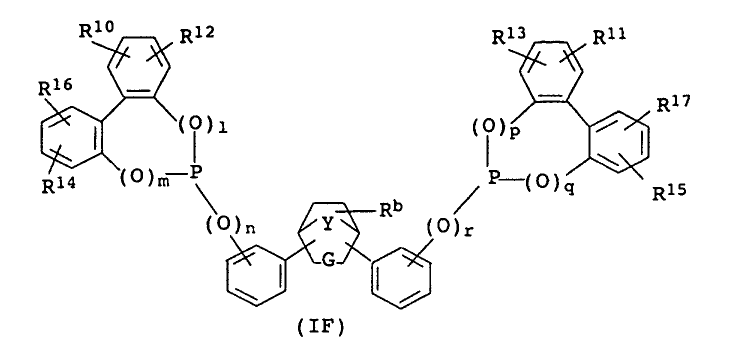 where
R¹⁰, R¹¹, R¹², R¹³, R¹⁴, R¹⁵, R¹⁶ and R¹⁷ are selected independently from among hydrogen, alkyl, cycloalkyl, aryl, alkoxy, cycloalkoxy, aryloxy, acyl, halogen, trifluoromethyl, nitro, cyano, carboxyl, alkoxycarbonyl and NE³E⁴, where E³ and E⁴ may be identical or different and are each alkyl, cycloalkyl or aryl,
l, m, n, p, q and r are each, independently of one another, 0 or 1,
R^{b} in the formulae IA and IB is selected from among hydrogen, alkyl, oxo and the ketals thereof and in the formulae IC, ID, IE and IF is selected from among hydrogen and alkyl,
G is a single bond, a double bond or an oxygen atom,
Y is O, S, CH₂ or a radical of the formulae III.1 to III.4 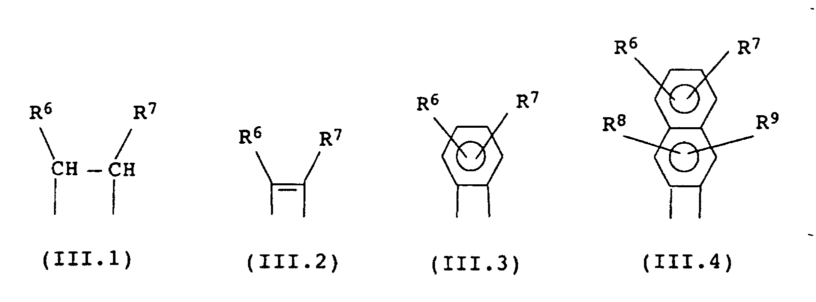 where
R⁶, R⁷, R⁸ and R⁹ are each, independently of one another, hydrogen, alkyl, cycloalkyl, aryl, alkoxy, halogen, SO₃H, sulfonate, NE¹E², alkylene-NE¹E², trifluoromethyl, nitro, alkoxycarbonyl, carboxyl or cyano, where E¹ and E² are each, independently of one another, alkyl, cycloalkyl or aryl.

6. A process as claimed in any of the preceding claims, wherein the ligand of the formula I is selected from among phosphine ligands of the formulae Ia-Ie 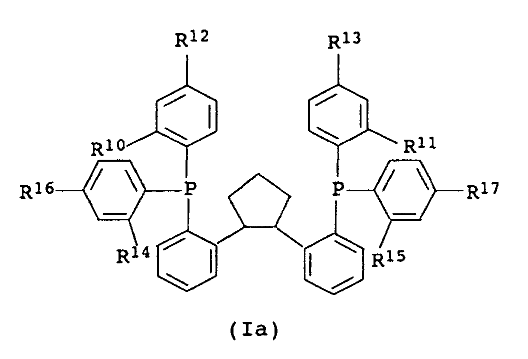 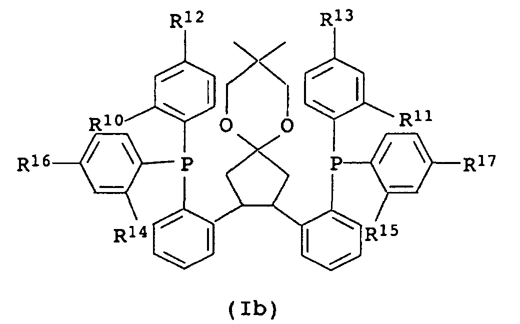 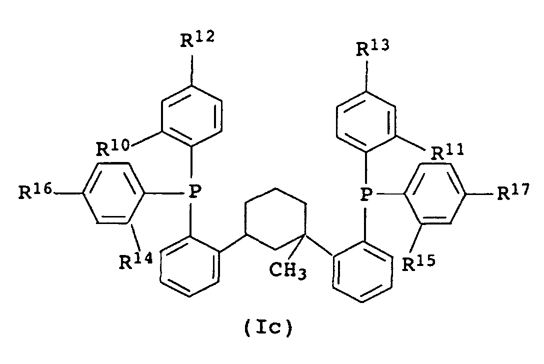 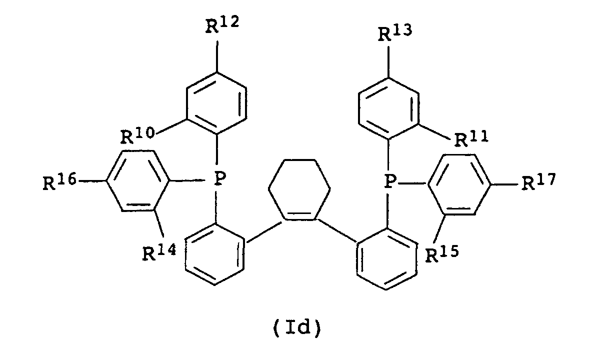 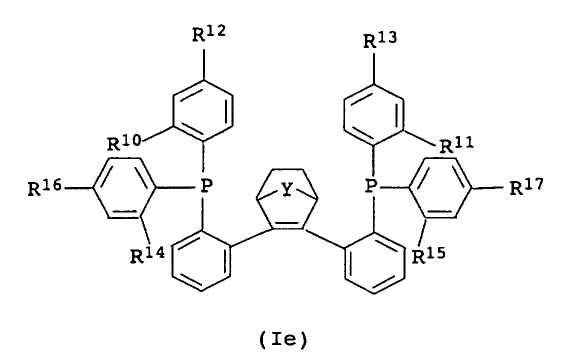 where, in the formulae Ia to Ie, the substituents R¹⁰, R¹¹,
R¹², R¹³, R¹⁴, R¹⁵, R¹⁶ and R¹⁷ have the following meanings:
| R¹⁰ | R¹¹ | R¹² | R¹³ | R¹⁴ | R¹⁵ | R¹⁶ | R¹⁷ |
|---|---|---|---|---|---|---|---|
| H | H | H | H | H | H | H | H |
| CH₃ | CH₃ | H | H | H | H | H | H |
| CH₃ | CH₃ | H | H | CH₃ | CH₃ | H | H |
| CH₃ | CH₃ | CH₃ | CH₃ | H | H | H | H |
| CH₃ | CH₃ | CH₃ | CH₃ | CH₃ | CH₃ | H | H |
| CH₃ | CH₃ | CH₃ | CH₃ | CH₃ | CH₃ | CH₃ | CH₃ |
and, in the formula Ie, Y is O, S, CH₂ or a radical of the formulae III.1 to III.4 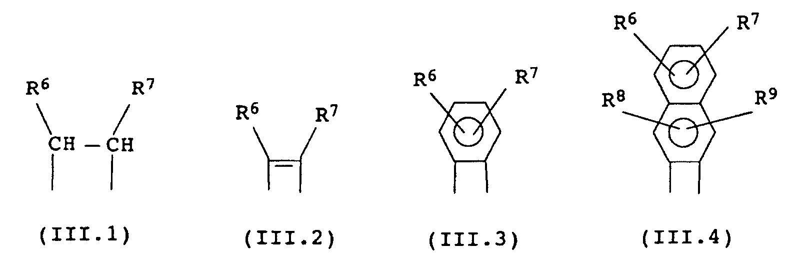 where
R⁶, R⁷, R⁸ and R⁹ are each, independently of one another hydrogen, alkyl, cycloalkyl, aryl, alkoxy, halogen, SO₃H, sulfonate, NE¹E², alkylene-NE¹E², trifluoromethyl, nitro, alkoxycarbonyl, carboxyl or cyano, where E¹ and E² are each, independently of one another, alkyl, cycloalkyl or aryl.

7. A compound of the formula I 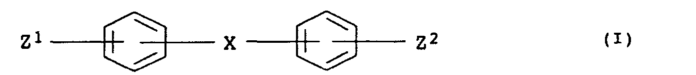 where
Z¹ and Z² are, independently of one another, radicals containing at least one phosphorus, arsenic or antimony atom, where at least two substituted or unsubstituted heteroatoms selected from among 0, S and NR^{c}, where R^{c} is hydrogen, alkyl, cycloalkyl or aryl, are in each case directly bound to the phosphorus, arsenic or antimony atom, and
a compound of the formula I' 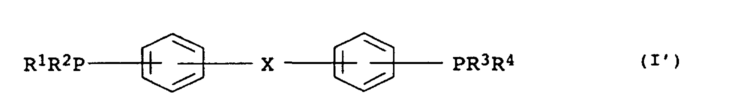 where
R¹, R², R³ and R⁴ are each, independently of another, alkyl, cycloalkyl, aryl or heteroaryl, where the aryl and heteroaryl groups may each bear one, two or three substituents selected from among alkyl, cycloalkyl, aryl, alkoxy, cycloalkoxy, aryloxy, acyl, halogen, trifluoromethyl, nitro, cyano, carboxyl, alkoxycarbonyl and NE¹E², where E¹ and E² may be identical or different and are each alkyl, cycloalkyl or aryl,
where, in the compounds of the formulae I and I', X is selected from among groups of the formulae II.a to II.k 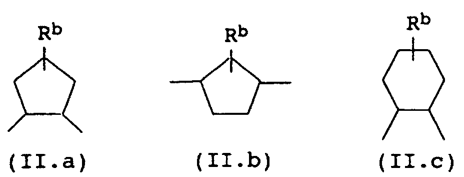 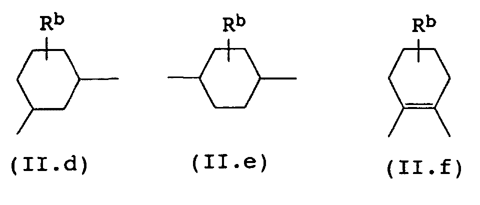 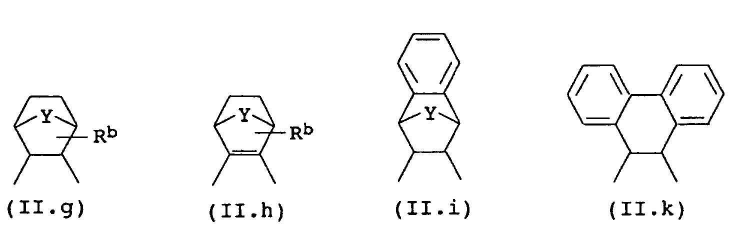 where
R^{b} is selected from among alkyl, cycloalkyl, aryl, alkoxy, acyl, carboxyl, alkoxycarbonyl, hydroxyl, nitro, cyano, trifluoromethyl, oxo and the ketals thereof, and NE¹E², where E¹ and E² may be identical or different and are each alkyl, cycloalkyl or aryl, and
Y is as defined in claim 1.

8. A catalyst comprising at least one complex of a metal of transition group VIII with at least one ligand selected from among compounds of the formulae I and I' as defined in claim 7.

9. A catalyst as claimed in claim 8, wherein the metal of transition group VIII is selected from among cobalt, ruthenium, iridium, rhodium, nickel, palladium and platinum.

10. A catalyst as claimed in claim 8 or 9 which further comprises at least one additional ligand selected from among halides, amines, carboxylates, acetylacetonate, arylsulfonates and alkylsulfonates, hydride, CO, olefins, dienes, cycloolefins, nitriles, N-containing heterocycles, aromatics and heteroaromatics, ethers, PF₃, phospholes, phosphabenzenes and monodentate, bidentate and polydentate phosphine, phosphinite, phosphonite, phosphoramidite and phosphite ligands.

## Revendications

1. Procédé d'hydroformylation de composés qui contiennent au moins une double liaison à insaturation éthylénique, au moyen d'une réaction avec du monoxyde de carbone et de l'hydrogène en présence d'un catalyseur d'hydroformylation, **caractérisé en ce que** l'on met en oeuvre, en tant que catalyseur d'hydroformylation, au moins un complexe d'un métal du sous-groupe VIII avec au moins un ligand de formule générale I 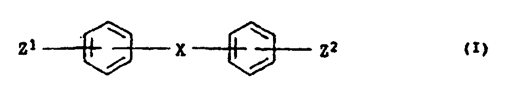 dans laquelle
Z¹ et Z² représentent, indépendamment l'un de l'autre, des groupes contenant au moins un atome de phosphore, d'arsenic ou d'antimoine, et
X représente un groupe pontant bivalent non aromatique, carbocyclique ou hétérocyclique de 3 à 8 éléments, qui peut porter une, deux ou trois doubles liaisons, et/ou un, deux, trois ou quatre substituants, qui sont choisis parmi un groupe alkyle, cycloalkyle, aryle, hétaryle, alcoxy, acyle, carboxyle, alcoxycarbonyle, hydroxy, nitro, cyano, trifluorométhyle, oxo et ses dérivés cétal, ou NE¹E², où E¹ et E² peuvent être identiques ou différents et représentent un groupe alkyle, cycloalkyle ou aryle, et/ou le groupe pontant X peut faire partie d'un système cyclique condensé contenant 1, 2 ou 3 autres cycles, les cycles condensés pouvant chacun porter un, deux ou trois substituants, qui sont choisis parmi ceux mentionnés précédemment en tant que substituants du groupe X, et/ou le groupe pontant X pouvant être lui-même ponté par -O-, -S-, -N(R^{a})- ou un groupe pontant bivalent différent, R^{a} représentant un atome d'hydrogène, un groupe alkyle, cycloalkyle ou aryle, et X étant choisi parmi des groupes de formule II.1 à II.3 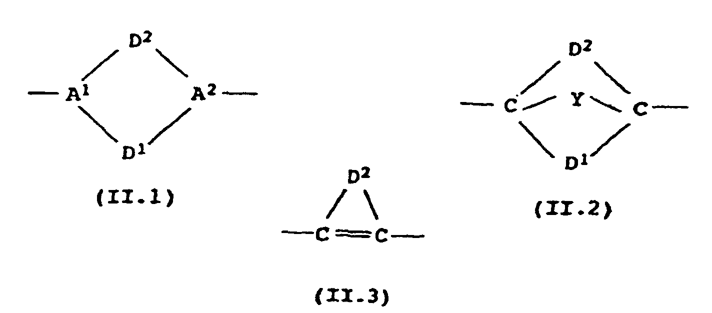 dans lesquelles
A¹ et A² représentent, indépendamment l'un de l'autre, B, N, P ou CR⁵, où R⁵ représente un atome d'hydrogène, un groupe alkyle, cycloalkyle, aryle ou hétaryle,
D¹ représente une liaison simple ou un pont alkylène en C₁ à C₃, qui peut porter une double liaison et/ou un ou deux substituants, qui sont choisis parmi un groupe alkyle, cycloalkyle, aryle, alcoxy, acyle, carboxyle, alcoxycarbonyle, hydroxy, nitro, cyano, trifluorométhyle, oxo et ses dérivés cétal, ou NE¹E², où E¹ et E² peuvent être identiques ou différents et représentent un groupe alkyle, cycloalkyle ou aryle,
D² représente un pont alkylène en C₃ à C₆, qui peut présenter une, deux ou trois doubles liaisons et/ou un, deux, trois ou quatre des substituants mentionnés pour D¹ et/ou le pont alkylène D² dans les formules II.1 et II.3 peut être ponté par un groupe -Y-, et
Y représente O, S, CR^{x}R^{y} ou un groupe de formule III.1 à III.4 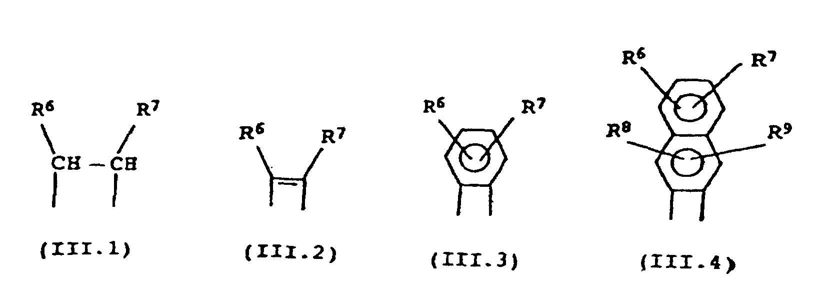 dans lesquelles
R^{x} et R^{y} sont choisis parmi un atome d'hydrogène, un groupe alkyle, cycloalkyle, aryle ou hétaryle,
R⁶, R⁷, R⁸ et R⁹ représentent, indépendamment les uns des autres, un atome d'hydrogène, un groupe alkyle, cycloalkyle, aryle, alcoxy, halogéno, SO₃H, sulfonate, NE¹E², alkylène-NE¹E², trifluorométhyle, nitro, alcoxycarbonyle, carboxyle ou cyano, E¹ et E² représentant, indépendamment l'un de l'autre, un groupe alkyle, cycloalkyle ou aryle.

2. Procédé selon la revendication 1, dans lequel, dans la formule I, Z¹ et Z² représentent chacun, indépendamment l'un de l'autre, un groupe de formule PR¹R², OPR¹R², P(OR¹)R², P(OR¹)(OR²), OP(OR¹)R² ou OP(OR¹)(OR²), dans lesquelles
R¹ et R² représentent, indépendamment l'un de l'autre, un groupe alkyle, cycloalkyle, hétérocycloalkyle, aryle ou hétaryle, lesquels peuvent éventuellement présenter un, deux ou trois substituants choisis parmi un groupe alkyle, cycloalkyle, hétérocycloalkyle, aryle, hétaryle, COOR^{f}, COO-M⁺, SO³R^{f}, SO⁻₃M⁺, NE³E⁴, alkylène-NE³E⁴, NE³E⁴E⁵+X⁻, alkylène-NE³E⁴E⁵⁺X⁻, OR^{f}, SR^{f}, (CHR^{g}CH₂O)_{y}R^{f}, (CH₂N(E³))_{y}R^{f}, (CH₂CH₂N(E³))_{y}R^{f}, halogéno, trifluorométhyle, nitro, acyle ou cyano, où
R^{f}, E³, E⁴ et E⁵ représentent à chaque fois des groupes identiques ou différents choisis parmi un atome d'hydrogène, un groupe alkyle, cycloalkyle ou aryle,
R^{g} représente un atome d'hydrogène, un groupe méthyle ou éthyle,
M⁺ représente un cation,
X⁻ représente un anion, et
y représente un nombre entier de 1 à 120, ou bien
R¹ et R² représentent, conjointement avec l'atome de phosphore et éventuellement le ou les atomes d'oxygène auxquels ils sont liés, un hétérocycle de 5 à 8 éléments qui est en plus éventuellement condensé une, deux ou trois fois avec un groupe cycloalkyle, hétérocycloalkyle, aryle ou hétaryle, l'hétérocycle et, si présent, le groupe condensé, pouvant porter chacun, indépendamment l'un de l'autre, un, deux, trois ou quatre substituants qui sont choisis parmi un groupe alkyle, cycloalkyle, hétérocycloalkyle, aryle, hétaryle, COOR^{f}, COO⁻M⁺, SO₃R^{f}, SO⁻₃M⁺, NE³E⁴, alkylène-NE³E⁴, NE³E⁴E⁵⁺X⁻, alkylène-NE³E⁴E⁵⁺X⁻, OR^{f}, SR^{f}, (CHR^{g}CH₂O)_{y}R^{f}, (CH₂N(E¹))_{y}R^{f}, (CH₂CH₂N(E⁴))_{y}R^{f}, halogéno, trifluorométhyle, nitro, acyle ou cyano, R^{f}, R^{g}, E³, E⁴, E⁵, M⁺, X- et y ayant les significations mentionnées précédemment.

3. Procédé selon la revendication 1, **caractérisé en ce que**, l'on met en oeuvre, en tant que catalyseur d'hydroformylation, au moins un complexe d'un métal du sous-groupe VIII avec au moins un ligand phosphine de formule générale I' 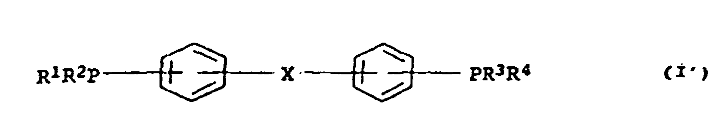 dans laquelle
R¹, R², R³ et R⁴ représentent, indépendamment les uns des autres, un groupe alkyle, cycloalkyle, aryle ou hétaryle, les groupes cycloalkyle, aryle et hétaryle pouvant porter chacun un, deux ou trois substituants qui sont choisis parmi un groupe alkyle, cycloalkyle, aryle, alcoxy, cycloalcoxy, aryloxy, acyle, halogéno, trifluorométhyle, nitro, cyano, carboxyle, alcoxycarbonyle ou NE³E⁴, où E³ et E⁴ peuvent être identiques ou différents et représentent un groupe alkyle, cycloalkyle ou aryle, et
X représente un groupe pontant bivalent non aromatique, carbocyclique ou hétérocyclique de 3 à 8 éléments, qui peut porter une, deux ou trois doubles liaisons, et/ou un, deux, trois ou quatre substituants, qui sont choisis parmi un groupe alkyle, cycloalkyle, aryle, alcoxy, acyle, carboxyle, alcoxycarbonyle, hydroxy, nitro, cyano, trifluorométhyle, oxo et ses dérivés cétal, ou NE¹E², où E¹ et E² peuvent être identiques ou différents et représentent un groupe alkyle, cycloalkyle ou aryle, et/ou le groupe pontant X peut faire partie d'un système cyclique condensé contenant 1, 2 ou 3 autres cycles, les cycles condensés pouvant chacun porter un, deux ou trois substituants, qui sont choisis parmi ceux mentionnés précédemment en tant que substituants du groupe X, et/ou le groupe pontant X pouvant être lui-même ponté par -O-, -S-, -N(R^{a})- ou un groupe pontant bivalent différent, R^{a} représentant un atome d'hydrogène, un groupe alkyle, cycloalkyle ou aryle, et X étant choisi parmi des groupes de formule II.1 à II.3 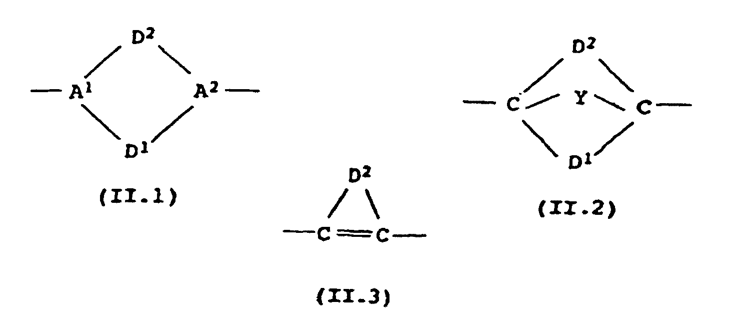
dans lesquelles
A¹ et A² représentent, indépendamment l'un de l'autre, B, N, P ou CR⁵, où R⁵ représente un atome d'hydrogène, un groupe alkyle, cycloalkyle, aryle ou hétaryle,
D¹ représente une liaison simple ou un pont alkylène en C₁ à C₃, qui peut porter une double liaison et/ou un ou deux substituants, qui sont choisis parmi un groupe alkyle, cycloalkyle, aryle, alcoxy, acyle, carboxyle, alcoxycarbonyle, hydroxy, nitro, cyano, trifluorométhyle, oxo et ses dérivés cétal, ou NE¹E², où E¹ et E² peuvent être identiques ou différents et représentent un groupe alkyle, cycloalkyle ou aryle,
D² représente un pont alkylène en C₃ à C₆, qui peut présenter une, deux ou trois doubles liaisons et/ou un, deux, trois ou quatre des substituants mentionnés pour D¹ et/ou le pont alkylène D² dans les formules II.1 et II.3 peut être ponté par un groupe -Y-, et
Y représente O, S, CR^{x}R^{y} ou un groupe de formule III.1 à III.4 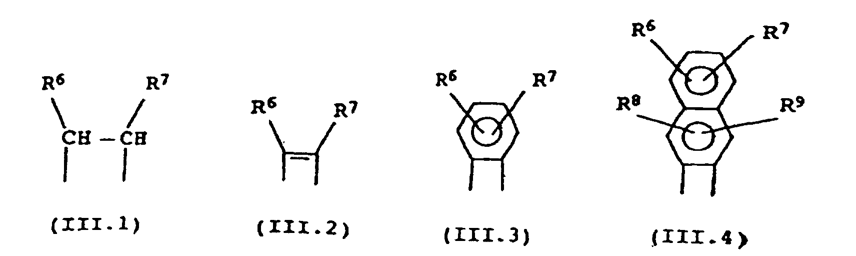 dans lesquelles
R^{x} et R^{y} sont choisis parmi un atome d'hydrogène, un groupe alkyle, cycloalkyle, aryle ou hétaryle,
R⁶, R⁷, R⁸ et R⁹ représentent, indépendamment les uns des autres, un atome d'hydrogène, un groupe alkyle, cycloalkyle, aryle, alcoxy, halogéno, SO₃H, sulfonate, NE¹E², alkylène-NE¹E², trifluorométhyle, nitro, alcoxycarbonyle, carboxyle ou cyano, E¹ et E² représentant, indépendamment l'un de l'autre, un groupe alkyle, cycloalkyle ou aryle.

4. Procédé selon l'une quelconque des revendications précédentes, dans lequel X est choisi parmi des groupes de formule II.a à II.k 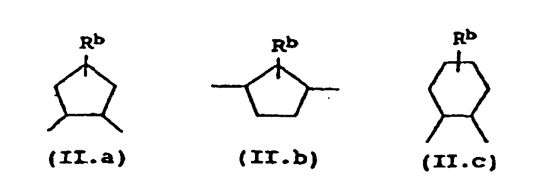 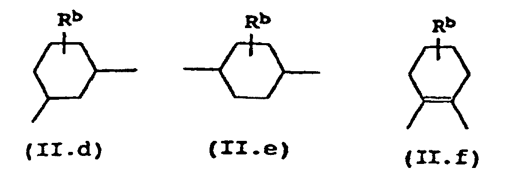 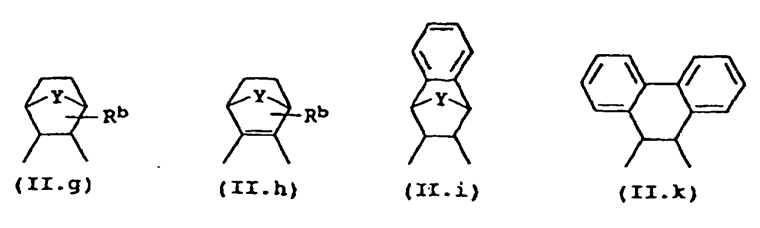 dans lesquelles
R^{b} est choisi parmi un groupe alkyle, cycloalkyle, aryle, alcoxy, acyle, carboxyle, alcoxycarbonyle, hydroxy, nitro, cyano, trifluorométhyle, oxo et ses dérivés cétal, ou NE¹E², où E¹ et E² peuvent être identiques ou différents et représentent un groupe alkyle, cycloalkyle ou aryle, et
Y est tel que défini dans la revendication 1.

5. Procédé selon l'une quelconque des revendications précédentes, dans lequel le ligand de formule I est choisi parmi des ligands de formule IA à IF 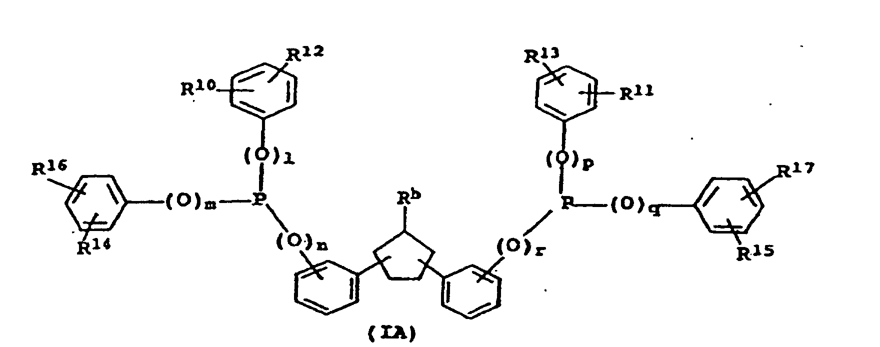 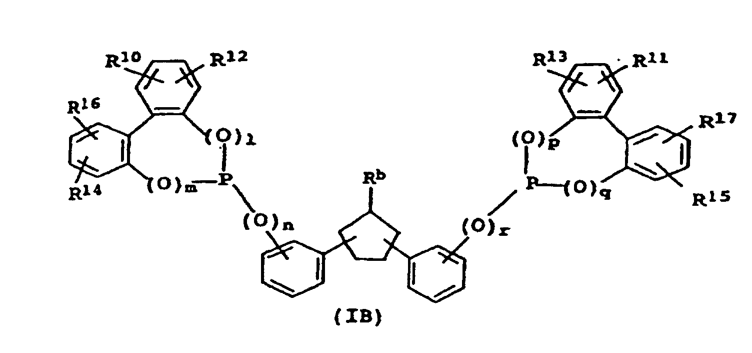 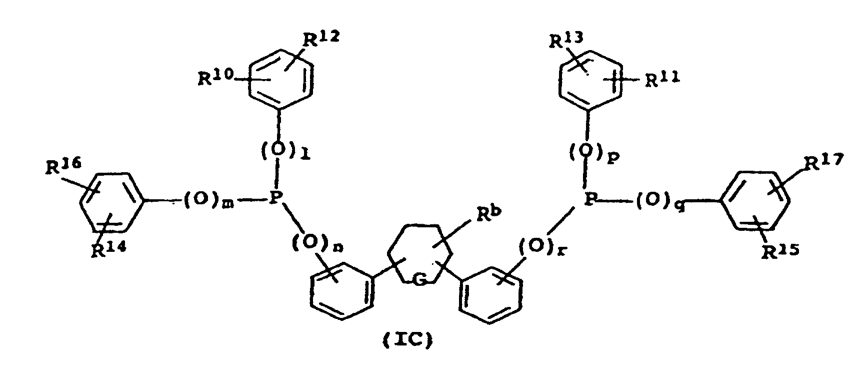 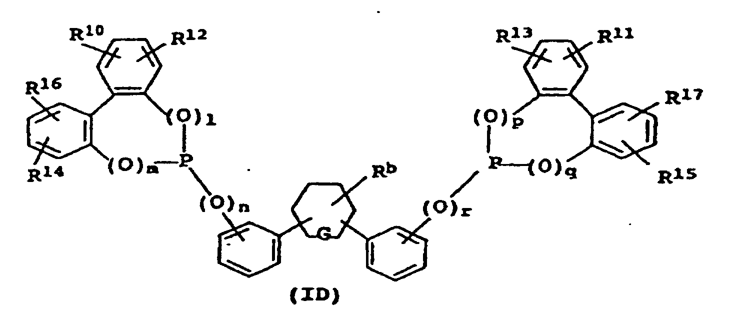 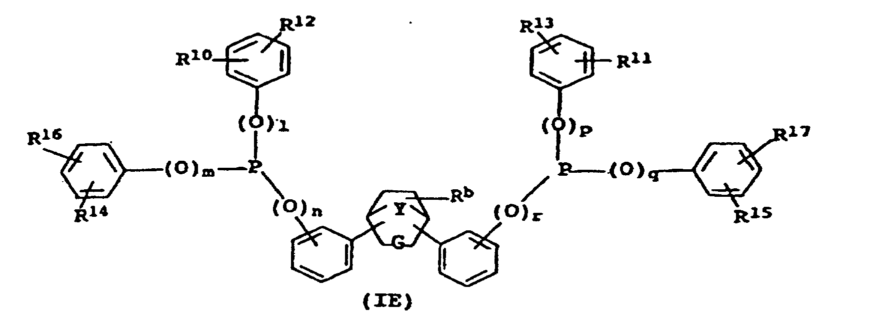 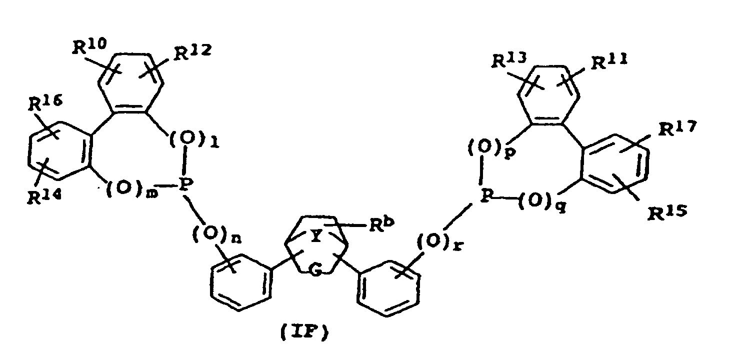 dans lesquelles
R¹⁰, R¹¹, R¹², R¹³, R¹⁴, R¹⁵, R¹⁶ et R¹⁷ représentent, indépendamment les uns des autres, un atome d'hydrogène, un groupe alkyle, cycloalkyle, aryle, alcoxy, cycloalcoxy, aryloxy, acyle, halogéno, trifluorométhyle, nitro, cyano, carboxyle, alcoxycarbonyle ou NE³E⁴, où E³ et E⁴ peuvent être identiques ou différents et représentent un groupe alkyle, cycloalkyle ou aryle,
l, m, n, p, q et r valent, indépendamment les uns des autres, 0 ou 1,
R^{b} est choisi, dans les formules IA et IB, parmi un atome d'hydrogène, un groupe alkyle, oxo et ses dérivés cétal et, dans les formules IC, ID, IE et IF, parmi un atome d'hydrogène et un groupe alkyle,
G représente une liaison simple, une double liaison ou un atome d'oxygène,
Y représente O, S, CH₂ ou un groupe de formule III.1 à III.4 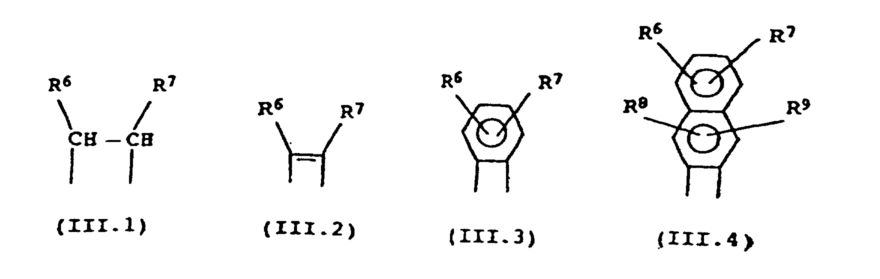 dans lesquelles
R⁶, R⁷, R⁸ et R⁹ représentent, indépendamment les uns des autres, un atome d'hydrogène, un groupe alkyle, cycloalkyle, aryle, alcoxy, halogéno, SO₃H, sulfonate, NE¹E², alkylène-NE¹E², trifluorométhyle, nitro, alcoxycarbonyle, carboxyle ou cyano, E¹ et E² représentant, indépendamment l'un de l'autre, un groupe alkyle, cycloalkyle ou aryle.

6. Procédé selon l'une quelconque des revendications précédentes, dans lequel le ligand de formule I est choisi parmi des ligands phosphine de formule Ia à Ie 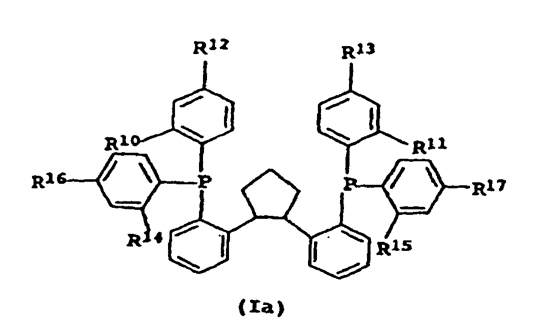 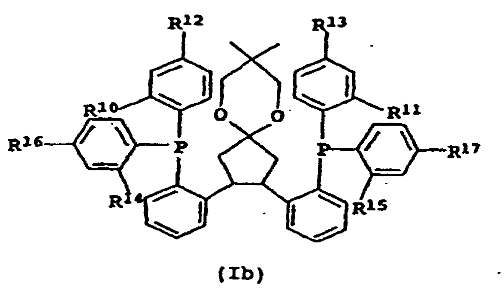 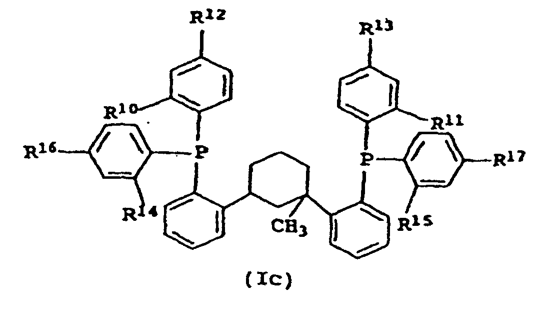 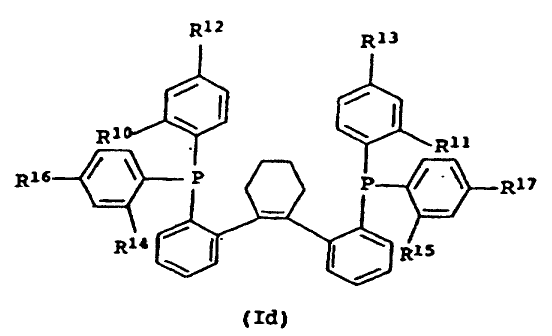 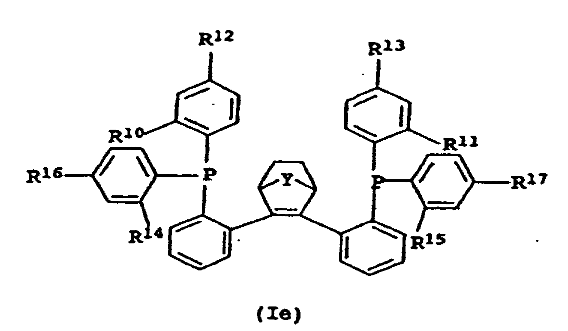 où, dans les formules Ia à Ie, les substituants R¹⁰, R¹¹, R¹², R¹³, R¹⁴, R¹⁵, R¹⁶ et R¹⁷ ont les significations suivantes :
| R¹⁰ | R¹¹ | R¹² | R¹³ | R¹⁴ | R¹⁵ | R¹⁶ | R¹⁷ |
|---|---|---|---|---|---|---|---|
| H | H | H | H | H | H | H | H |
| CH₃ | CH₃ | H | H | H | H | H | H |
| CH₃ | CH₃ | H | H | CH₃ | CH₃ | H | H |
| CH₃ | CH₃ | CH₃ | CH₃ | H | H | H | H |
| CH₃ | CH₃ | CH₃ | CH₃ | CH₃ | CH₃ | H | H |
| CH₃ | CH₃ | CH₃ | CH₃ | CH₃ | CH₃ | CH₃ | CH₃ |
et dans la formule le, Y représente 0, S, CH₂ ou un groupe de formule III.1 à III.4 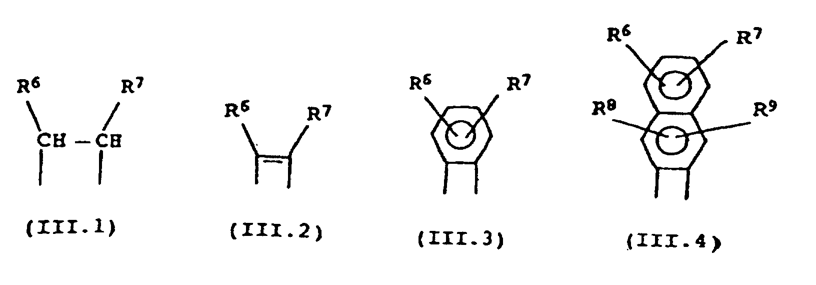 dans lesquelles
R⁶, R⁷, R⁸ et R⁹ représentent, indépendamment les uns des autres, un atome d'hydrogène, un groupe alkyle, cycloalkyle, aryle, alcoxy, halogéno, SO₃H, sulfonate, NE¹E², alkylène-NE¹E², trifluorométhyle, nitro, alcoxycarbonyle, carboxyle ou cyano, E¹ et E² représentant, indépendamment l'un de l'autre, un groupe alkyle, cycloalkyle ou aryle.

7. Composés de formule générale I 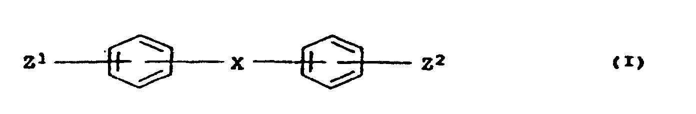 dans lesquelles
Z¹ et Z² représentent, indépendamment l'un de l'autre, des groupes contenant au moins un atome de phosphore, d'arsenic ou d'antimoine, où à chaque fois au moins deux hétéroatomes éventuellement substitués qui sont choisis parmi O, S et NR^{C} sont directement liés l'atome de phosphore, d'arsenic ou d'antimoine, R^{C} représentant un atome d'hydrogène, un groupe alkyle, cycloalkyle ou aryle,
et composés I de formule générale I' 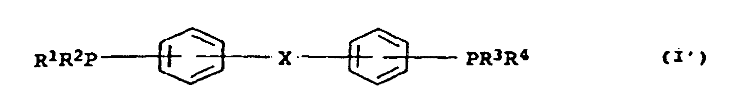 dans lesquelles
R¹, R², R³ et R⁴ représentent, indépendamment les uns des autres, un groupe alkyle, cycloalkyle, aryle ou hétaryle, les groupes aryle et hétaryle pouvant porter chacun un, deux ou trois substituants qui sont choisis parmi un groupe alkyle, cycloalkyle, aryle, alcoxy, cycloalcoxy, aryloxy, acyle, halogéno, trifluorométhyle, nitro, cyano, carboxyle, alcoxycarbonyle ou NE¹E², où E¹ et E² peuvent être identiques ou différents et représentent un groupe alkyle, cycloalkyle ou aryle, et
où, dans les composés de formule I et I', X est choisi parmi des groupes de formule II.a à I.k 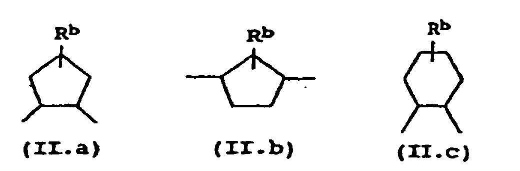 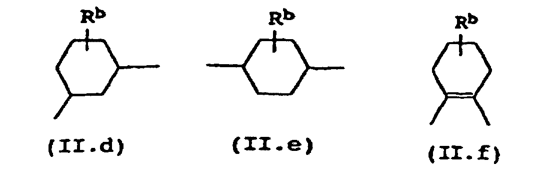 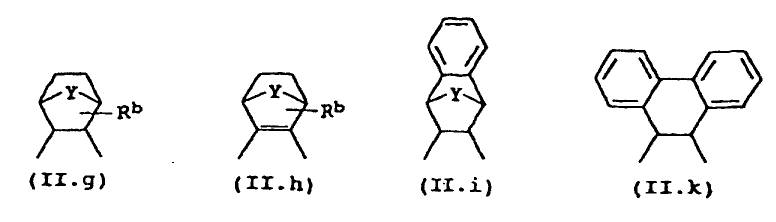 dans lesquelles
R^{b} est choisi parmi un groupe alkyle, cycloalkyle, aryle, alcoxy, acyle, carboxyle, alcoxycarbonyle, hydroxy, nitro, cyano, trifluorométhyle, oxo et ses dérivés cétal, ou NE¹E², où E¹ et E² peuvent être identiques ou différents et représentent un groupe alkyle, cycloalkyle ou aryle, et
Y est tel que défini dans la revendication 1.

8. Catalyseur, comprenant au moins un complexe d'un métal du sous-groupe VIII contenant au moins un ligand qui est choisi parmi les composés de formule générale I et I' comme défini dans la revendication 7.

9. Catalyseur selon la revendication 8, **caractérisé en ce que** le métal du sous-groupe VIII est choisi parmi le cobalt, le ruthénium, l'iridium, le rhodium, le nickel, le palladium et le platine.

10. Catalyseur selon l'une quelconque des revendications 8 ou 9 qui présente en outre au moins un autre ligand choisi parmi les halogénures, les amines, les carboxylates, l'acétylacétonate, les aryl- ou alkylsulfonates, les hydrures, CO, les oléfines, les diènes, les cyclooléfines, les nitriles, les hétérocycles azotés, les composés aromatiques et les composés hétéroaromatiques, les éthers, PF₃, les phosphols, les phosphabenzènes ainsi que les ligands phosphine, phosphinite, phosphonite, phosphoramidite et phosphit bidentés ou polydentés.
